(19) Europäisches Patentamt
European Patent Office
Office européen des brevets

(11) EP 3 172 775 B1

(12) **EUROPÄISCHE PATENTSCHRIFT**

(45) Veröffentlichungstag und Bekanntmachung des Hinweises auf die Patenterteilung:
**20.04.2022 Patentblatt 2022/16**

(21) Anmeldenummer: **15733629.8**

(22) Anmeldetag: **30.06.2015**

(51) Internationale Patentklassifikation (IPC):
***H01L 51/30*** (2006.01) ***C07D 235/18*** (2006.01)
***H01L 51/05*** (2006.01) ***H01L 51/42*** (2006.01)
***H01L 51/50*** (2006.01)

(52) Gemeinsame Patentklassifikation (CPC):
**H01L 51/0067; C07D 235/18; C07D 401/10; C07D 401/14; C07D 403/04; C07D 403/10; C07D 403/14; C07D 405/10; C07D 405/14; C09K 11/025; H01L 51/0052; H01L 51/0058; H01L 51/0072;** H01L 51/5012; H01L 51/5072;
(Forts.)

(86) Internationale Anmeldenummer:
**PCT/EP2015/001315**

(87) Internationale Veröffentlichungsnummer:
**WO 2016/012075 (28.01.2016 Gazette 2016/04)**

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.07.2014 EP 14002528**

(43) Veröffentlichungstag der Anmeldung:
**31.05.2017 Patentblatt 2017/22**

(73) Patentinhaber: **Merck Patent GmbH**
**64293 Darmstadt (DE)**

(72) Erfinder:
- **JATSCH, Anja**
  **60489 Frankfurt am Main (DE)**
- **PARHAM, Amir Hossain**
  **60486 Frankfurt am Main (DE)**
- **EBERLE, Thomas**
  **76829 Landau (DE)**
- **GROSSMANN, Tobias**
  **64297 Darmstadt (DE)**
- **KROEBER, Jonas Valentin**
  **60311 Frankfurt am Main (DE)**

(56) Entgegenhaltungen:
**DE-A1-102008 036 982** **DE-A1-102010 033 080**
**KR-A- 20140 014 956** **US-A1- 2013 200 356**

EP 3 172 775 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
Y02E 10/549

## Beschreibung

Die vorliegende Erfindung betrifft Verbindungen, Zusammensetzungen, Formulierungen und elektronische Vorrichtungen enthaltend die Verbindungen oder Zusammensetzungen.

Der Aufbau organischer Elektrolumineszenzvorrichtungen (z.B. OLEDs - organic light emitting diodes oder OLECs - organic light emitting electrochemical cells), in denen organische Halbleiter als organische funktionelle Materialien eingesetzt werden, ist beispielsweise in US 4539507, US 5151629, EP 0676461 und WO 98/27136 beschrieben. Als emittierende Materialien werden hierbei neben fluoreszierenden Emittern zunehmend metallorganische Komplexe eingesetzt, die Phosphoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es sowohl bei OLEDs, die Singulettemission zeigen, wie auch bei OLEDs, die Triplettemission zeigen, immer noch Verbesserungsbedarf, insbesondere im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Verbindungen enthaltend sowohl Triazin- und Benzimidazolgruppen als Elektronentransportmaterialien sind im Stand der Technik bekannt. WO 2010/126270 offenbart solche Verbindungen. Beide Gruppen sind an einen Fluorengrundkörper gebunden, wobei die beiden aromatischen Ringe des Fluorens weiter aromatisch kondensiert vorliegen.

Auch KR 101257695 offenbart Fluorene enthaltend eine Triazin- und eine Benzimidazolgruppe. Allerdings ist die Triazingruppe nur in zwei der drei kohlenstoffhaltigen Positionen substituiert.

WO 2013/100464 offenbart Spirobifluorene, die sowohl eine Benzimidazolals auch eine Triazingruppe enthalten. Allerdings sind die Triazine unsubstituiert. Außerdem wurde an das Spirobifluoren eine Benzothiophengruppe kondensiert.

US 2013/200356 A1 offenbart Verbindngen, die neben Benzimidazol- auch Triazingruppen enthalten sowie deren Verwendung in organischen lichtemittierenden Dioden.

DE 102010033080 A1 offenbart Polymere, die als Struktureinheiten Fluorene, Indenofluorene oder Spirobifluorene enthalten können, die mit elektronentransportierenden Gruppen substituiert sein können sowie deren Verwendung in organischen Elektrolumineszenzvorrichtungen.

DE 102008036982 A1 offenbart Fluorene und Spirobifluorene, die mit Triazin- oder Pyrimidingruppen substituiert sind sowie deren Verwendung in phosphoreszierenden organischen Elektrolumineszenzvorrichtungen.

Allerdings besteht bei Verwendung dieser Materialien ebenso wie bei anderen Materialien noch Verbesserungsbedarf, insbesondere in Bezug auf die Effizienz, Betriebsspannung und die Lebensdauer organischer elektronischer Vorrichtungen.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Überraschend wurde gefunden, dass bestimmte, unten näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Ausfürhungsformen sind daher Gegenstand der vorliegenden Erfindung. Gegenstand der vorliegenden Erfindung ist eine chemische Verbindung, gemäß Formel (1)

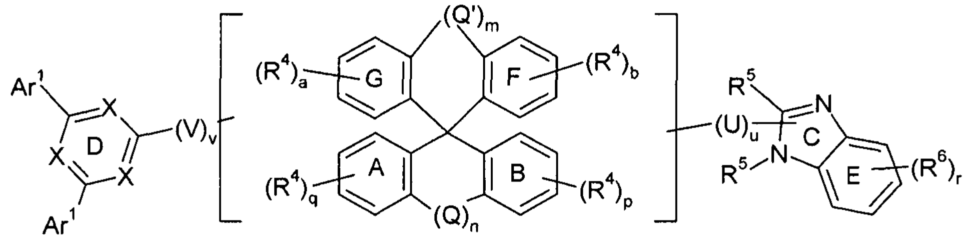

Formel (1)

wobei die Indizes und Symbole wie in Anspruch 1 definiert sind.

Gemäß Formel (1) kann der Ring D, beispielsweise, über V an den Ring A gebunden sein, wohingegen der Ring C über U an eine andere, beliebige Stelle binden kann.

Bevorzugt ist, wenn der Ring D über V an einen der angegebenen Ringe A, B, C, E, F oder G kovalent bindet und wenn der Ring C über U an einen der angegebenen Ringe A, B, D, F oder G kovalent bindet, wobei wenigstens einer der beiden Ringe C und D über die bivalente Gruppe U bzw. V an einen der Ringe A, B, F oder G kovalent gebunden ist;

Der Substituent enthaltend den Ring C bindet dabei gemäß einer der beiden im Folgenden dargestellten Formeln und ersetzt somit jeweils einen der Reste $R^5$ am Ring C.

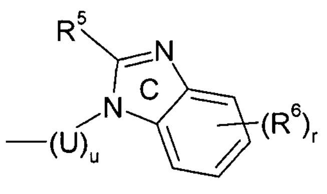

Formel (2)

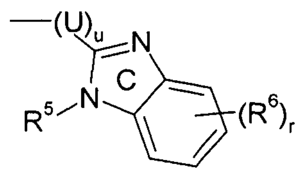

Formel (3)

Es ist bevorzugt, wenn V und U, gleich oder verschieden bei jedem Auftreten, ein aromatisches oder heteroaromatisches Ringsystem darstellen, das 5 bis 60 Ringatome umfasst. Ganz bevorzugt ist, wenn es sich bei U und V um eine Phenylen-, Biphenylen- oder Terphenylengruppe, ganz besonders bevorzugt um eine Phenylen- oder Biphenylengruppe und insbesondere bevorzugt um eine Phenylengruppe handelt.

Bevorzugt ist, wenn der Ring D über die Brücke V direkt an den Ring A bindet, wobei q dann maximal 3 sein kann. Sofern der Ring C über die Brücke U ebenso direkt an A bindet, ist q maximal 2. Ferner versteht es sich von selbst, dass p maximal 3 sein kann, wenn der Ring C über die Brücke U direkt an den Ring B bindet. Ganz analog hängen die maximalen Werte für die Parameter a und b von der Substitution der Ringen G und F mit den Gruppen der Formeln (G-2) und (G-3) ab.

Die Schreibweise für die Verbindung der Formel (1) bedeutet demnach auch, dass, beispielsweise, die Gruppe enthaltend den Ring D zunächst an Ring A des Grundkörpers bindet und die Gruppe enthaltend den Ring C an die Gruppe enthaltend den Ring D binden kann.

Weiter bevorzugt ist eine Verbindung der allgemeinen Formel (4)

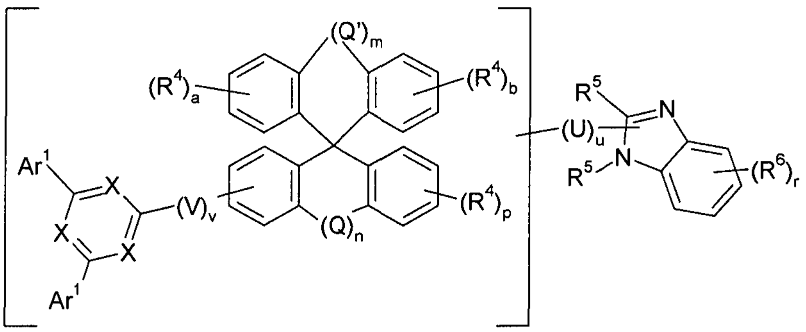

Formel (4)

wobei für die verwendeten Symbole und Indizes obige Definitionen gelten und deren bevorzugte Ausführungsformen auch bevorzugte Ausführungsformen für die Verbindung der Formen (4) darstellen.

Ferner bevorzugt ist eine Verbindung der allgemeinen Formel (5)

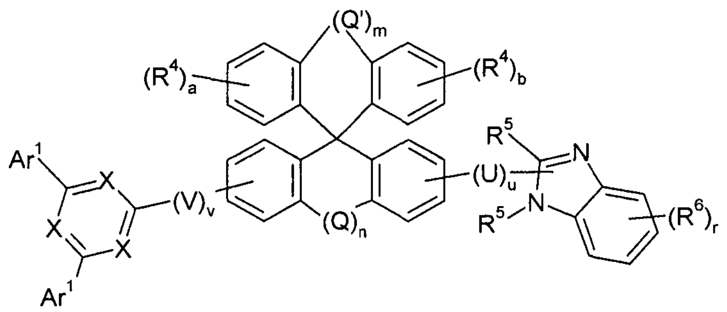

Formel (5)

wobei für die verwendeten Symbole und Indizes obige Definitionen gelten und deren bevorzugte Ausführungsformen auch bevorzugte Ausführungsformen für die Verbindung der Formen (5) darstellen.

Weiterhin bevorzugt ist eine Verbindung der allgemeinen Formel (6)

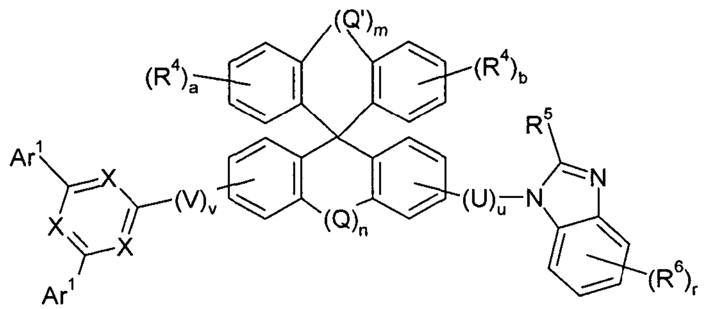

Formel (6)

wobei für die verwendeten Symbole und Indizes obige Definitionen gelten und deren bevorzugte Ausführungsformen auch bevorzugte Ausführungsformen für die Verbindung der Formen (6) darstellen.

Weiterhin bevorzugt ist eine Verbindung der allgemeinen Formel (7)

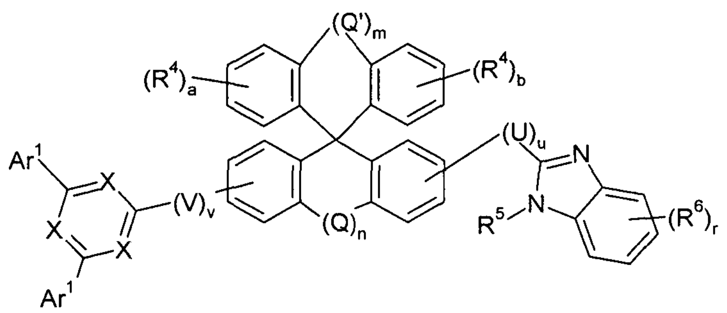

Formel (7)

wobei für die verwendeten Symbole und Indizes obige Definitionen gelten und deren bevorzugte Ausführungsformen auch bevorzugte Ausführungsformen für die Verbindung der Formen (7) darstellen.

Ganz bevorzugt ist eine Verbindung der folgenden allgemeinen Formeln (8) bis (10), wobei für die verwendeten Symbole und Indizes obige Definitionen gelten und deren bevorzugte Ausführungsformen auch bevorzugte Ausführungsformen für die Verbindung der Formeln (8) bis (10) darstellen,

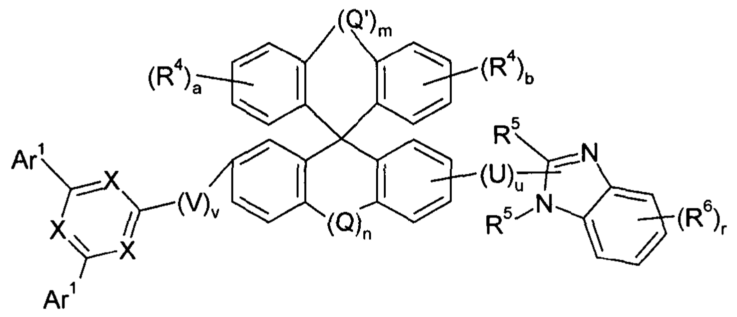

Formel (8)

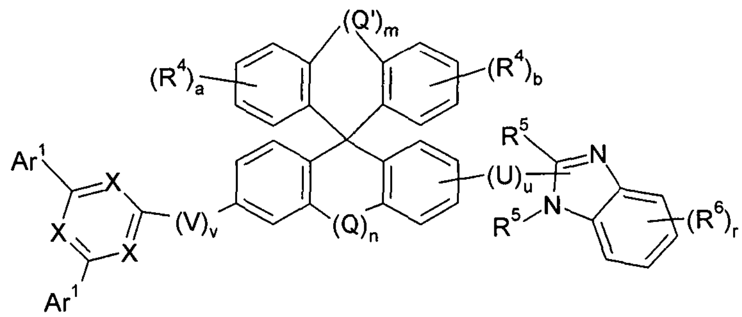

Formel (9)

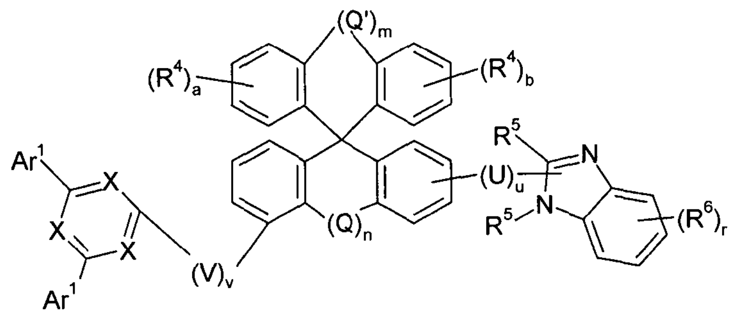

Formel (10)

wobei von den Verbindungen der allgemeinen Formeln (8) bis (10), die der Formeln (8) und (10) besonders bevorzugt sind.

Besonders bevorzugt ist eine Verbindung der folgenden allgemeinen Formel (11), wobei für die verwendeten Symbole und Indizes obige Definitionen gelten und deren bevorzugte Ausführungsformen auch bevorzugte Ausführungsformen für die Verbindung der Formel (11) darstellen.

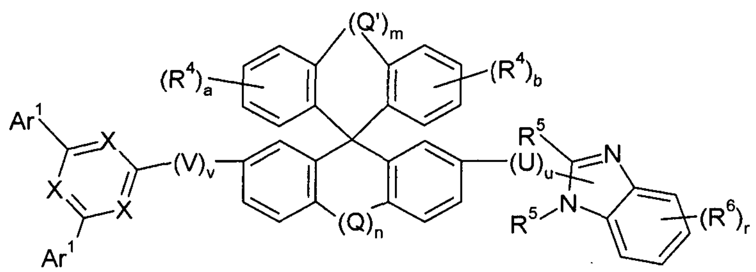

Formel (11)

Ganz besonders bevorzugt ist eine Verbindung der folgenden allgemeinen Formeln (12) und (13), wobei für die verwendeten Symbole und Indizes obige Definitionen gelten und deren bevorzugte Ausführungsformen auch bevorzugte Ausführungsformen für die Verbindung der Formeln (12) und (13) darstellen.

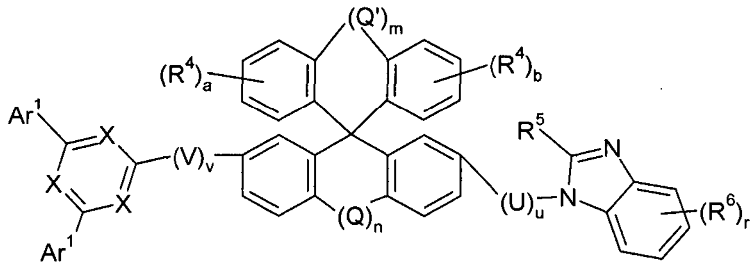

Formel (12)

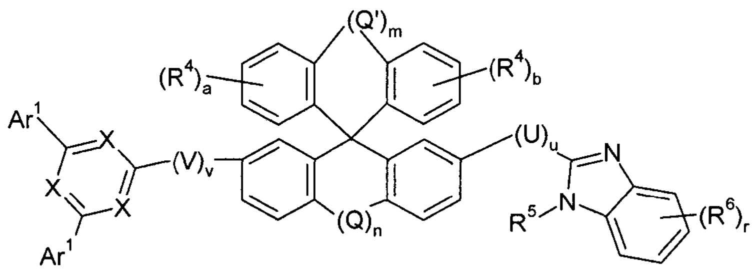

Formel (13)

In einer weiteren, bevorzugten Ausführungsform weist die Verbindung die allgemeine Formel (14) auf

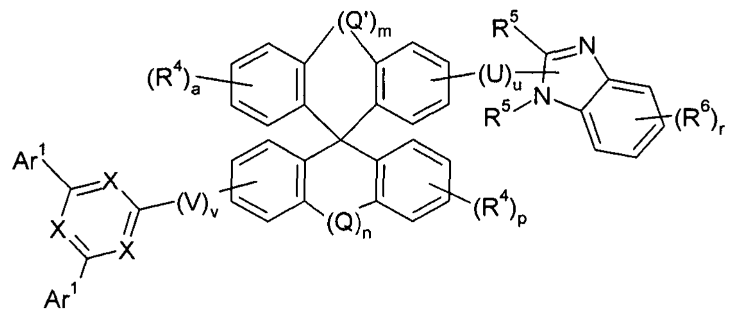

Formel (14)

wobei für die verwendeten Symbole und Indizes obige Definitionen gelten und deren bevorzugte Ausführungsformen auch bevorzugte Ausführungsformen für die Verbindung der Formen (14) darstellen.

Weiterhin bevorzugt ist eine Verbindung der allgemeinen Formel (15)

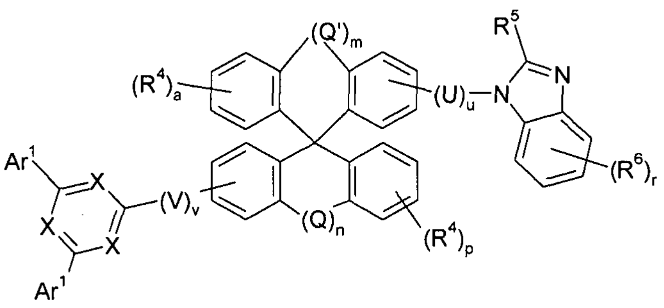

Formel (15)

wobei für die verwendeten Symbole und Indizes obige Definitionen gelten und deren bevorzugte Ausführungsformen auch bevorzugte Ausführungsformen für die Verbindung der Formen (15) darstellen.

Weiterhin bevorzugt ist eine Verbindung der allgemeinen Formel (16)

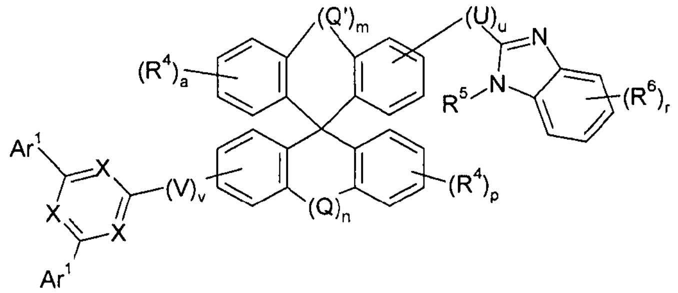

Formel (16)

wobei für die verwendeten Symbole und Indizes obige Definitionen gelten und deren bevorzugte Ausführungsformen auch bevorzugte Ausführungsformen für die Verbindung der Formen (16) darstellen.

Ganz bevorzugt ist eine Verbindung der folgenden allgemeinen Formeln (17) bis (19), wobei für die verwendeten Symbole und Indizes obige Definitionen gelten und deren bevorzugte Ausführungsformen auch bevorzugte Ausführungsformen für die Verbindung der Formeln (17) bis (19) darstellen,

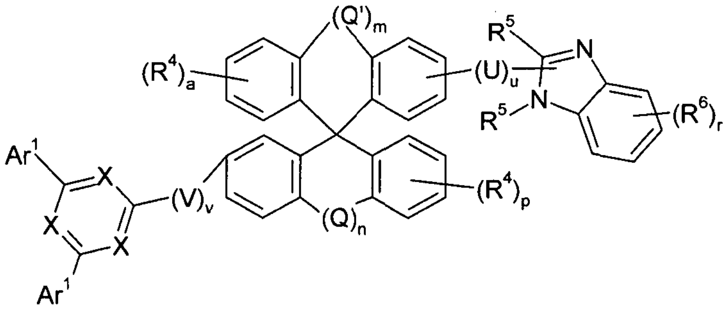

Formel (17)

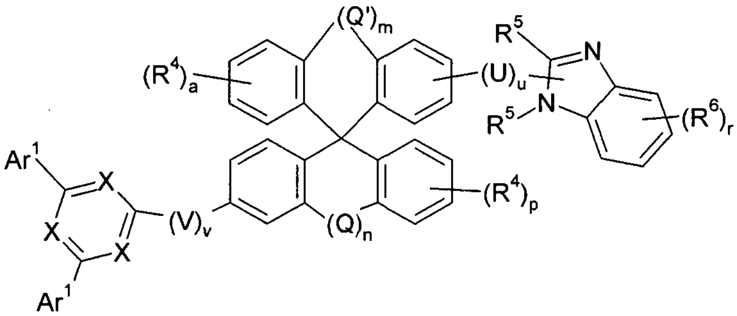

Formel (18)

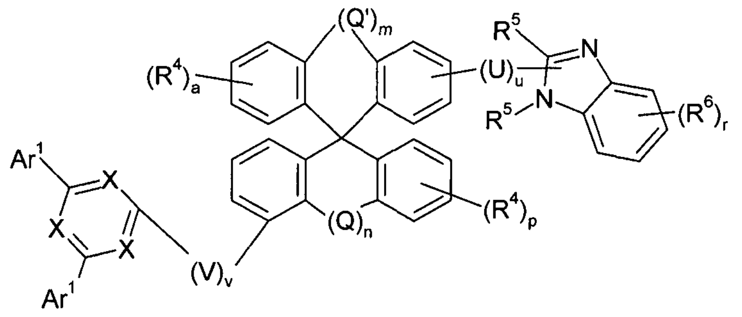

Formel (19)

wobei von den Verbindungen der allgemeinen Formeln (17) bis (19), die der Formel (17) und (19) besonders bevorzugt ist.

Besonders bevorzugt ist eine Verbindung der folgenden allgemeinen Formel (20), wobei für die verwendeten Symbole und Indizes obige Definitionen gelten und deren bevorzugte Ausführungsformen auch bevorzugte Ausführungsformen für die Verbindung der Formel (20) darstellen.

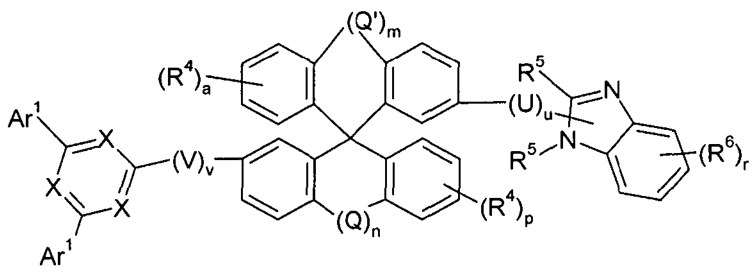

Formel (20)

Ganz besonders bevorzugt ist eine Verbindung der folgenden allgemeinen Formeln (21) und (22), wobei für die verwendeten Symbole und Indizes obige Definitionen gelten und deren bevorzugte Ausführungsformen auch bevorzugte Ausführungsformen für die Verbindung der Formeln (21) und (22) darstellen.

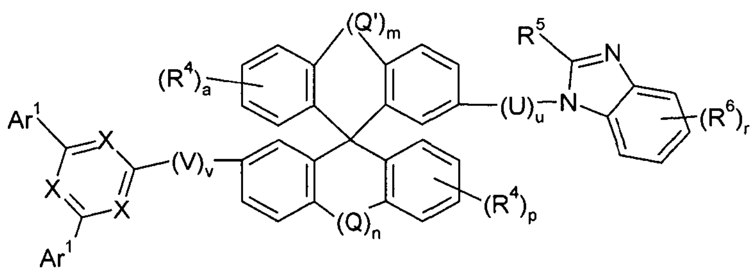

Formel (21)

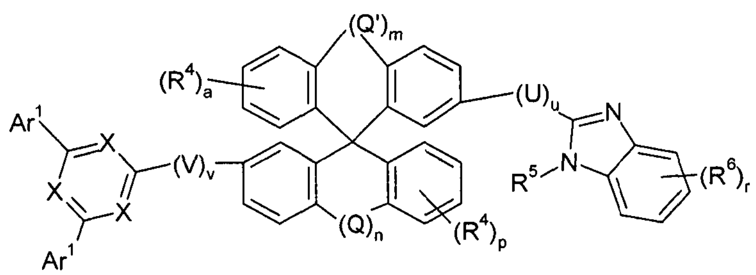

Formel (22)

In einer weiteren, bevorzugten Ausführungsform weist die Verbindung die allgemeine Formel (23) auf

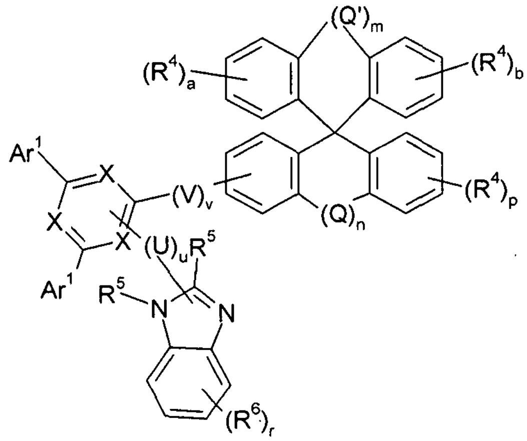

Formel (23)

wobei für die verwendeten Symbole und Indizes obige Definitionen gelten und deren bevorzugte Ausführungsformen auch bevorzugte Ausführungsformen für die Verbindung der Formel (23) darstellen.

Ferner bevorzugt ist eine Verbindung der allgemeinen Formel (23a)

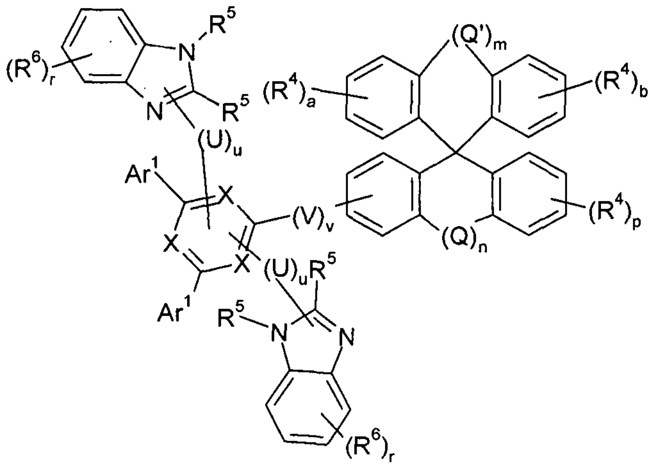

Formel (23a)

wobei für die verwendeten Symbole und Indizes obige Definitionen gelten und deren bevorzugte Ausführungsformen auch bevorzugte Ausführungsformen für die Verbindung der Formel (23a) darstellen.

Ganz bevorzugt ist dabei eine Verbindung der allgemeinen Formel (24), wobei für die verwendeten Symbole und Indizes obige Definitionen gelten und deren bevorzugte Ausführungsformen auch bevorzugte Ausführungsformen für die Verbindung der Formel (24) darstellen.

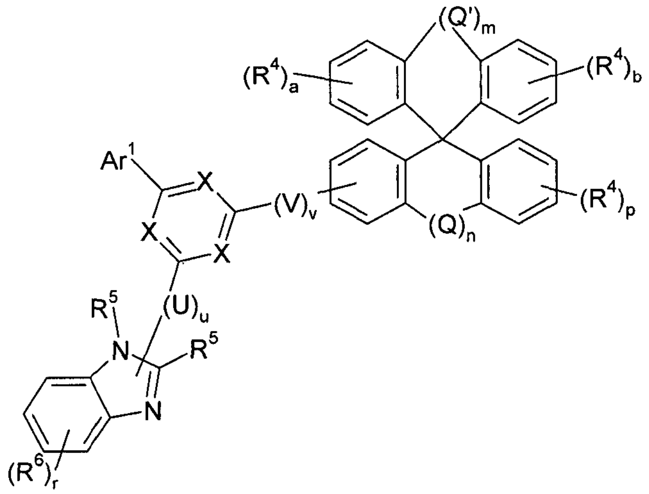

Formel (24)

Ferner ganz bevorzugt ist dabei eine Verbindung der allgemeinen Formel (24a), wobei für die verwendeten Symbole und Indizes obige Definitionen gelten und deren bevorzugte Ausführungsformen auch bevorzugte Ausführungsformen für die Verbindung der Formel (24a) darstellen.

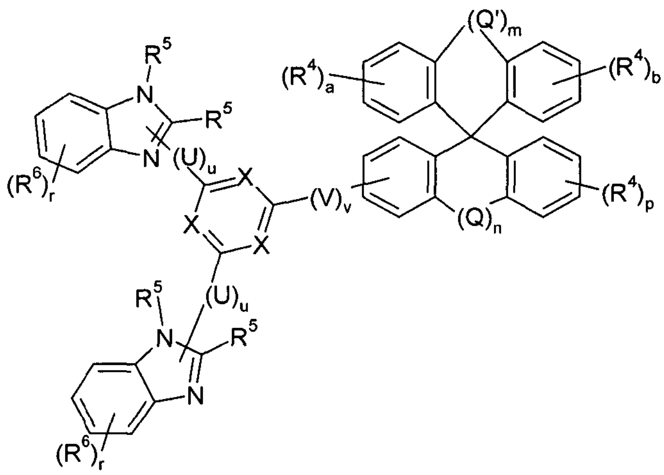

Formel (24a)

Besonders bevorzugt ist dabei eine Verbindung der allgemeinen Formeln (25) und (26), wobei für die verwendeten Symbole und Indizes obige Definitionen gelten und deren bevorzugte Ausführungsformen auch bevorzugte Ausführungsformen für die Verbindung der Formeln (25) und (26) darstellen.

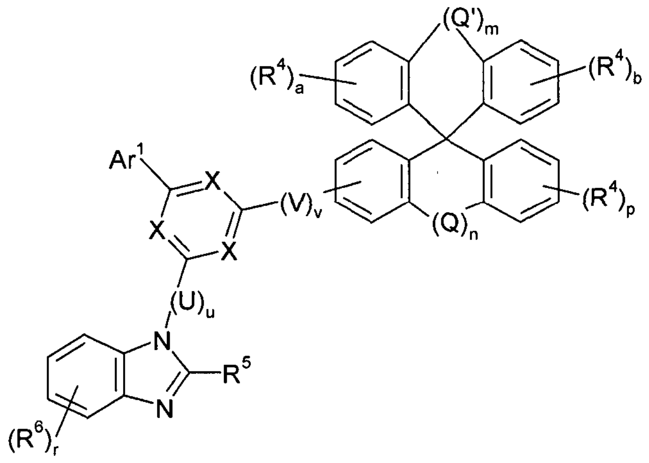

Formel (25)

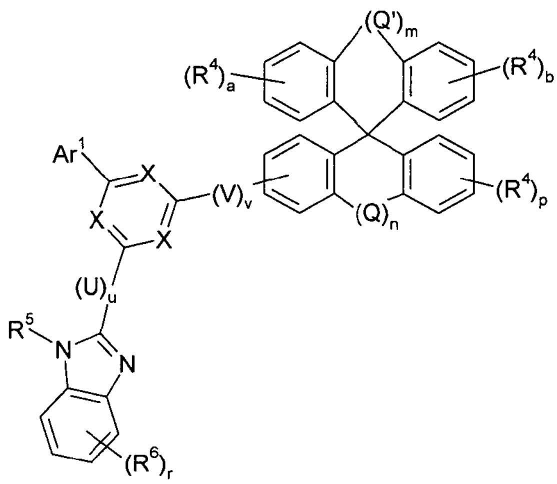

Formel (26)

Ganz besonders bevorzugt ist dabei eine Verbindung der allgemeinen Formeln (27) bis (29), wobei für die verwendeten Symbole und Indizes obige Definitionen gelten und deren bevorzugte Ausführungsformen auch bevorzugte Ausführungsformen für die Verbindung der Formeln (27) bis (29) darstellen.

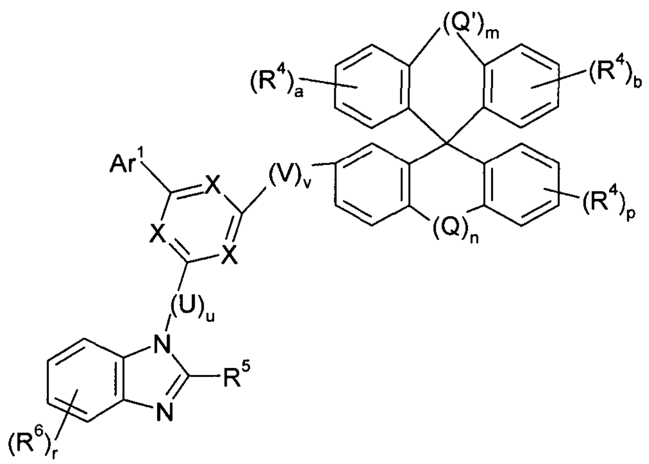

Formel (27)

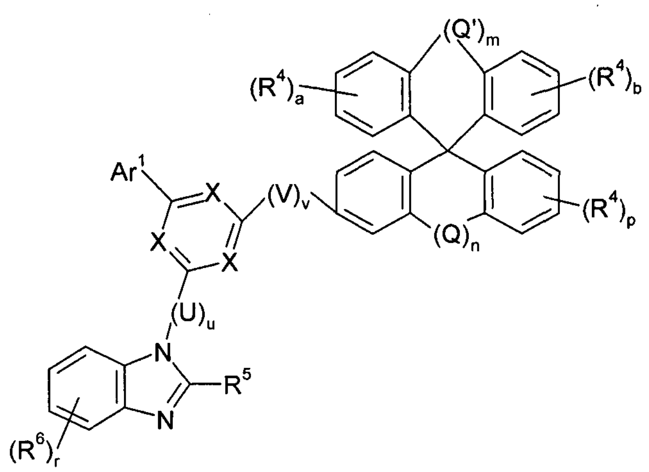

Formel (28)

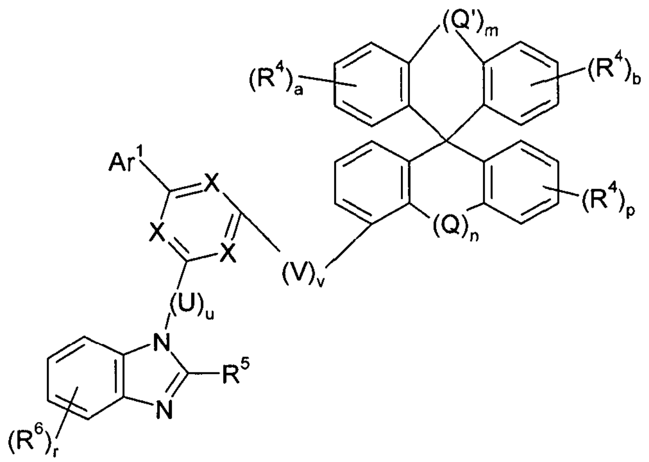

Formel (29)

Ferner ganz besonders bevorzugt ist dabei eine Verbindung der allgemeinen Formeln (30) bis (32), wobei für die verwendeten Symbole und Indizes obige Definitionen gelten und deren bevorzugte Ausführungsformen auch bevorzugte Ausführungsformen für die Verbindung der Formeln (30) bis (32) darstellen.

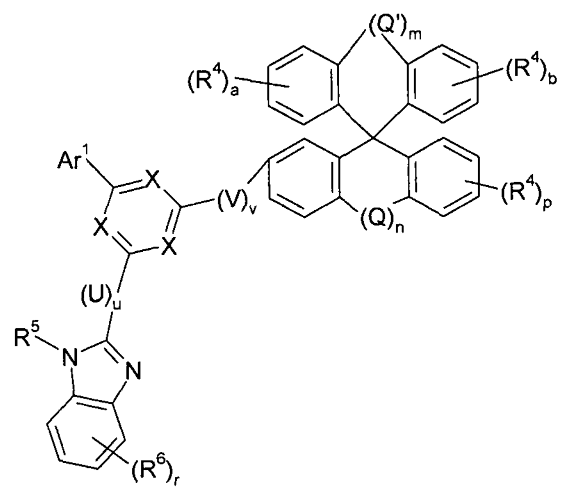

Formel (30)

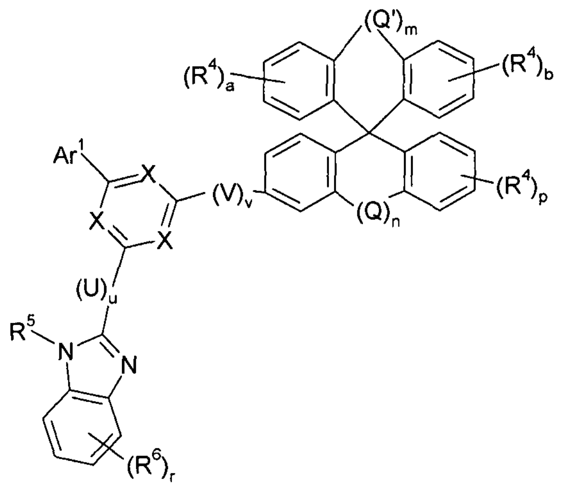

Formel (31)

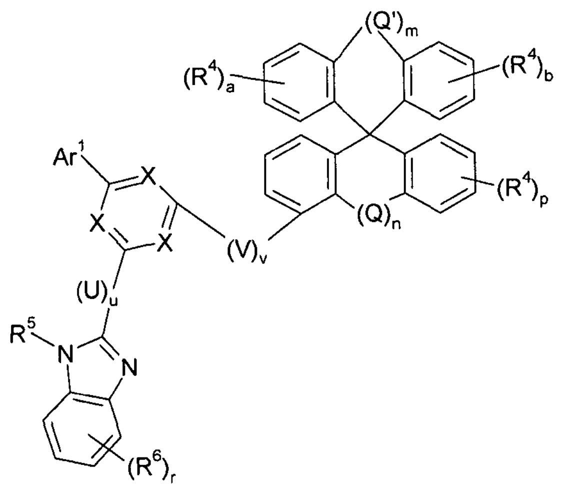

Formel (32)

Insbesondere bevorzugt ist, wenn in den Verbindungen der oben genannten Formeln entweder n oder m gleich 0 sind, und noch bevorzugter ist, wenn sowohl n als auch m gleich 0 sind.

Am meisten bevorzugt im Sinne der vorliegenden Erfindung ist, wenn a, b, q und p gleich 0 sind sowie u und v unabhängig voneinander entweder 0 oder 1 sind, wobei sofern v gleich 1 ist, die Gruppe V eine Phenylengruppe ist und sofern u gleich 1 ist, die Gruppe U eine Phenylengruppe ist.

Es ist ferner bevorzugt, wenn der Rest $R^1$ bei bei jedem Auftreten gleich oder verschieden eine geradkettige Alkyl-, Alkoxy- oder Thioalkoxygruppe mit 1 bis 40 C-Atomen oder eine geradkettige Alkenyl-oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Kombination dieser Systeme.

Ganz bevorzugt ist, wenn der Rest $R^1$ bei bei jedem Auftreten gleich oder verschieden eine geradkettige Alkylgruppe mit 1 bis 40 C-Atomen oder eine verzweigte oder cyclische Alkylgruppe mit 3 bis 40 C-Atomen, die jeweils mit einem oder mehreren Resten $R^2$ substituiert sein kann, oder ein aromatisches oder heteroaromatisches Ringsystem mit 5 bis 60 aromatischen Ringatomen, das jeweils durch einen oder mehrere Reste $R^2$ substituiert sein kann, ist.

Besonders bevorzugt ist, wenn der Rest $R^1$ bei bei jedem Auftreten gleich oder verschieden eine geradkettige Alkylgruppe mit 1 bis 40 C-Atomen, die jeweils mit einem oder mehreren Resten $R^2$ substituiert sein kann, oder ein aromatisches oder heteroaromatisches Ringsystem mit 5 bis 60 aromatischen Ringatomen, das jeweils durch einen oder mehrere Reste $R^2$ substituiert sein kann, ist.

Ganz besonders bevorzugt ist, wenn der Rest $R^1$ bei bei jedem Auftreten gleich oder verschieden eine geradkettige Alkylgruppe mit 1 bis 40 C-Atomen ist, wobei insbesondere bevorzugt ist, wenn der $R^1$ bei bei jedem Auftreten gleich oder verschieden eine geradkettige Alkylgruppe mit 1 bis 15 C-Atomen ist.

Ferner bevorzugt ist, wenn $R^5$ bei jedem Auftreten gleich oder verschieden eine geradkettige Alkyl-, Alkoxy- oder Thioalkoxygruppe mit 1 bis 40 C-Atomen oder eine geradkettige Alkenyl-oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Kombination dieser Systeme; wobei zwei oder mehrere benachbarte Substituenten $R^5$ miteinander kein mono- oder polycyclisches, aliphatisches oder aromatisches Ringsystem bilden können.

Ganz bevorzugt ist, wenn $R^5$ bei jedem Auftreten gleich oder verschieden eine geradkettige Alkylgruppe mit 1 bis 40 C-Atomen oder eine verzweigte oder cyclische Alkylgruppe mit 3 bis 40 C-Atomen, die jeweils mit einem oder mehreren Resten $R^2$ substituiert sein kann, oder ein aromatisches oder heteroaromatisches Ringsystem mit 5 bis 60 aromatischen Ringatomen, das jeweils durch einen oder mehrere Reste $R^2$ substituiert sein kann, wobei zwei oder mehrere benachbarte Substituenten $R^5$ miteinander kein mono- oder polycyclisches, aliphatisches oder aromatisches Ringsystem bilden können.

Besonders bevorzugt ist, wenn $R^5$ bei jedem Auftreten gleich oder verschieden eine geradkettige Alkylgruppe mit 1 bis 40 C-Atomen, die jeweils mit einem oder mehreren Resten $R^2$ substituiert sein kann, oder ein aromatisches oder heteroaromatisches Ringsystem mit 5 bis 60 aromatischen Ringatomen, das jeweils durch einen oder mehrere Reste $R^2$ substituiert sein kann, wobei zwei oder mehrere benachbarte Substituenten $R^5$ miteinander kein mono- oder polycyclisches, aliphatisches oder aromatisches Ringsystem bilden können.

Ganz besonders bevorzugt ist, wenn $R^5$ bei jedem Auftreten gleich oder verschieden eine geradkettige Alkylgruppe mit 1 bis 40 C-Atomen oder ein aromatisches Ringsystem mit 5 bis 60 aromatischen Ringatomen, das jeweils durch einen oder mehrere Reste $R^2$ substituiert sein kann, wobei zwei oder mehrere benachbarte Substituenten $R^5$ miteinander kein mono- oder polycyclisches, aliphatisches oder aromatisches Ringsystem bilden können. Dabei sind ganz besonders bevorzugte aromatische Ringsysteme ausgewählt aus der Gruppe bestehend aus der Phenyl-. Biphenyl-, Terphenylgruppe.

Ferner bevorzugt ist, wenn $Ar^1$ bei jedem Auftreten gleich oder verschieden ein aromatisches oder heteroaromatisches Ringsystem mit 5 bis 30 aromatischen Ringatomen, das mit einem oder mehreren Resten $R^2$ substituiert sein kann; dabei können auch zwei Reste $Ar^1$ durch eine Einfachbindung oder eine Brücke, ausgewählt aus $C(R^2)_2$, O und S miteinander verknüpft sein; bevorzugt ist, wenn zwei Reste $Ar^1$ nicht miteinander verknüpft sind.

Ganz bevorzugte Gruppen für $Ar^1$ sind ausgewählt aus der Gruppe bestehend aus der Phenyl-, Biphenyl-, Terphenyl-, Quarterphenyl-, Dibenzofuranyl-, Dibezothiophenyl-, Fluorenyl-, Benzimidazolyl-, Carbazolyl-, Indenocarbazolyl-, Indolocarbazolylgruppe, wobei die Gruppen mit einem oder mehreren, gleichen oder verschiedenen Resten $R^2$ substituiert sein können, wobei die genannten Phenylgruppen am meisten bevorzugt sind.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

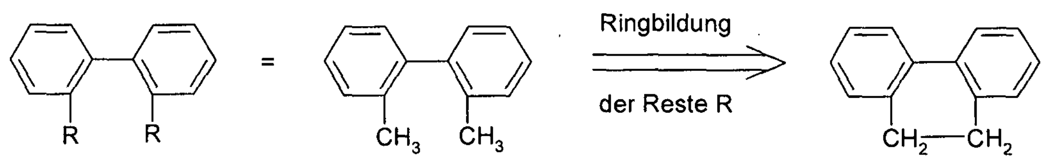


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

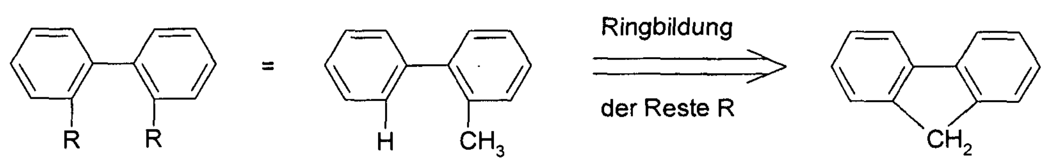


Eine Arylgruppe im Sinne dieser Erfindung enthält mindestens 6 C-Atome; eine Heteroarylgruppe im Sinne dieser Erfindung enthält mindestens 2 C-Atome und mindestens 1 Heteroatom, mit der Maßgabe, dass die Summe aus

C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Pyren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein $sp^3$-hybridisiertes C-, Si-, N- oder O-Atom, ein $sp^2$-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5-60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Näphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder $CH_2$-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Die erfindungsgemäßen Verbindungen können gemäß Schema 1 und 2 dargestellt werden. Die entsprechenden Monoboronsäuren sind kommerziell erhältlich und können durch Suzuki-Kupplung zu den entsprechenden Zielmolekülen umgesetzt werden.

Schema 1

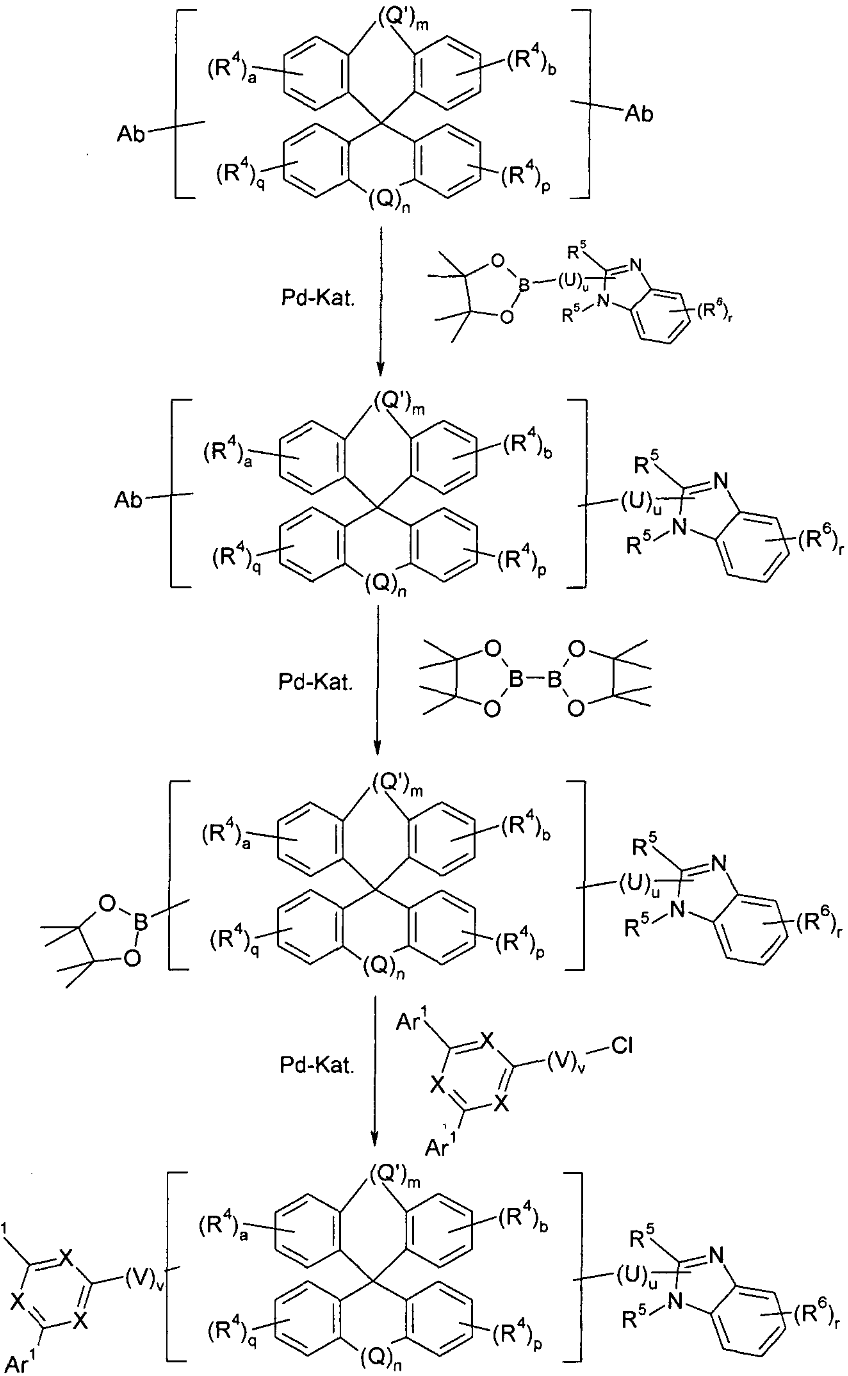


wobei Ab für eine Abgangsgruppe steht, die vorzugsweise ausgewählt ist aus Cl, Br, I und Triflat, ganz bevorzugt ist diese gleich Br. Neben Bor-Pinakolester können dabei auch andere Ester oder Boronsöure selbst eingesetzt werden. Diese sind dem Fachmann gut geläufig.

Schema 2

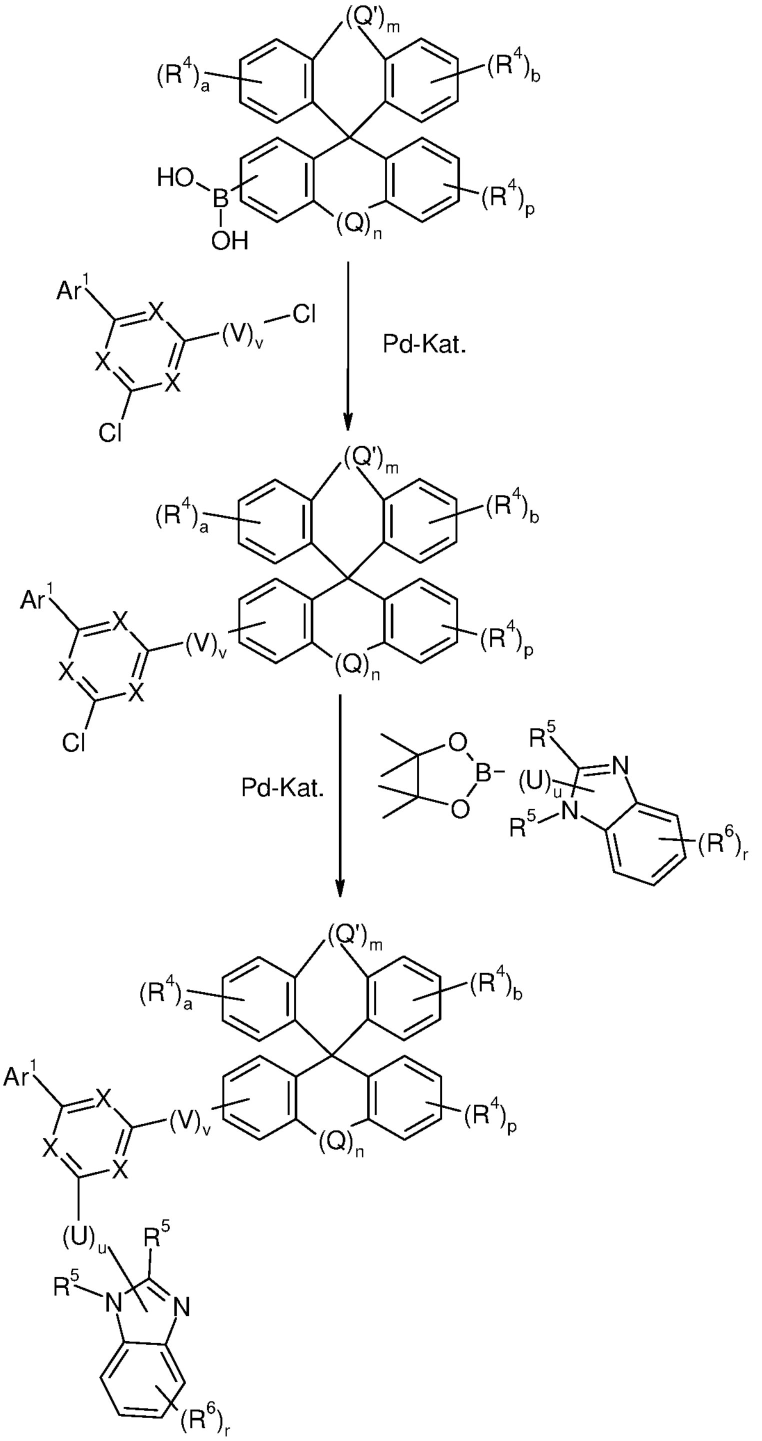


Fluorene können ganz analog durch Suzuki-Kupplung hergestellt werden. Hierbei können auch andere Borverbindungen eingesetzte werden. Diese sind dem Fachmann gut geläufig.

Das gezeigte, allgemeine Verfahren zur Synthese der erfindungsgemäßen Verbindungen ist exemplarisch. Der Fachmann kann alternative Synthesewege im Rahmen seines allgemeinen Fachwissens entwickeln.

Die folgende Übersicht enthält eine beispielhafte Darstellung erfindungsgemäßer Verbindungen, die nach einem der hierin beschriebenen Verfahren hergestellt werden können.

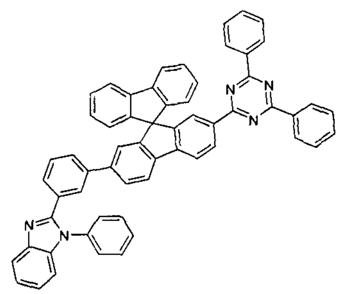

Formel (E-1)

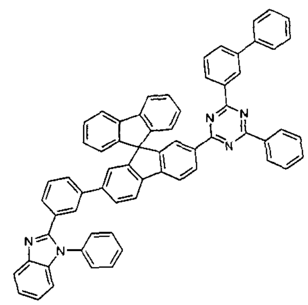

Formel (E-2)

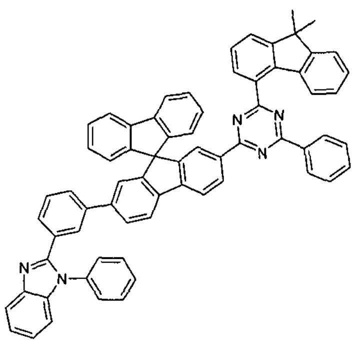

Formel (E-3)

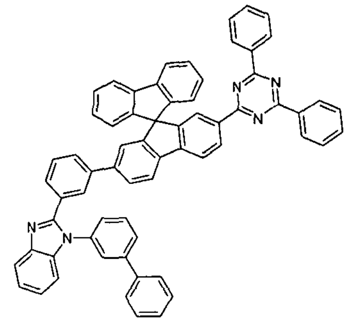

Formel (E-4)

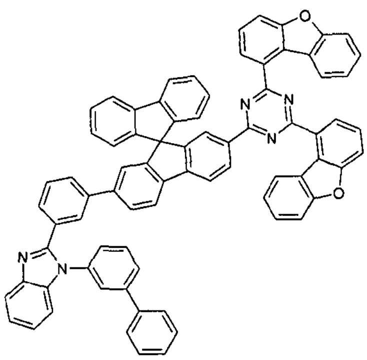

Formel (E-5)

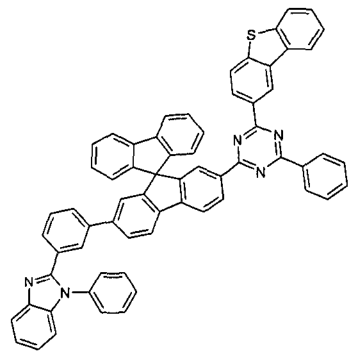

Formel (E-6)

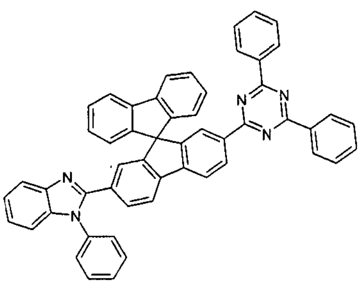

Formel (E-7)

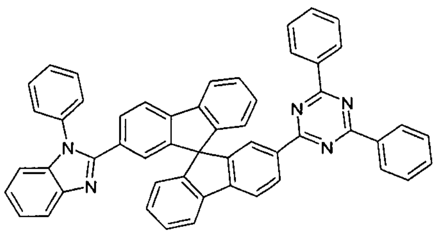

Formel (E-8)

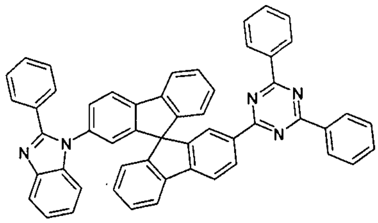

Formel (E-9)

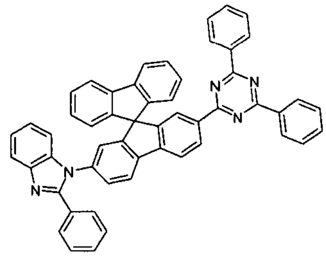

Formel (E-10)

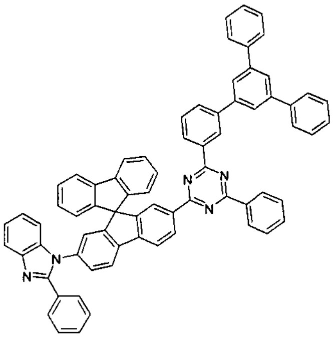

Formel (E-11)

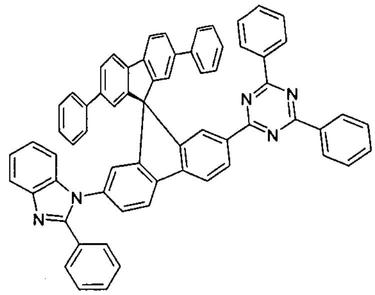

Formel (E-12)

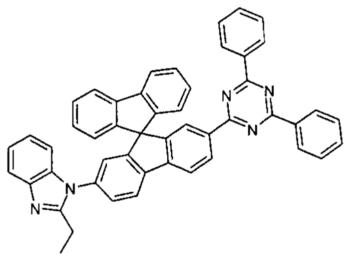

Formel (E-13)

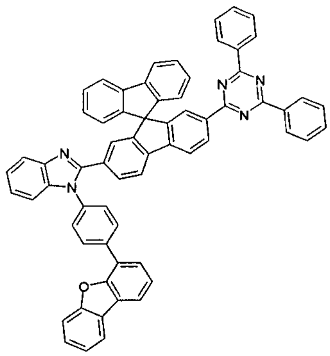

Formel (E-14)

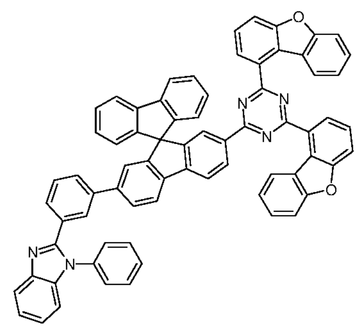

Formel (E-15)

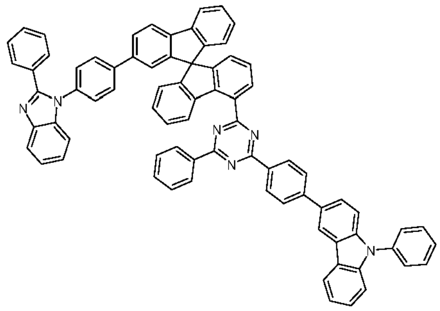

Formel (E-29)

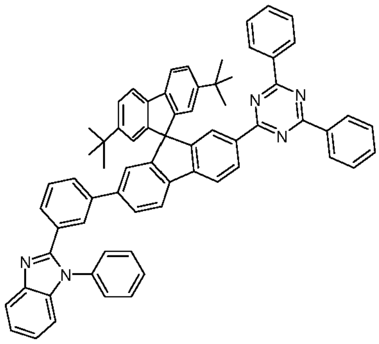

Formel (E-46)

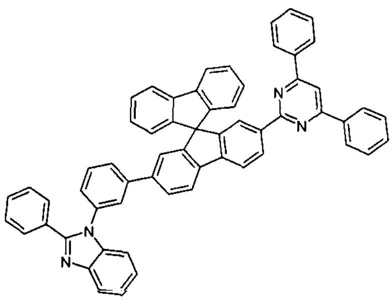

Formel (E-49)

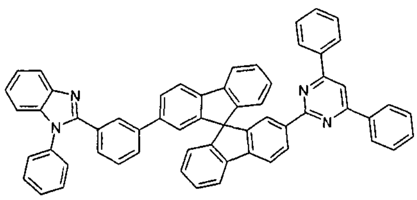

Formel (E-50)

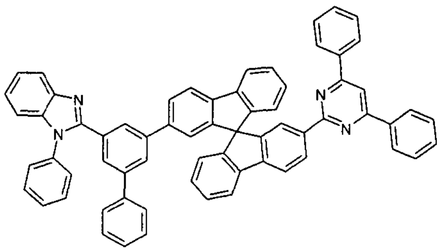

Formel (E-51)

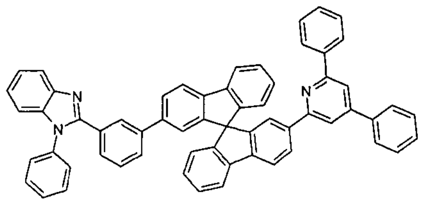

Formel (E-52)

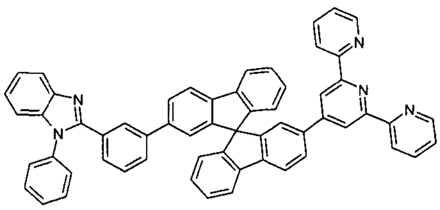

Formel (E-53)

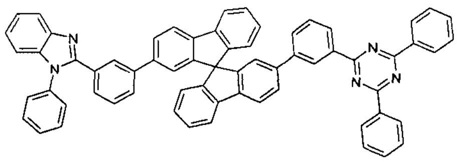

Formel (E-54)

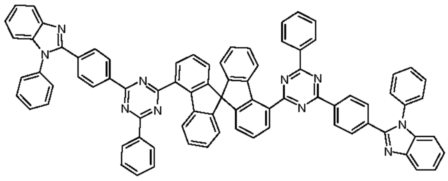

Formel (E-59)

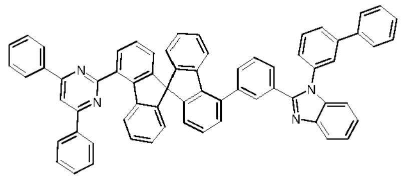

Formel (E-60)

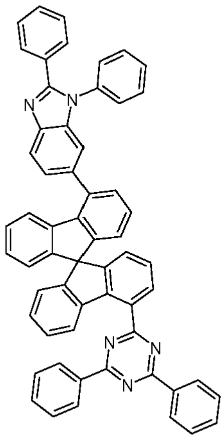

Formel (E-62)

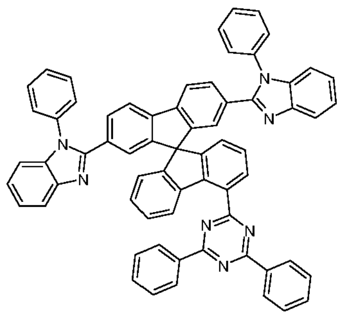

Formel (E-70)

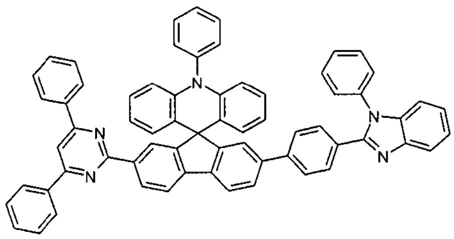

Formel (E-83)

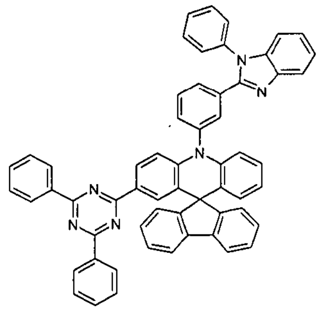

Formel (E-84)

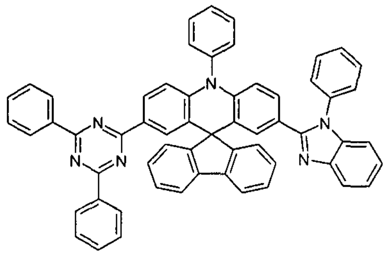

Formel (E-85)

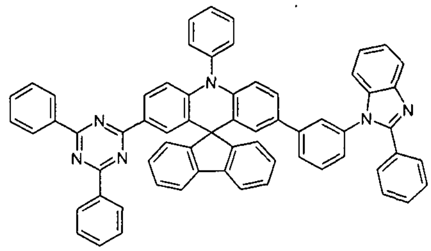

Formel (E-86)

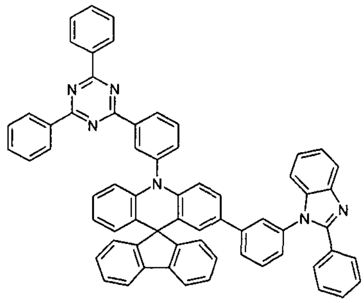

Formel (E-87)

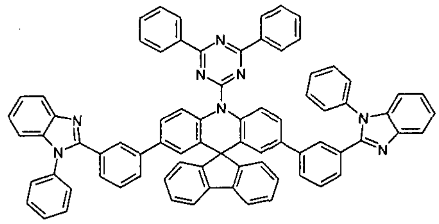

Formel (E-88)

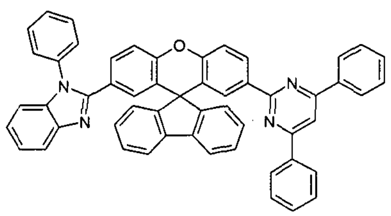

Formel (E-89)

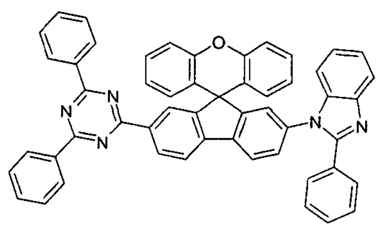

Formel (E-90)

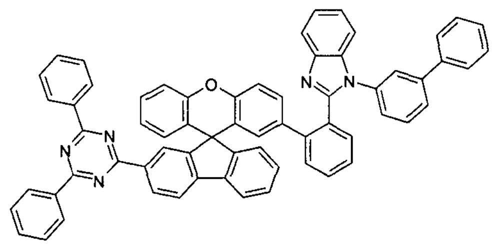

Formel (E-91)

Die erfindungsgemäßen Verbindungen können in organischen elektronischen Vorrichtungen verwendet werden. Dabei werden die erfindungsgemäßen Verbindungen in elektroneninjizierenden, elektronentransportierenden und/oder in emittierenden Schichten eingesetzt.

Ein weiterer Gegenstand der vorliegenden Erfindung ist daher die Verwendung einer erfindungsgemäßen Verbindung in einer elektronischen Vorrichtung, bevorzugt in einer elektroneninjezierenden, elektronentransportierenden und/oder in einer emittierenden Schicht.

Die erfindungsgemäßen Verbindungen können dabei die einzige Komponente einer Schicht sein oder in Kombination mit einem oder mehreren anderen Materialien in einer Schicht verwendet werden.

Dabei können weitere Verbesserungen von Effizienzdaten von elektronischen Vorrichtungen enthaltend die Verbindungen erreicht werden, wenn die erfindungsgemäßen Verbindungen in Zusammensetzungen mit anderen Materialen eingesetzt werden.

Bevorzugt werden die erfindungsgemäßen Verbindungen in Zusammensetzung mit Materialien eingesetzt, die typischerweise in Vorrichtungen, insbesondere elektronischen Vorrichtungen wie Elektrolumineszenzvorrichtungen, eingesetzt werden.

Die vorliegende Erfindung betrifft daher auch eine Zusammensetzung enthaltend eine oder mehrere erfindungsgemäße Verbindungen sowie mindestens ein zusätzliches funktionelles Molekül ausgewählt aus der Gruppe bestehend aus fluoreszierenden Emittern, phosphoreszierenden Emittern, Matrix-Materialien, Elektronentransportmaterialien, Elektroneninjektionsmaterialien, Lochleitermaterialien, Lochinjektionsmaterialien, Elektronenblockiermaterialien, Lochblockiermaterialien und n-Dotanden.

In der Literatur wird häufig auch der Begriff Host-Material anstelle des Begriffs Matrix-Material verwendet. Einige Autoren verwenden den Begriff Host-Material für Matrix-Materialien für fluoreszierende Emitter, wohingegen der Begriff Matrix-Material für solche Materialien steht, die zusammen mit phosphoreszierenden Emittern in der Emissionsschicht verwendet werden. Vorliegend wird der Begriff Matrix-Material (Host-material, Wirtsmaterial) unabhängig von dem Emitter verwendet und kennzeichnet ein Material das in der Emissionschicht mit fluoreszierenden oder phosphoreszierenden Emittern eingesetzt werden kann.

Dem Fachmann sind die einzelnen Materialien gut bekannt und es bereitet ihm keinerlei Schwierigkeiten aus einem großen Repertoire geeignete Verbindungen auszuwählen.

In einer bevorzugten Ausführungsform der vorliegenden Erfindung enthält die organische elektronische Vorrichtung eine oder mehrere der erfindungsgemäßen Verbindungen in der Elektronentransportschicht (ETL). Dabei kann die ETL eine Reinschicht bestehend aus der erfindungsgemäßen Verbindung sein oder sie kann noch wenigstens ein Elektronentransportmaterial enthalten. Dabei kann der Fachmann aus einer Vielzahl bekannter Elektronentransportmaterialien auswählen. Die kombinierte Verwendung der erfindungsgemäßen Verbindung mit einem Elektronentransportmaterial in der ETL führt zu organischen elektronischen Vorrichtungen mit besonders guten Leistungsdaten.

Die vorliegende Erfindung betrifft daher eine Zusammensetzung enthaltend wenigstens eine der erfindungsgemäßen Verbindungen sowie wenigstens ein ETM. Das ETM ist vorzugsweise ausgewählt aus der Gruppe der nichtmetallischen Elektronentransportmaterialien.

Bevorzugte Elektronentransportmaterialien für die erfindungsgemäße Zusammensetzungsind ausgewählt aus den Pyridinen, Pyrimidinen, Pyridazinen, Pyrazinen, Oxadiazolen, Chinolinen, Chinoxalinen, Anthracenen, Benzanthracenen, Pyrenen, Perylenen, Benzimidazolen, Triazinen, Ketonen, Lactamen, Oxazolen, Phenanthrolinen, Phosphinoxiden und Phenazinen. Ganz bevorzugt sind dabei die Triazine und Lactame.

Die Schichtdicke der ETL beträgt vorzugsweise 5 bis 150 nm.

Die Konzentration der erfindungsgemäßen Verbindung in der ETL liegt bevorzugt im Bereich von 10 Vol-% bis 90 Vol-%, ganz bevorzugt im

Bereich von 20 Vol-% bis 80 Vol-%, ganz besonders bevorzugt im Bereich von 30 Vol-% bis 70 Vol-% und insbesondere bevorzugt im Bereich von 40 Vol-% bis 60 Vol-% bezogen auf die gesamte Elektronentransportschicht.

Zusätzlich zur Elektronentransportschicht kann die erfindungsgemäße Vorrichtung auch noch eine Elektroneninjektionsschicht (EIL) aufweisen, die sich zwischen der Kathode und der Elektronentransportschicht befindet. Bevorzugte Materialien, die als Elektroneninjektionsmaterial (EIM) in der EIL eingesetzt werden sind ausgewählt aus der Gruppe der Alkalimetalle, Alkalimetallkomplexe, insbesondere der Alkali-Oxinate (insbesondere substituierte oder unsubstituierte Li-Hydroxychinoline), sowie der Alkalimetall- oder Erdalkalimetallfluoride, und auch deren Oxide oder Carbonate. Ganz bevorzugte Elektroneninjektionsmaterialien sind Li, LiF, $Li_2O$, $BaF_2$, MgO, NaF, Cs, CsF, $Cs_2CO_3$, Liq, wobei LiF und Liq besonders bevorzugt sind und LiF ein ganz besonders bevorzugtes Elektroneninjektionsmaterial darstellt.

Die Schichtdicke der EIL beträgt vorzugsweise 0,5 bis 5 nm.

Weiterhin bevorzugte EIMs sind substituierte Lithium-8-Hydroxychinolinate, insbesondere solche die in DE 102013013876.0 offenbart werden und ganz besonders die der folgenden Formeln (C-1) bis (C-34).

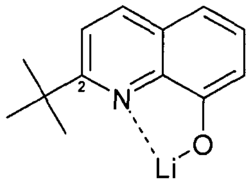

Formel (C-1)

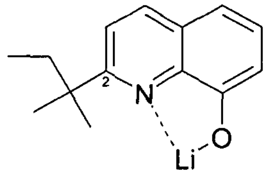

Formel (C-2)

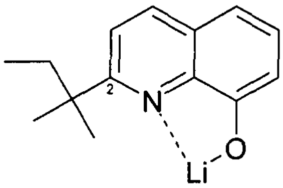

Formel (C-3)

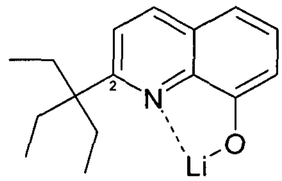

Formel (C-4)

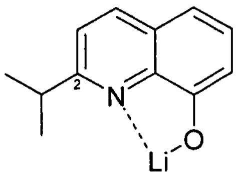

Formel (C-5)

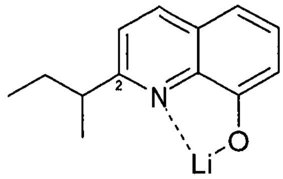

Formel (C-6)

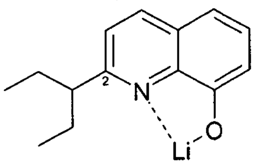

Formel (C-7)

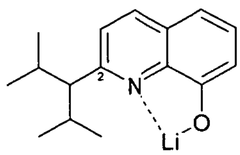

Formel (C-8)

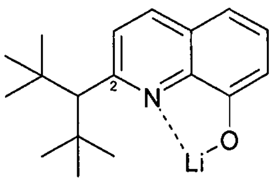

Formel (C-9)

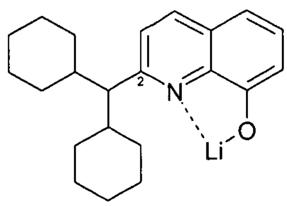

Formel (C-10)

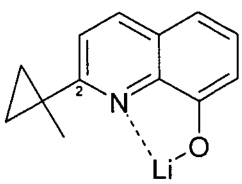

Formel (C-11)

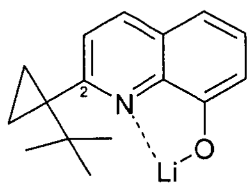

Formel (C-12)

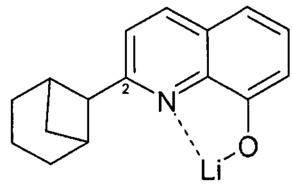

Formel (C-13)

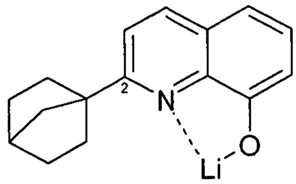

Formel (C-14)

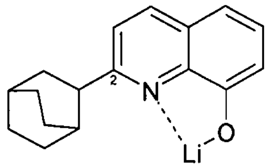

Formel (C-15)

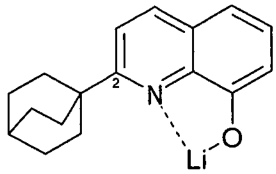

Formel (C-16)

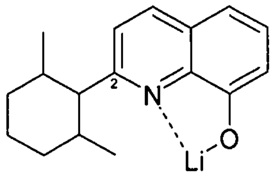

Formel (C-17)

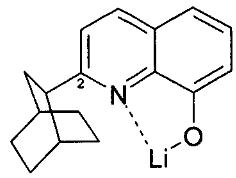

Formel (C-18)

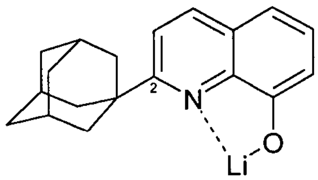

Formel (C-19)

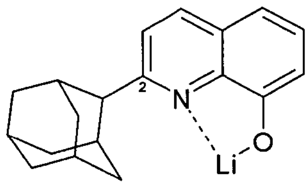

Formel (C-20)

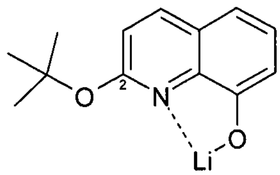

Formel (C-21)

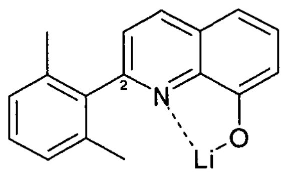

Formel (C-22)

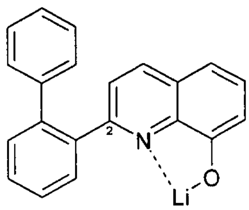

Formel (C-23)

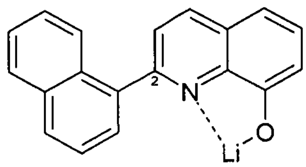

Formel (C-24)

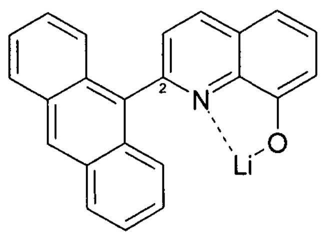

Formel (C-25)

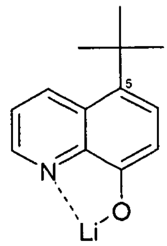

Formel (C-26)

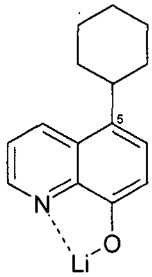

Formel (C-27)

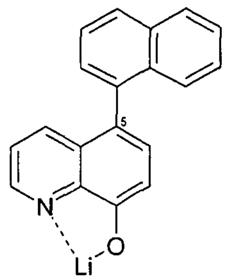

Formel (C-28)

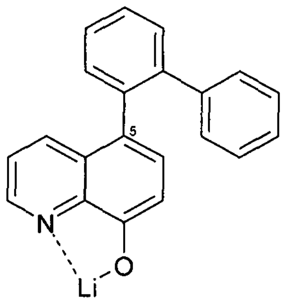

Formel (C-29)

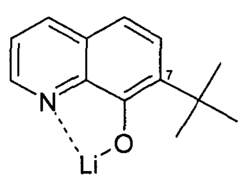

Formel (C-30)

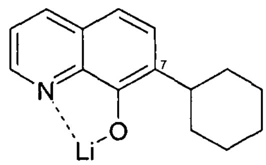

Formel (C-31)

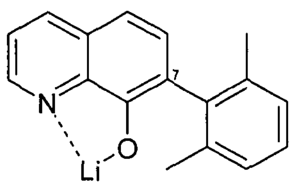

Formel (C-32)

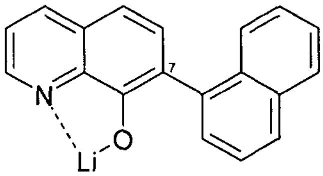

Formel (C-33)

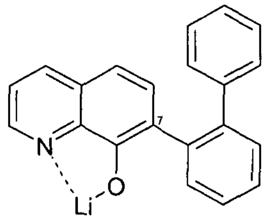

Formel (C-34)

Weitere Leistungssteigerungen organischer elektronischer Vorrichtungen können erzielt werden, wenn die erfindungsgemäße Verbindung in einer Elektronentransportschicht in Kombination mit einem n-Dotanden eingesetzt wird. Der n-Dotand kann dabei sowohl ein anorganisches wie auch ein organisches Material sein.

Unter einem n-Dotanden wird vorliegend eine organische oder anorganische Verbindung verstanden, die in der Lage ist Elektronen abzugeben (Elektronendonator), d.h. eine Verbindung, die als Reduktionsmittel wirkt.

Die zur n-Dotierung eingesetzten Verbindungen können als Precursor eingesetzt werden, wobei diese Precursor-Verbindungen durch Aktivierung n-Dotanden freisetzen.

Bevorzugten sind n-Dotanden ausgewählt aus elektronenreichen Metallkomplexen; P=N-Verbindungen; N-Heterocyclen, besonders bevorzugt, Naphthylencarbodiimide, Pyridinen, Acridinen und Phenazinen; Fluorenen und Radikal-Verbindungen.

Besonders bevorzugte elektronenreichen Metallkomplexe werden unter anderem in WO 2005/86251 A2 beschrieben, wobei diese Druckschrift zu Offenbarungszwecken in die vorliegende Anmeldung durch Referenz hierauf eingefügt wird. Hierbei sind neutrale elektronenreiche Metallkomplexe bevorzugt.

Besonders bevorzugte P=N-Verbindungen werden unter anderem in WO 2012/175535 A1 offenbart, wobei diese Druckschrift zu Offenbarungszwecken in die vorliegende Anmeldung durch Referenz hierauf eingefügt wird.

Eine weitere Gruppe von n-Dotanden stellen N-Heterocyclen dar. N-Heterocyclen sind cyclische Verbindungen, deren Ringstruktur neben Wasserstoff und Kohlenstoff mindestens ein Stickstoffatom aufweist. Diese Verbindungen können gesättigt, teilweise ungesättigt oder heteroaromatisch sein.

N-Heterocyclen können bevorzugt als Precursor eingesetzt werden, wobei sich Precursor-Verbindungen dadurch auszeichnen, dass ihre Wirkung als n-Dotand erst nach einer Aktivierung einsetzt. Bevorzugte N-Heterocyclen, die sich insbesondere als Precursor eingesetzt werden können, sind unter anderem in WO 2009/00237 A1 beschrieben, wobei diese Druckschrift zu Offenbarungszwecken in die vorliegende Anmeldung durch Referenz hierauf eingefügt wird.

Eine weitere Gruppe N-Heterocyclen, die sich als n-Dotand eignen stellen Naphthylencarbodiimide dar. Naphthylencarbodiimide umfassen mindestens eine Carbodiimid-Gruppe (N=C=N) und eine Naphthylen-Gruppe.

Überraschende Vorteile können durch die in WO 2012/168358 A1 beschriebenen Naphthylencarbodiimide erzielt werden, wobei diese Druckschrift zu Offenbarungszwecken in die vorliegende Anmeldung durch Referenz hierauf eingefügt wird.

Zu den bevorzugten, als n-Dotanden einsetzbare N-Heterocylcen zählen weiterhin Pyridin-, Acridin- und Phenazin-Derivate. Diese Verbindungen umfassen Pyridin-, Acridin- und Phenazin-Strukturelemente und sind in der Fachwelt bekannt. Bevorzugte Acridine und Phenazine sind unter anderem in US 2007/0145355 A1 dargelegt, wobei diese Druckschrift zu Offenbarungszwecken in die vorliegende Anmeldung durch Referenz hierauf eingefügt wird.

Überraschende Vorteile können durch die in EP 2 452 946 A1 und EP 2 463 927 A1 beschriebenen Pyridine erzielt werden, wobei diese Druckschriften zu Offenbarungszwecken in die vorliegende Anmeldung durch Referenz hierauf eingefügt wird.

Gemäß einer besonderen Ausgestaltung der vorliegenden Erfindung können Fluorene als n-Dotanden eingesetzt werden. Bevorzugte Fluorene sind unter anderem in WO 2012/031735 A1 beschrieben, wobei diese Druckschrift zu Offenbarungszwecken in die vorliegende Anmeldung durch Referenz hierauf eingefügt wird.

Zu den bevorzugten n-Dotanden zählen Radikal-Verbindungen, die in der Fachwelt bekannt sind. Bevorzugte Radikal-Verbindungen umfassen heterocyclische Gruppen. Besonders bevorzugte Radikal-Verbindungen sind unter anderem in EP 1 837 926 A1 und WO 2007/107306 A1 offenbart, wobei diese Druckschrift zu Offenbarungszwecken in die vorliegende Anmeldung durch Referenz hierauf eingefügt wird.

Von den genannten n-Dotanden sind die in WO 2005/86251 A2 dargelegten elektronenreichen Metallkomplexe besonders bevorzugt, wobei die Metallkomplexe der Formel $W_2(hpp)_4$ ganz besonders bevorzugt sind, worin hpp für das Anion von 1, 3, 4, 6, 7, 8-Hexahydro-2H-pyrimido [1, 2-a] pyrimidin steht. Hierbei sind neutrale elektronenreiche Metallkomplexe besonders bevorzugt.

Bei einer n-Dotierung erfolgt ein Elektronentransfer vom HOMO (highest occupied molecular orbital) Niveau des n-Dotanden zum LUMO (lowest unoccupied molecular orbital) Niveau des Matrixmaterials, wobei das Elektron im Allgemeinen nicht stark lokalisiert ist, sondern zu den Ladungsträgern beizählt.

Eine Weiterbildung der Erfindung sieht vor, dass der Betrag einer Differenz zwischen dem HOMO des n-Dotanden und dem LUMO der erfindungsgemäßen Verbindung bevorzugt kleiner als etwa 1 eV ist, weiter bevorzugt ist der Betrag der Differenz kleiner als etwa 0.5 eV.

Bevorzugt haben die erfindungsgemäßen Verbindungen ein LUMO Niveau von etwa 1 eV oder größer, ganz bevorzugt von 1.5 eV oder größer.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden vorliegend mit Hilfe quantenchemischer Rechnungen bestimmt. Zur Berechnung organischer Substanzen ohne Metalle wird zuerst eine Geometrieoptimierung mit der Methode "Ground State/Semiempirical/Default Spin/AM1/Charge 0/Spin Singlet" durchgeführt. Im Anschluss erfolgt auf Grundlage

der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

In noch einer weiteren bevorzugten Ausführungsform der vorliegenden Erfindung enthält die organische elektronische Vorrichtung eine oder mehrere der erfindungsgemäßen Verbindungen in der Emissionschicht (EML). Dabei wird die erfindungsgemäße Verbindung als Matrix-Material in der EML zusammen mit einem oder mehreren Emittern eingesetzt. Als Emitter kommen hierbei sowohl fluoreszierende als auch phosphoreszierende Emitter in Betracht, wobei phosphoreszierende Emitter bevorzugt sind.

Vom Begriff phosphoreszierender Emitter (auch phosphoreszierender Dotand genannt) sind typischerweise Verbindungen umfasst, bei denen die Lichtemission zum überwiegenden Teil durch einen spinverbotenen Übergang erfolgt, beispielsweise einen Übergang aus einem Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen.

Vorzugsweise handelt es sich bei den phosphoreszierenden Verbindungen um solche, die Licht aus einem Triplett-Zustand emittieren.

Vom Begriff fluoreszierender Emitter (auch fluoreszierender Dotand) sind Verbindungen umfasst, die zum überwiegenden Teil Lichtemission durch Übergang aus einem angeregten Singulett-Zustand aufweisen.

Sowohl fluoreszierende als auch phosphoreszierende Emitter sind dem Fachmann aus dem Stand der Technik gut bekannt und es bereitet ihm keinerlei Schwierigkeiten geeignete Emitter auszwählen.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244,

WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

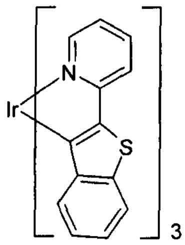

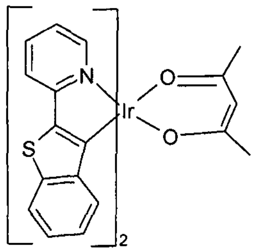

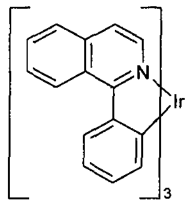

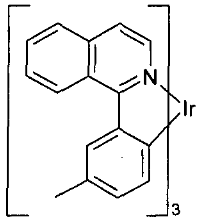

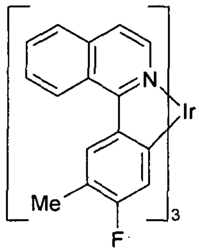

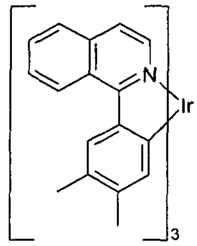

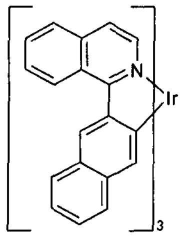

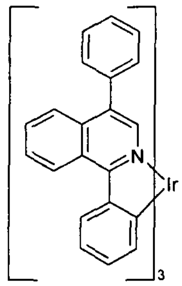

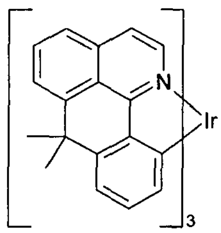

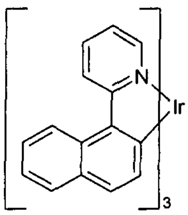

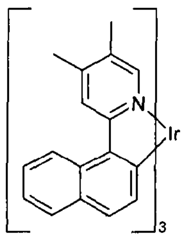

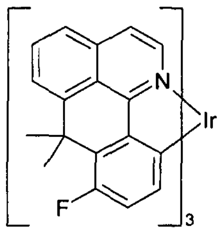

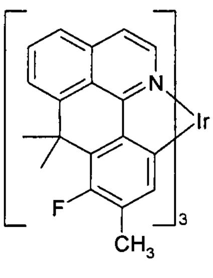

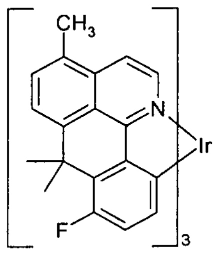

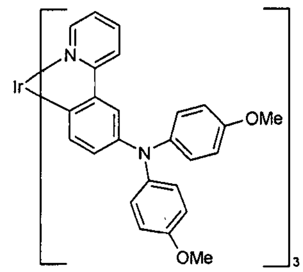

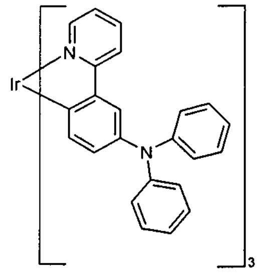
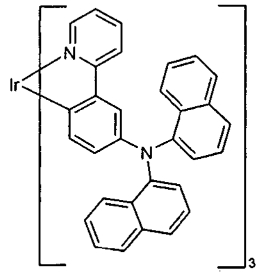
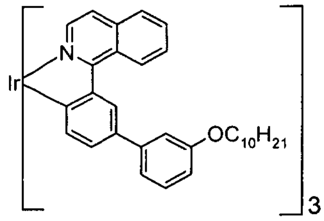
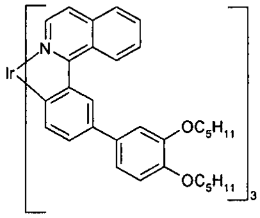
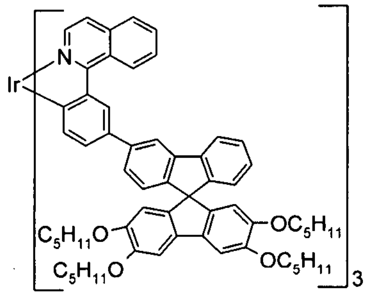
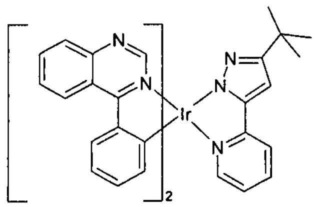
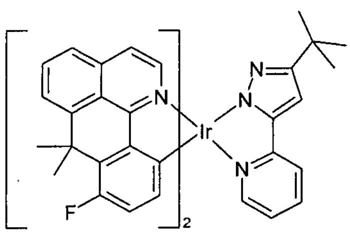
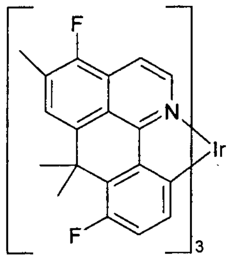
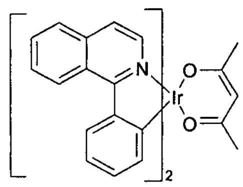
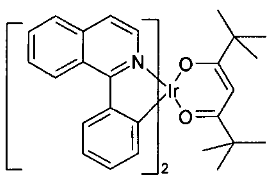
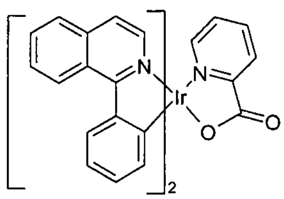
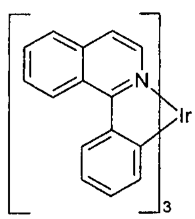
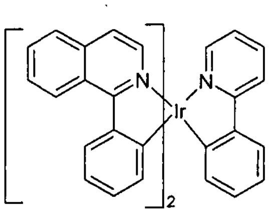
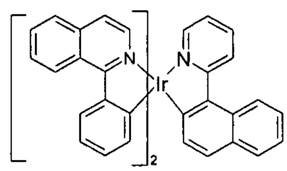
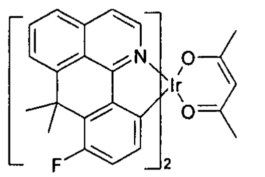
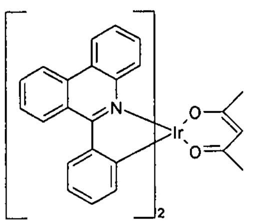
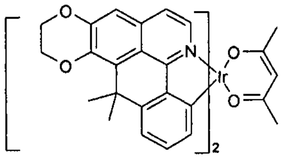
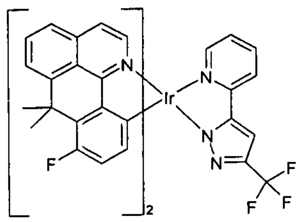

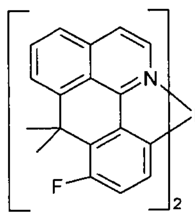
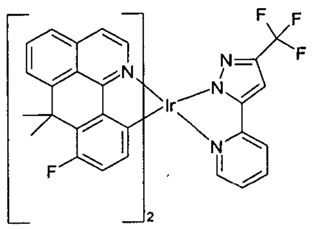
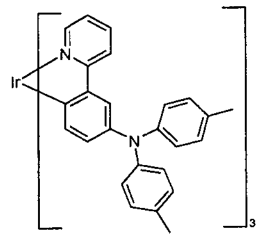

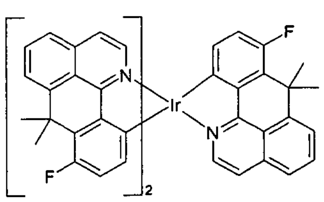
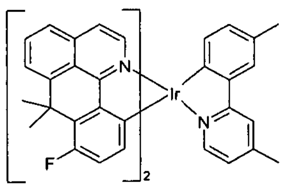
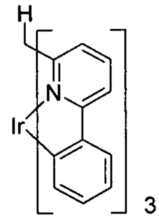

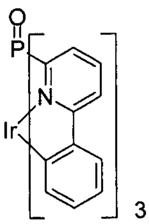
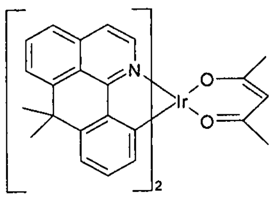
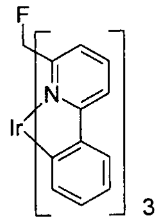

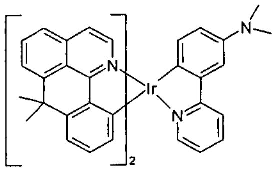
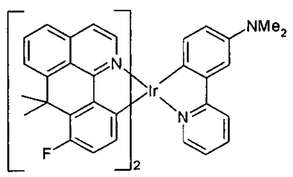
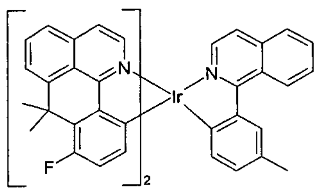

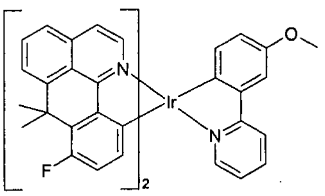
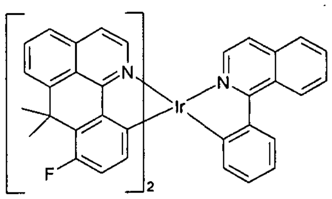
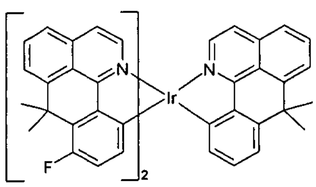

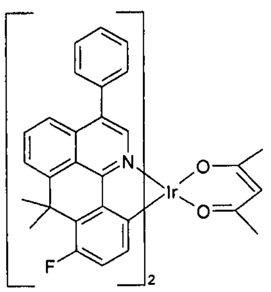
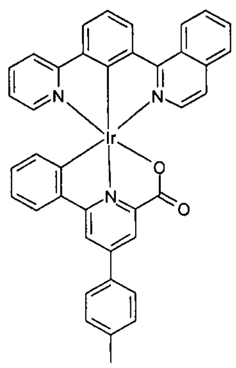
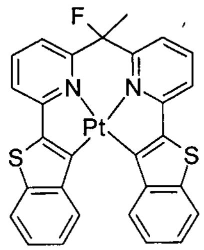

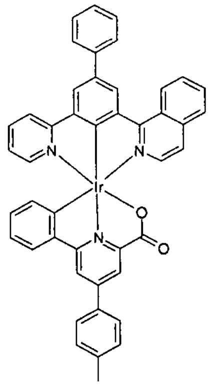
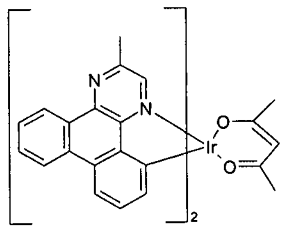
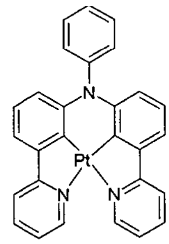
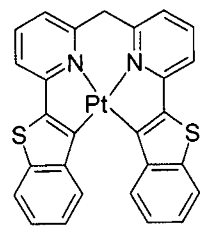
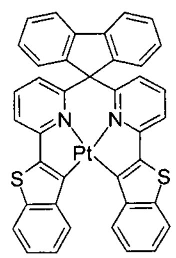
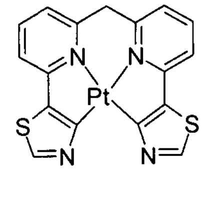
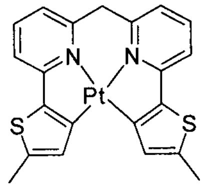
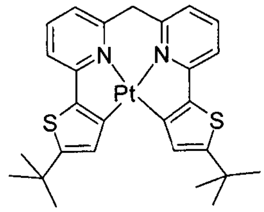
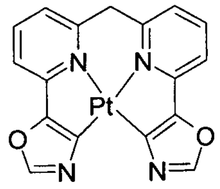
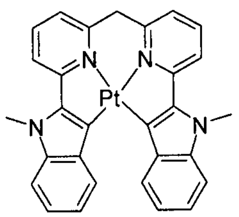
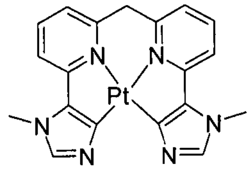
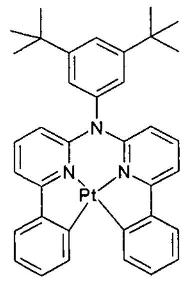
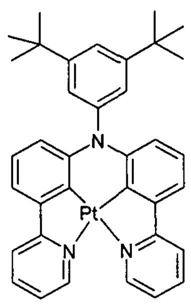
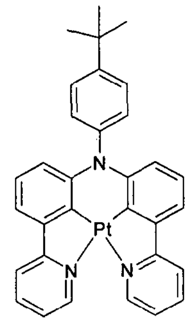
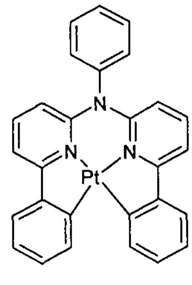

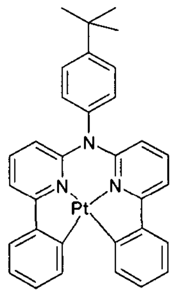
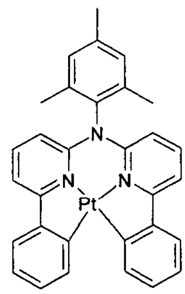
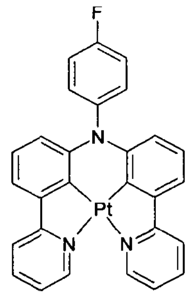

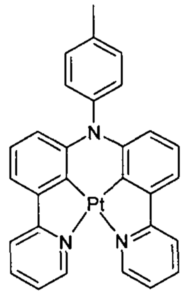
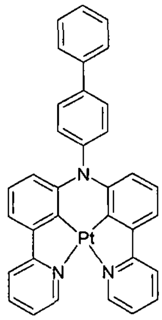
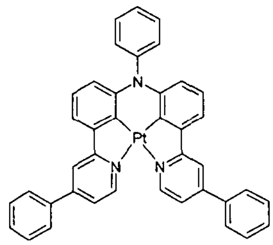

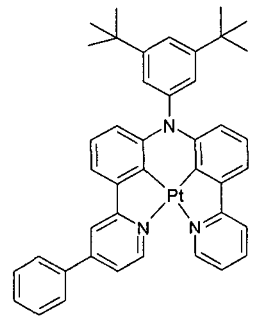
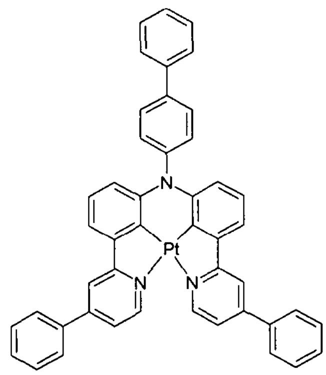
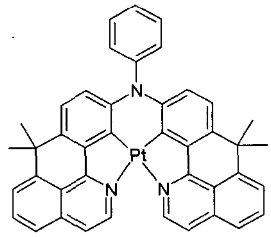

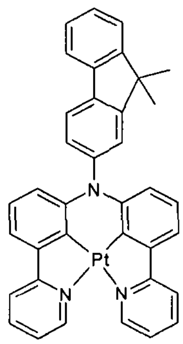
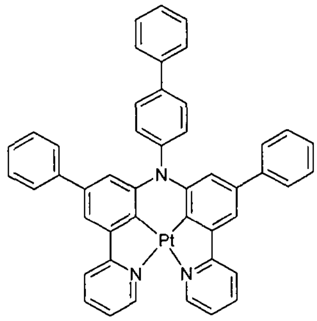
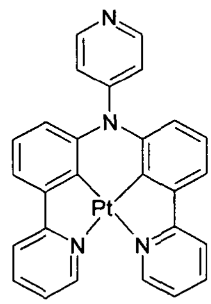

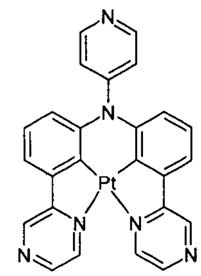
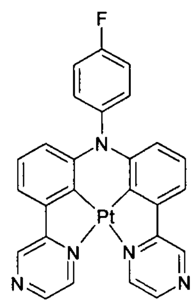
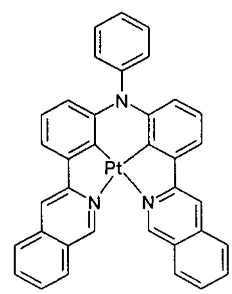

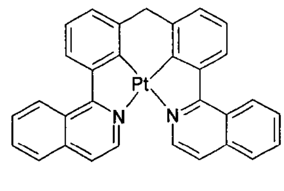
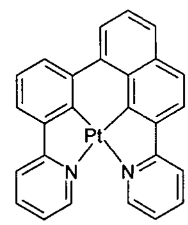
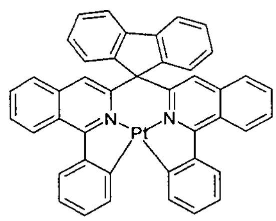

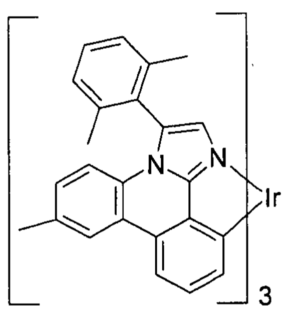
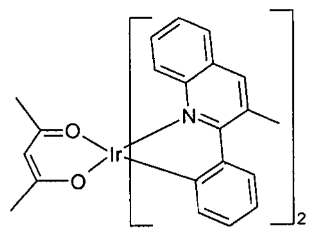
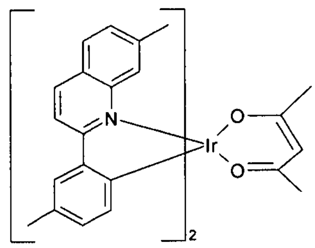

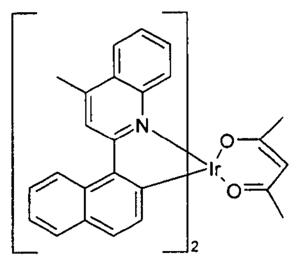
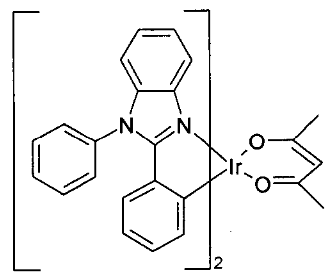
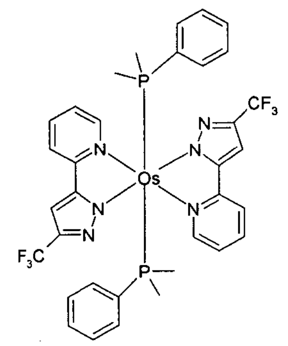

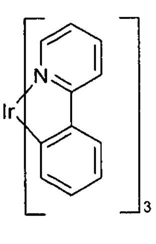
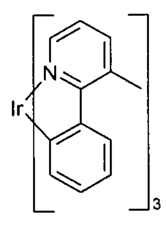
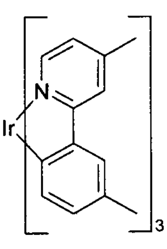

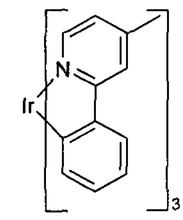
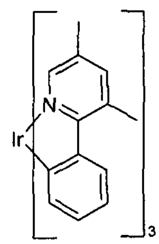
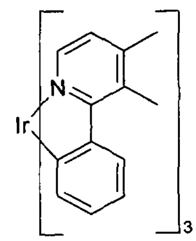
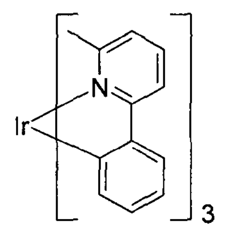
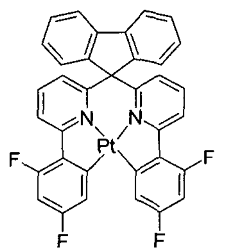
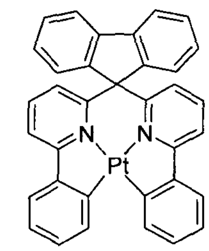
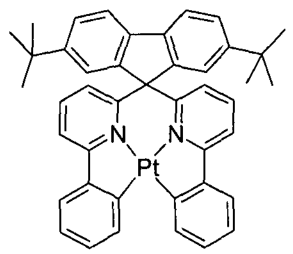
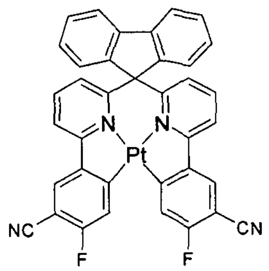
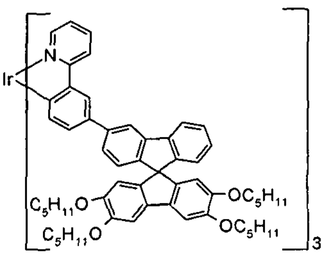
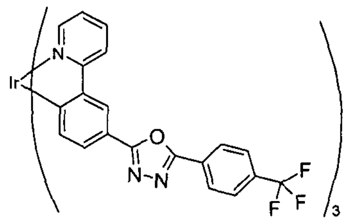
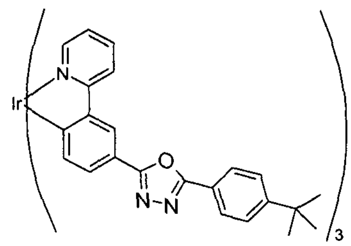
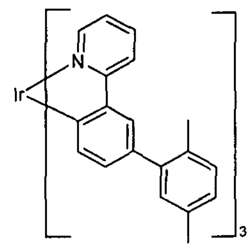
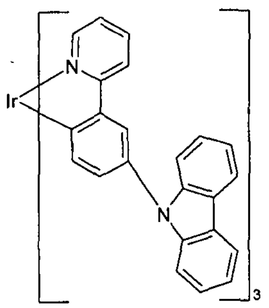
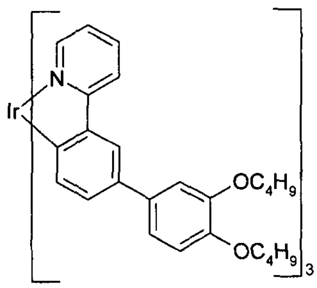
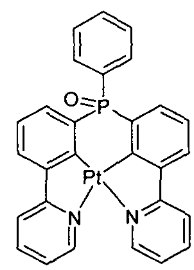
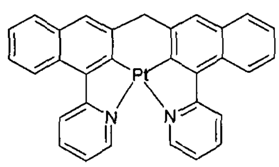
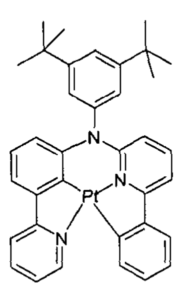
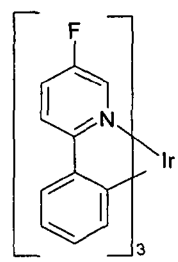

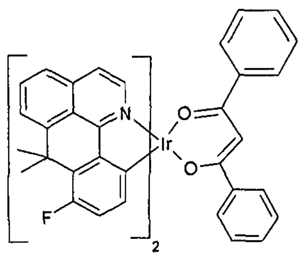
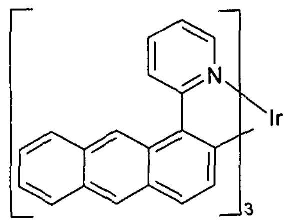
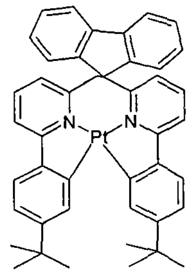

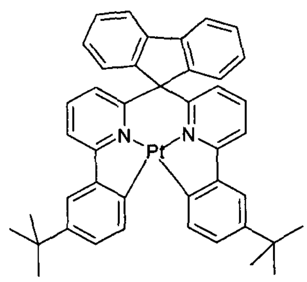
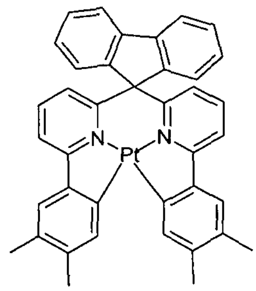
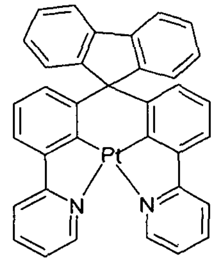

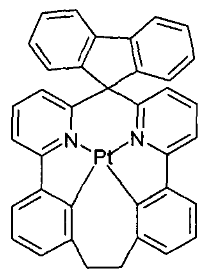
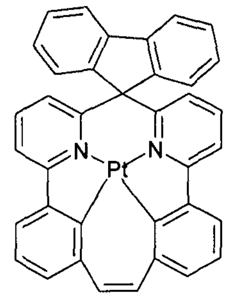
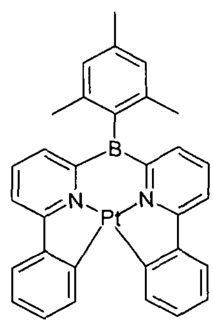

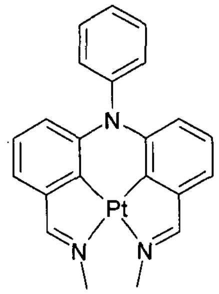
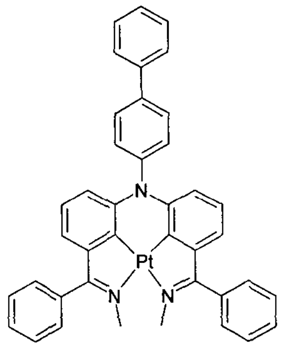
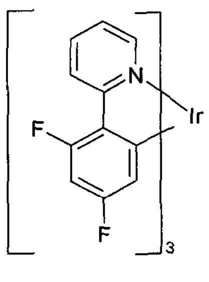

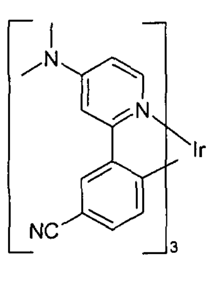
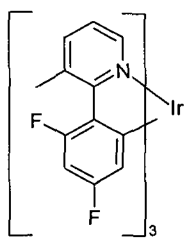
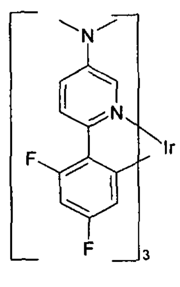

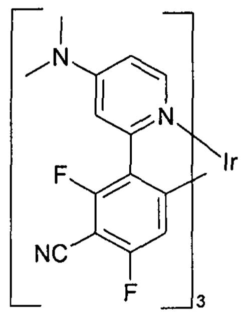
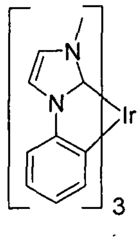
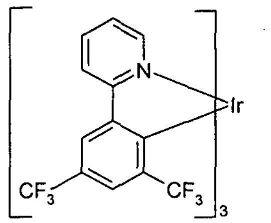

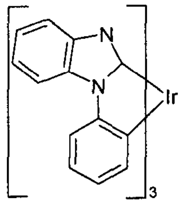
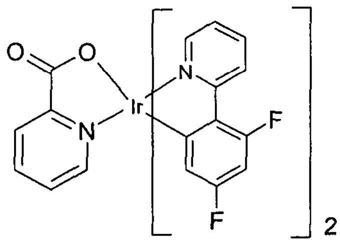
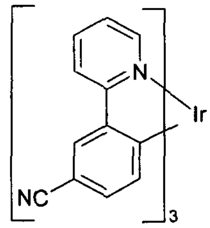

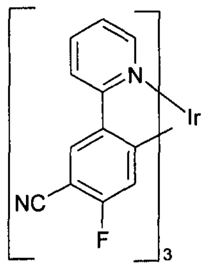
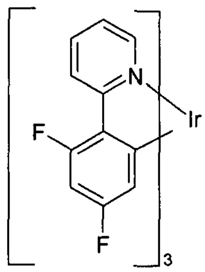
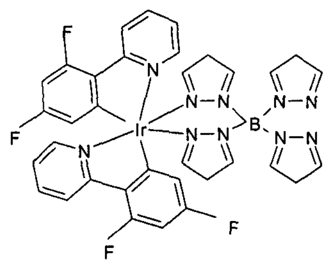

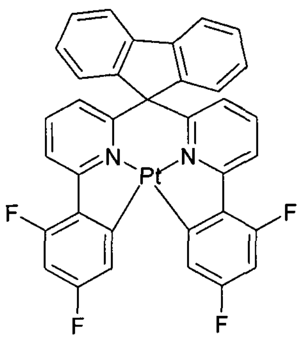
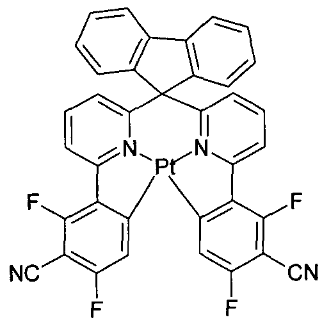
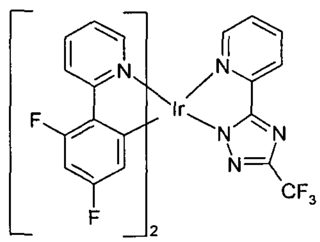

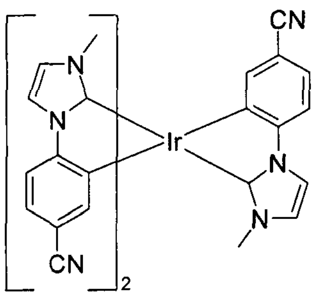
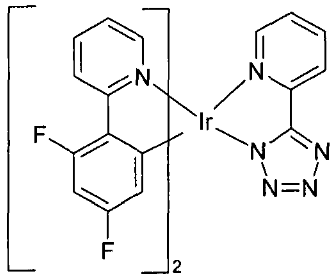
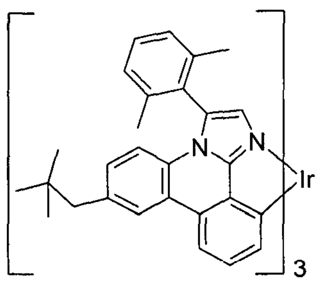

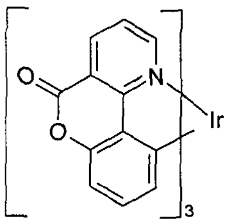

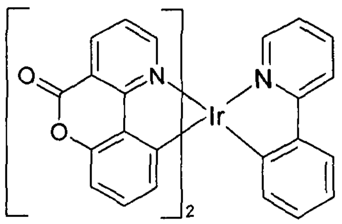

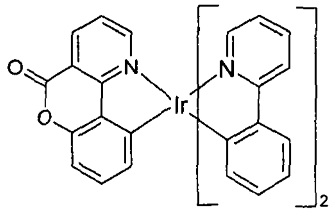

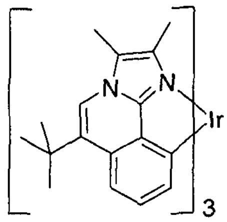

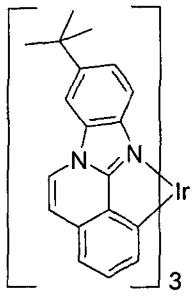

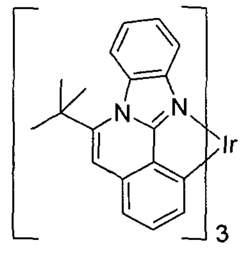

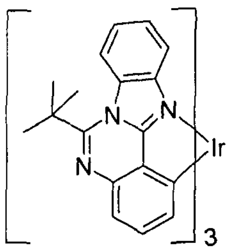

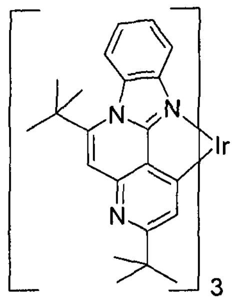

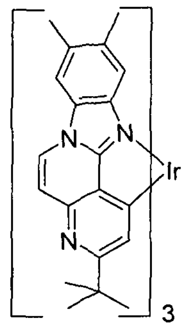

In einer weiteren bevorzugten Ausführungsform der Erfindung werden die Verbindungen als eine Matrix-Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrix-Komponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Als Matrixmaterialien, bevorzugt für fluoreszierende Dotanden, kommen Materialien verschiedener Stoffklassen in Frage. Bevorzugte Matrixmaterialien sind ausgewählt aus den Klassen der Lactame (z.B. WO 2011/116865, WO 2011/137951, WO 2013/064206, WO 2014/056567, EP 13003343.4, EP 14000729.5), Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und

der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind aromatische Amine, insbesondere , z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAIQ.

Die vorliegende Erfindung betrifft daher auch eine Zusammensetzung enthaltend wenigstens eine erfindungsgemäße Verbindung sowie wenigstens ein weiteres Matrixmaterial.

Ferner betrifft die vorliegende Erfindung auch eine Zusammensetzung enthaltend wenigstens eine erfindungsgemäße Verbindung sowie wenigstens ein weiteres Matrixmaterial und weiterhin wenigstens einen fluoreszierenden oder phosphoreszierenden Emitter, vorzugsweise einen phosphoreszierenden Emitter.

Die vorliegende Erfindung betrifft auch eine Zusammensetzung enthaltend wenigstens eine erfindungsgemäße Verbindung sowie wenigstens ein wide band gap Material, wobei unter wide band gap Material ein Material im Sinne der Offenbarung von US 7,294,849 verstanden wird. Diese Systeme zeigen besondere vorteilhafte Leistungsdaten in elektrolumineszierenden Vorrichtungen.

Systeme umfassend mehrere Matrixmaterialien (Mixed-Matrix-Systeme) werden in lichtemittierenden Schichten organischer Elektrolumineszenzvorrichtungen eingesetzt. Die lichtemittierende Schicht enthält weiter noch einen oder mehrere Dotanden.

Die erfindungsgemäßen Verbindungen und Zusammensetzungen können in organischen elektronischen Vorrichtungen eingesetzt werden. Daher betrifft die vorliegende Erfindung auch eine organische elektronische Vorrichtung enthaltend eine oder meherer der erfindungsgemäßen Verbindungen oder Zusammensetzungen.

Die erfindungsgemäße organische elektronische Vorrichtung ist bevorzugt ausgewählt aus der Gruppe bestehend aus den organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs, LECs oder LEECs), organischen Laserdioden (O-Laser) und organischen lichtemittierenden Dioden (OLEDs). Besonders bevorzugt sind dabei die organischen Elektrolumineszenzvorrichtungen, ganz besonders bevorzugt die OLECs und OLEDs und insbesondere bevorzugt die OLEDs.

Unter OLEDs im Sinne der vorliegenden Erfindung werden sowohl organische lichtemittierende Dioden enthaltend kleine organische Moleküle (SMOLEDs) als auch polymere lichtemittierende Dioden (PLEDs) verstanden, wobei SMOLEDs bevorzugte OLEDs darstellen.

Wie oben bereits erwähnt handelt es sich bei der organschen Schicht enthaltend die erfindungsgemäße Verbindung oder Zusammensetzung vorzugsweise um eine Schicht der Vorrichtung mit elektronentransportierender Funktion. Besonders bevorzugt ist sie eine Elektroneninjektionsschicht (EIL), Elektronentransportschicht (ETL), eine Lochblockierschicht (HBL)oder eine emittierende Schicht (EML).

Eine Lochtransportschicht (HTL) gemäß der vorliegenden Anmeldung ist eine Schicht mit lochtransportierender Funktion, welche sich zwischen Anode und emittierender Schicht (EML) befindet.

Eine Elektronenransportschicht (ETL) gemäß der vorliegenden Anmeldung ist eine Schicht mit elektronentransportierender Funktion, welche sich zwischen Kathode und emittierender Schicht (EML) befindet.

Der Aufbau organischer elektronischer Vorrichtungen, und insbesondere auch von organischen Elektrolumineszenzvorrichtungen ist dem Fachmann aus dem Stand der Technik gut bekannt.

Eine erfindungsgemäße elektronische, insbesondere elektrolumineszierende, organische Vorrichtung umfasst mindestens eine organische Schicht. Eine organische Schicht zeichnet sich dadurch aus, dass diese mindestens eine organische bzw. metallorganische Verbindung enthält. Eine organische Vorrichtung muss nicht notwendigerweise nur Schichten enthalten, welche aus organischen oder metallorganischen Materialien aufgebaut sind. So ist es auch möglich, dass eine oder mehrere Schichten anorganische Materialien enthalten oder ganz aus anorganischen Materialien aufgebaut sind.

Die erfindungsgemäße organische elektronische Vorrichtung enthält Anode, Kathode und mindestens eine

organische Schicht, welche mindestens eine erfindungsgemäße Verbindung umfasst.

Wie bereits erwähnt sind besonders bevorzugte organische elektronische Vorrichtungen im Sinne der vorliegenden Erfindung die organischen Elektrolumineszenzvorrichtungen, und hierbei insbesondere die OLEDs.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat) oder substituierte Li-Hydroxychinolinate.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. $Al/Ni/NiO_x$, $Al/PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $MoO_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

Gemäß einer bevorzugten Ausführungsform können zwischen der Anode und der Katode mindestens zwei organische Schichten angeordnet sein.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren. Typische ETMs, EIMs, Emitter und Matrixmaterialien wurden weiter oben bereits offenbart.

Eine organische Elektrolumineszenzvorrichtung enthält Kathode, Anode und mindestens eine emittierende Schicht (EML). Außer diesen Schichten kann sie noch weitere Schichten enthalten, beispielsweise jeweils eine oder mehrere Lochinjektionsschichten (HIL), Lochtransportschichten (HTL), Lochblockierschichten (HBL), Elektronentransportschichten (ETL), Elektroneninjektionsschichten (EIL), Exzitonenblockierschichten (ExBL), Elektronenblockierschichten (EBL), Ladungserzeugungsschichten und/oder organische oder anorganische p/n-Übergänge. Ein typischer Aufbau einer organischen Elektrolumineszenzvorrichtung ist:

Anode/HIL/HTL/EML/ETL/EIL/Kathode

Dabei ist es möglich, dass eine oder mehrere Lochtransportschichten p-dotiert sind, beispielsweise mit Metalloxiden, wie $MoO_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, dadurch gekennzeichnet, dass eine oder mehrere Schichten mit einem Sublimationsverfahren beschichtet

werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend mindestens einen Emitter und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Schwierigkeiten auf elektronischen Vorrichtungen, insbesondere organische Elektrolumineszenzvorrichtungen enthaltend eine erfindungsgemäße Verbindung angewendet werden.

Für die Herstellung organischer elektronischer Vorrichtungen aus Lösung sind Formulierungen enthaltend die erfindungsgemäße Verbindung oder die erfindungsgemäße Zusammensetzung erforderlich.

Die vorliegende Erfindung betrifft daher auch eine Formulierung enthaltend wenigstens eine erfindungsgemäße Verbindung oder wenigstens eine erfindungsgemäße Zusammensetzung sowie wenigstens ein Lösungsmittel.

Diese Formulierung kann dabei,beispielsweise, eine Lösung, Dispersion oder Emulsion sein. Es kann bevorzugt sein, hierfür Mischungen aus zwei oder mehr Lösungsmitteln zu verwenden. Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, □-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethyheneglycolbutylmethyl ether, Triethylenglycolbutylmethyl ether, Diethyleneglycoldibutyl ether, Triethylenglycoldimethyl ether, Diethylenglycolmonobutylether, Tripropyleneglycol dimethyl ether, Tetraethyleneglycol dimethyl ether, 2-isopropyl naphthalin, Pentylbenzol, Hexylbenzol, Heptylbenzol, octylbenzol, 1,1-Bis(3,4-Dimethylphenyl)ethan oder Mischungen dieser Lösungsmittel.

Vorrichtungen enthaltend die erfindungsgemäße Verbindung oder die erfindungsgemäße Zusammensetzung können sehr vielseitig eingesetzt werden. So können, beispielsweise, elektrolumineszierende Vorrichtungen in Displays für Fernseher, Mobiltelefone, Computer und Kameras eingesetzt werden. Die Vorrichtungen können aber auch in Beleuchtungsanwendungen verwendet werden. Weiterhin können elektrolumineszierende Vorrichtungen in der Medizin oder Kosmetik zu Phototherapie genutzt werden. Somit kann eine Vielzahl von Erkrankungen (Psoriasis, atopische Dermatitis, Inflammation, Akne, Hautkrebs etc.) oder die Vermeidung sowie Reduktion von Hautfaltenbildung, Hautrötung und Hautalterung behandelt werden. Weiterhin können die lichtemittierenden Vorrichtungen dazu genutzt werden, um Getränke, Speisen oder Lebensmittel frisch zu halten oder um Geräte (bspw. medizinische Geräte) zu sterilisieren.

Gegenstand der vorliegenden Erfindung ist daher eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine erfindungsgemäße Verbindung oder wenigstens eine erfindungsgemäße Zusammensetzung zur Verwendung in der Medizin zur Phototherapie.

Ein weiterer bevorzugter Gegenstand der vorliegenden Erfindung betrifft eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine erfindungsgemäße Verbindung oder wenigstens eine erfindungsgemäße Zusammensetzung zur Verwendung zur phototherapeutischen Behandlung von Hautkrankheiten.

Ein weiterer ganz bevorzugter Gegenstand der vorliegenden Erfindung betrifft eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine erfindungsemäße Verbindung oder wenigstens eine erfindungsgemäße Zusammensetzung zur Verwendung zur phototherapeutischen Behandlung von Psoriasis, atopische Dermatitis,

Entzündungserkrankungen, Vitiligo, Wundheilung, Hautkrebs und Ikterus, insbesondere Neugeborenen-Ikterus.

Die vorliegende Erfindung betrifft weiterhin die Verwendung der elektronischen Vorrichtung, bevorzugt einer organischen Elektrolumineszenzvorrichtung, ganz bevorzugt einer OLED oder OLEC und ganz besonders bevorzugt einer OLED enthaltend wenigstens eine erfindungsgemäße Verbindung oder wenigstens eine erfindungsgemäße Zusammensetzung in der Kosmetik, bevorzugt zur Behandlung von Akne, alternder Haut (Skin Ageing), und von Zellulithe.

Die erfindungsgemäßen elektronischen Vorrichtungen, insbesonder die organischen Elektrolumineszenzvorrichtungen zeichnen sich durch folgende überraschende Vorteile gegenüber dem Stand der Technik aus:

1. Die erfindungsgemäßen organischen Elektrolumineszenzvorrichtungen weisen eine sehr hohe Effizienz auf.

2. Die erfindungsgemäßen organischen Elektrolumineszenzvorrichtungen weisen gleichzeitig eine verbesserte Lebensdauer auf.

3. Die erfindungsgemäßen organischen Elektrolumineszenzvorrichtungen weisen gleichzeitig eine verringerte Betriebsspannung auf.

4. Die erfindungsgemäßen Verbindungen lassen sich sehr leicht und kostengünstig herstellen, sind gut zu prozessieren und lassen sich sehr gut verdampfen, so dass sich die erfindungsgemäßen Verbindungen sehr gut für die kommerzielle Massenproduktion organischer elektronischer Vorrichtungen, insbesondere von organischen Elektrolumineszenzvorrichtungen eignen.

Die oben genannten bevorzugten Ausführungsformen sind beliebig miteinander kombinierbar. In einer besonders bevorzugten Ausführungsform der Erfindung gelten die oben genannten bevorzugten Ausführungsformen gleichzeitig.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

## Beispiele

### Beispiel 1

### Synthese von 2-[3-[7'-(4,6-diphenyl-1,3,5-triazin-2-yl)-9,9'-spirobi[fluoren]-2'-yl]phenyl]-1-phenyl-benzimidazol 6a

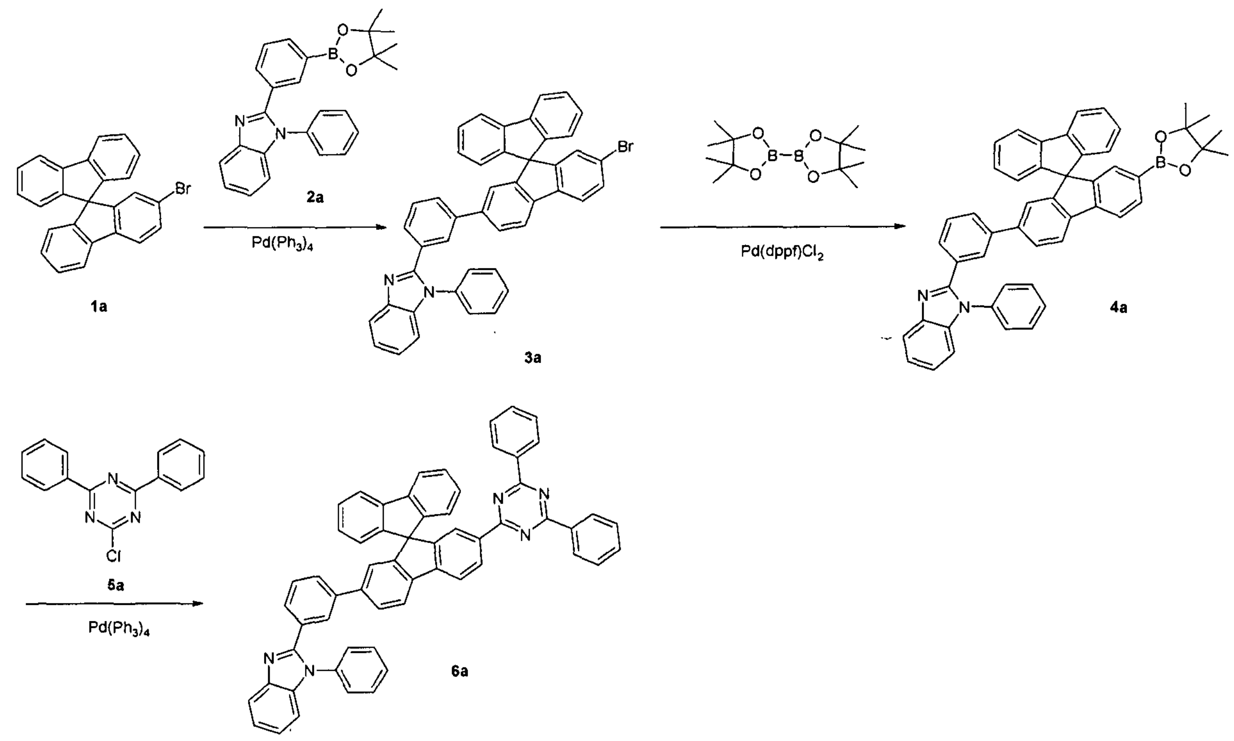


**Synthese von 2-[3-(7'-bromo-9,9'-spirobi[fluoren]-2'-yl)phenyl]-1-phenyl-benzimidazol (3a)**

**Variante A**

In einem 2 L-Vierhalskolben werden unter Schutzgas 50.0 g (105 mmol, 1.00 eq) 2,7-Dibromo-9,9'-spirobifluoren **1a,** 41.7 g (105 mmol, 1.00 eq) 1-Phenyl-2-[e-(4,4,5,5-tetramethyl-[1,3,2]dioxaborolan-y-yl)-phenyl]-1H-benzoimidazol **2a** und 36.4 g (263 mmol, 2.50 eq) Kaliumcarbonat in 400 ml Toluol, 400 ml 1,4-Dioxan und 200 ml VE-Wasser vorgelegt und entgast. Anschließend wird 1.22 g (1.05 mmol, 0.01 eq) Tetrakis(triphenylphosphin)-palladium(0) zugegeben und über Nacht am Rückfluss erhitzt. Nach beendeter Reaktion wird der Ansatz abgekühlt, über Celite filtriert und mit 1 L Toluol verdünnt. Die Lösung wird 3x mit je 300 ml halbgesättigter Natriumchloridlösung gewaschen und nach Trocknung über Natriumsulfat auf ca. 200 ml am Rotationsverdampfer eingeengt. Der ausgefallene Feststoff wird abfiltriert und im Vakuum getrocknet. Die Abtrennung des disubstituierten Nebenproduktes erfolgt mittels Sublimation. Es werden 22.0 g (33.1 mmol, 32%) des gewünschten Produktes **3a** erhalten.

**Variante B**

Die Durchführung erfolgt analog zu der von Variante A, wobei anstelle von Tetrakis(triphenylphosphin)palladium(0) 0.01 eq Palladium(II)-acetat und 0.01 eq Dicyclohexyl-(2',6'-dimethoxy-biphenyl-2-yl-phophan (SPhos) verwendet werden.

Analog werden umaesetzt:

| Nr. | Edukt 1 | Edukt 2 Produkt 3 | | Var. | Ausb. |
|---|---|---|---|---|---|
| 3b | 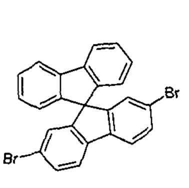 [171408-84-7] | 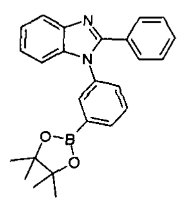 [1549686-33-0] | 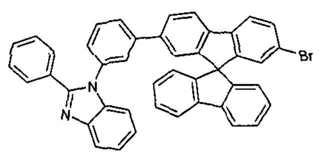 | A | 54% |

(fortgesetzt)

| Nr. | Edukt 1 | Edukt 2 | Produkt 3 | Var. | Ausb. |
|---|---|---|---|---|---|
| **3c** | 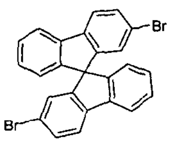 [67665-47-8] | 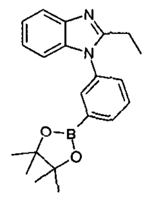 | 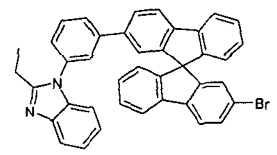 | A | 35% |
| **3d** | 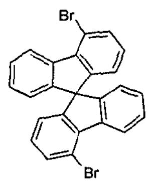 [1257321-41-7] | 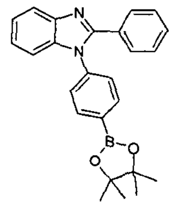 [1169709-19-6] | 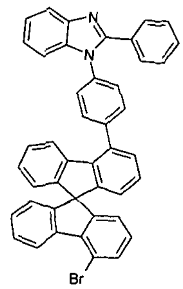 | B | 41% |
| **3e** | 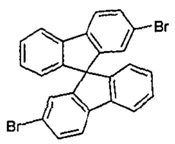 [67665-47-8] | 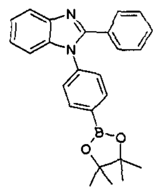 [1169709-19-6] | 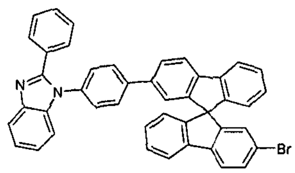 | A | 59% |
| **3f** | 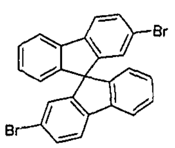 [67665-47-8] | 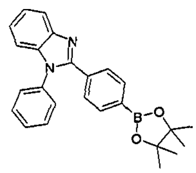 [1146340-38-6] | 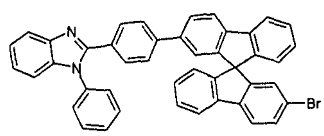 | B | 32% |
| **3g** | 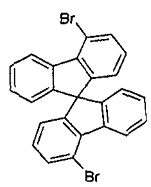 [1257321-41-7] | 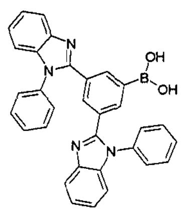 [1505512 -90-2] | 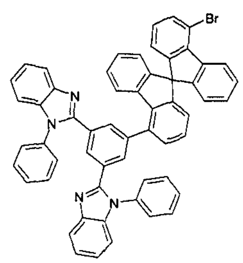 | B | 62% |
| **3h** | 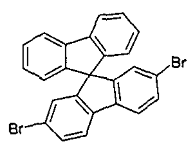 [171408-84-7] | 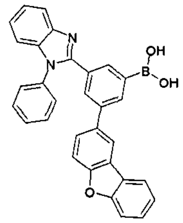 [1172134-18-7] | 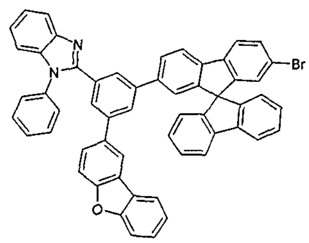 | B | 44% |

(fortgesetzt)

| Nr. | Edukt 1 | Edukt 2 | Produkt 3 | Var. | Ausb. |
|---|---|---|---|---|---|
| **3i** | 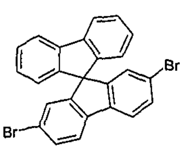 [171408-84-7] | 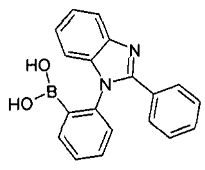 [1553408-82-4] | 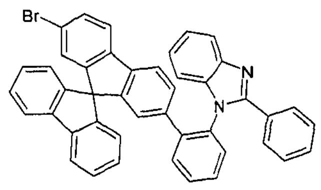 | A | 21% |
| **3j** | 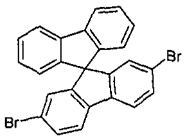 [171408-84-7] | 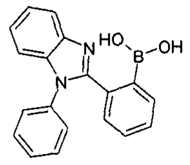 [58534-74-0] | 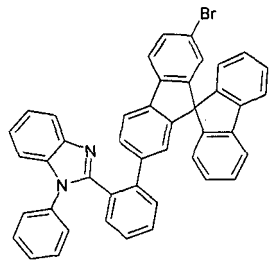 | B | 15% |
| **3k** | 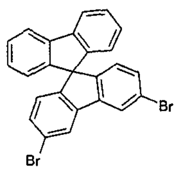 [1373114-50-1] | 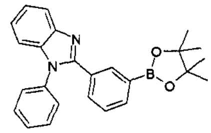 [952514-86-2] | 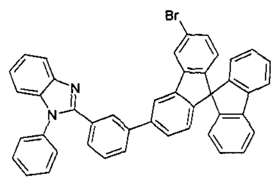 | A | 64% |
| **3l** | 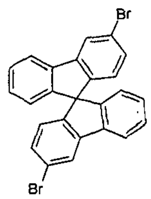 [1418299-83-8] | 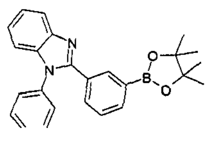 [952514-86-2] | 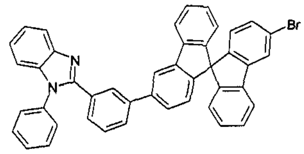 | A | 38% |
| **3m** | 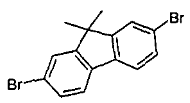 [28320-32-3] | 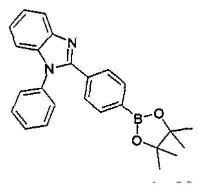 [1146340-38-6] | 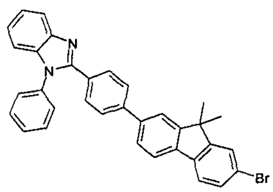 | B | 32% |
| **3n** | 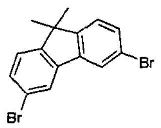 [865702-19-8] | 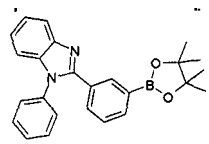 [952514-86-2] | 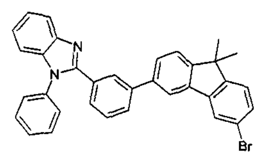 | B | 53% |
| **3o** | 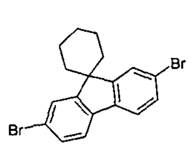 [736138-41-3] | 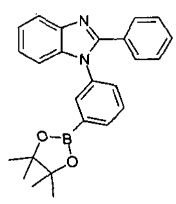 [1549686-33-0] | 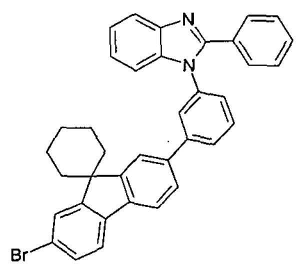 | A | 41% |

(fortgesetzt)

| Nr. | Edukt 1 | Edukt 2 | Produkt 3 | Var. | Ausb. |
|---|---|---|---|---|---|
| 3p | 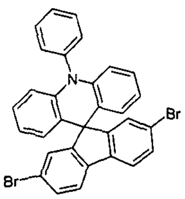 [880800-04-4] | 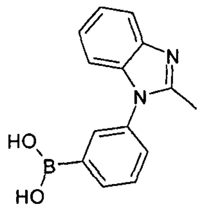 [1384871-80-0] | 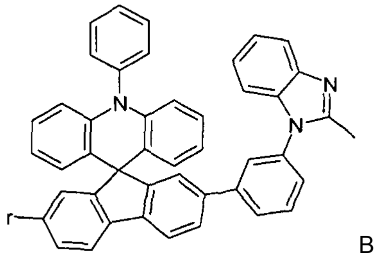 | B | 58% |
| 3q | 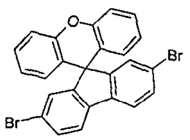 [198142-65-3] | 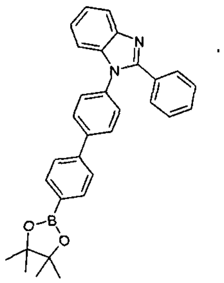 [1219956-29-2] | 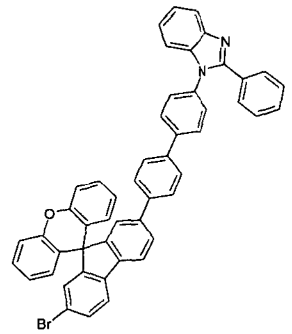 | B | 17% |
| 3r | 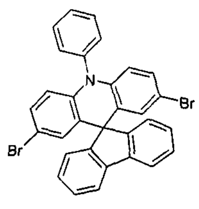 [1303969-26-7] | 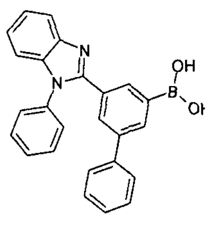 [1172134-16-5] | 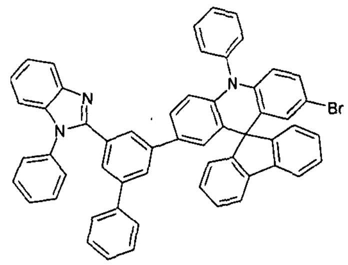 | A | 39% |
| 3s | 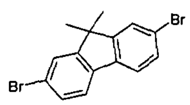 [28320-32-3] | 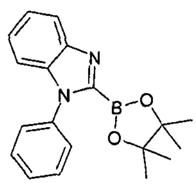 [1316275-47-4] | 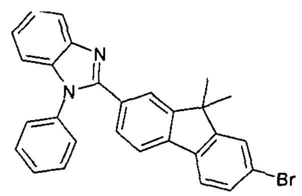 | B | 28% |
| *Var. - Variante; Ausb. - Ausbeute* | | | | | |

**Synthese von 1-Phenyl-2-[3-[7'-(4,4,5,5-tetramethyl-1,3,2-dioxaborolan-2-yl)-9,9'-spirobi[fluoren]-2'-yl]phenyl]benzimidazol 4a**

In einem 1 L-Vierhalskolben werden 22.0 g (33.1 mmol, 1.00 eq) 2-[3-(7'-bromo-9,9'-spirobi[fluoren]-2'-yl)phenyl]-1-phenyl-benzimidazol 3a, 8.84 g (30.1 mmol, 0.91 eq) Bis(pinacolato)diboron und 26.0 g (265 mmol, 8.00 eq) Kaliumacetat in 500 ml getrocknetem 1,4-Dioxan vorgelegt und für 30 Minuten entgast. Anschließend werden 812 mg (0.995 mmol, 0.0300 eq) 1,1-Bis(diphenylphosphino)ferrocen-dichloropalladium(II)-Komplex mit DCM zugegeben und auf 80°C Innentemperatur erhitzt. Nach Rühren über Nacht wird der Ansatz abgekühlt und der ausgefallene Feststoff abgesaugt. Das Filtrat wird am Rotationsverdampfer auf ca. 50 ml eingeengt und der ausgefallene Feststoff ebenfalls abgesaugt. Die Feststoffe werden vereint und getrocknet. Es werden 21.0 g (29.5 mmol, 89%) des Boronesters **4a** erhalten.

Analog werden umgesetzt:

| Nr. | Edukt 3 | Produkt 4 | Ausbeut e |
|---|---|---|---|
| **4b** | 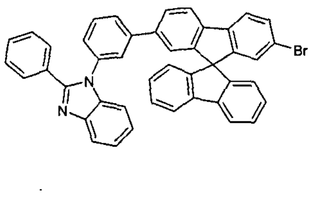 | 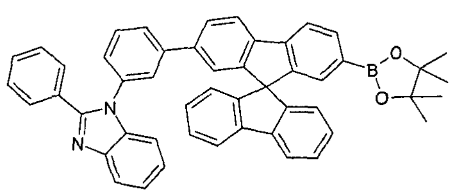 | 95% |
| **4c** | 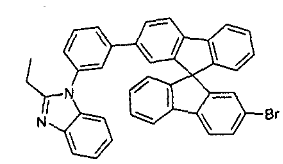 | 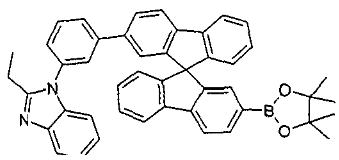 | 87% |
| **4d** | 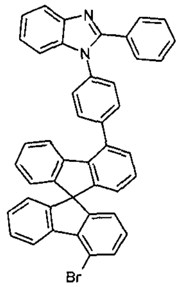 | 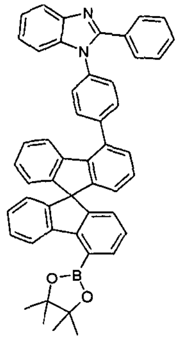 | 97% |
| **4e** | 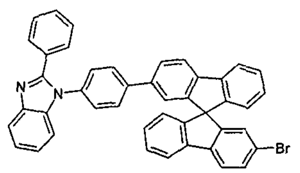 | 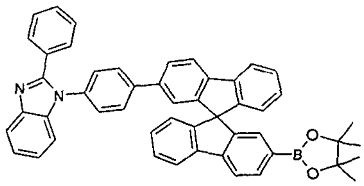 | 94% |
| **4f** | 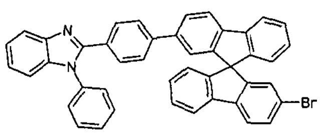 | 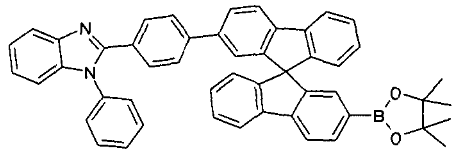 | 88% |
| **4g** | 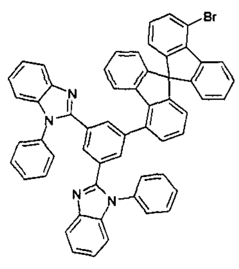 | 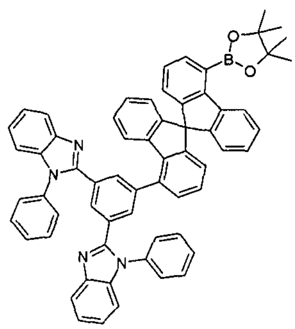 | 47% |

(fortgesetzt)

| Nr. | Edukt 3 | Produkt 4 | Ausbeut e |
|---|---|---|---|
| 4h | 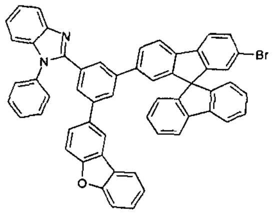 | 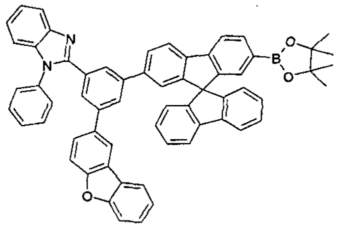 | 81% |
| 4i | 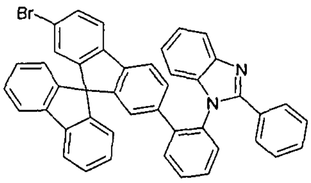 | 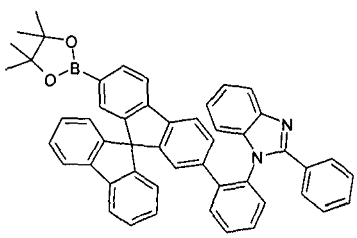 | 79% |
| 4j | 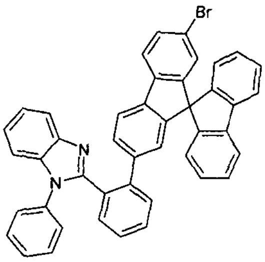 | 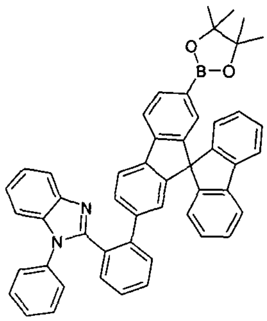 | 74% |
| 4k | 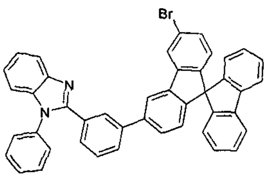 | 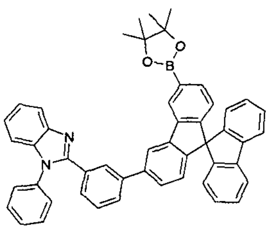 | 55% |
| 4l | 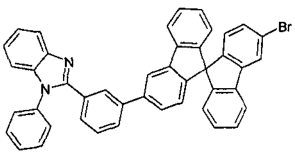 | 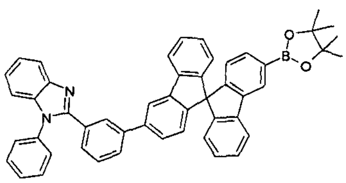 | 47% |
| 4m | 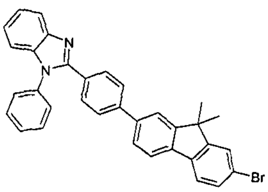 | 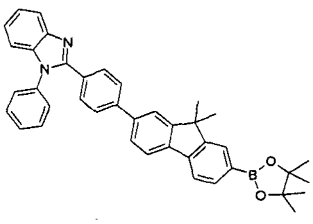 | 85% |

(fortgesetzt)

| Nr. | Edukt 3 | Produkt 4 | Ausbeut e |
|---|---|---|---|
| **4n** | 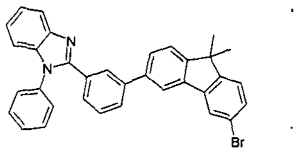 | 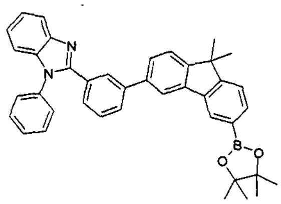 | 82% |
| **4o** | 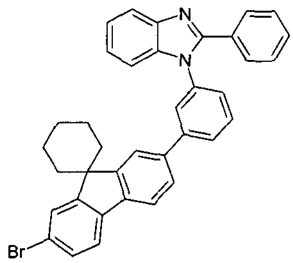 | 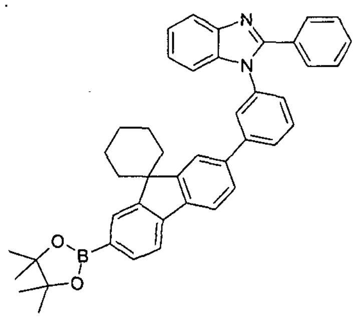 | 87% |
| **4p** | 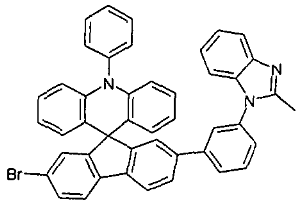 | 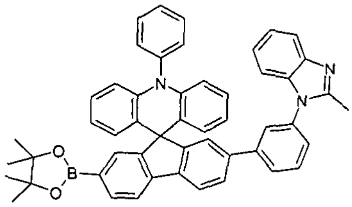 | 66% |
| **4q** | 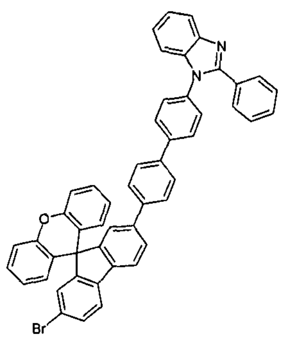 | 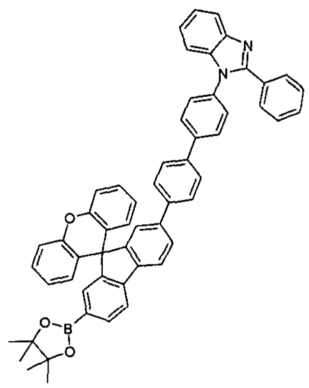 | 58% |
| **4r** | 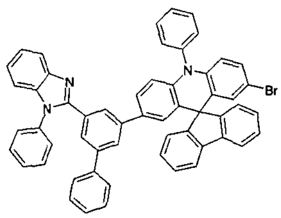 | 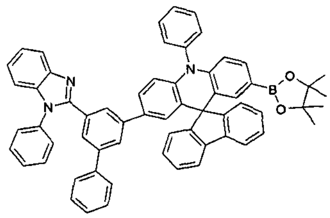 | 84% |
| **4s** | 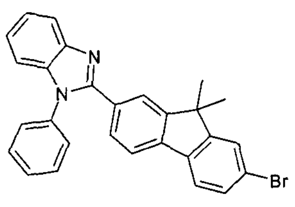 | 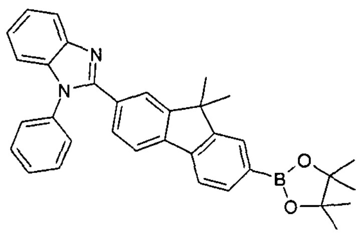 | 77% |

**Synthese von 2-[3-[7'-(4,6-diphenyl-1,3,5-triazin-2-yl)-9,9'-spirobi[fluoren]-2'-yl]phenyl]-1-phenyl-benzimidazol 6a**

**Variante A**

In einem 1 L-Dreihalskolben werden 21.0 g (29.5 mmol, 1.00 eq) 1-phenyl-2-[3-[7'-(4,4,5,5-tetramethyl-1,3,2-dioxaborolan-2-yl)-9,9'-spirobi[fluoren]-2'-yl]phenyl]benzimidazol **4a** und 7.90 g (29.5 mmol, 1.00 eq) 2-Chloro-4,6-diphenyl-[1,3,5]triazin **5a** zusammen mit 3.75 g (35.4 mmol, 1.20 eq) Natriumcarbonat in 200 ml Toluol, 200 ml 1,4-Dioxan und 100 ml VE-Wasser vorgelegt und für 20 Minuten entgast. Nach Zugabe von 1.02 g (0.885 mmol, 0.0300 eq) Tetrakis(triphenylphosphin)palladium(0) wird der Ansatz für 2 Tage unter Rückfluss erhitzt und nach beendeter Reaktion abgekühlt. Der ausgefallene Feststoff wird abgesaugt, mit Wasser und etwas Toluol gewaschen und anschließend mehrfach aus Toluol/Heptan umkristallisert bis eine HPLC-Reinheit von >99.9% erreicht wird. Nach Sublimation werden 11.5 g (14.0 mmol, 43%) eines farblosen Feststoffs **6a** erhalten.

**Variante B**

Die Durchführung erfolgt analog zu der von Variante A, wobei anstelle von Tetrakis(triphenylphosphin)palladium(0) 0.01 eq Palladium(II)-acetat und 0.04eq Tri(o-Tolyl)phosphin verwendet werden.

**Variante C**

Die Durchführung erfolgt analog zu der von Variante A, wobei anstelle von Tetrakis(triphenylphosphin)palladium(0) 0.01 eq Palladium(II)-acetat und 0.01 eq Dicyclohexyl-(2',6'-dimethoxy-biphenyl-2-yl-phophan (SPhos) verwendet werden.

Analog werden hergestellt:

| Nr. | Edukt 4 | Edukt 5 | Produkt 6 | Var. | Ausb |
|---|---|---|---|---|---|
| **6b** | 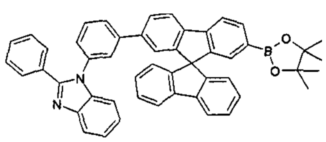 | 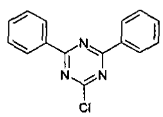 [3842-55-5] | 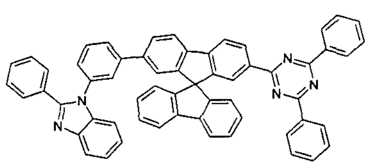 | B | 57% |
| **6c** | 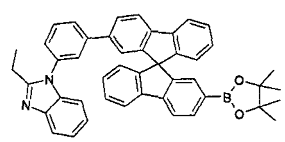 | 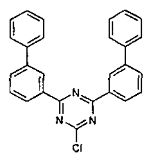 [1205748-61-3] | 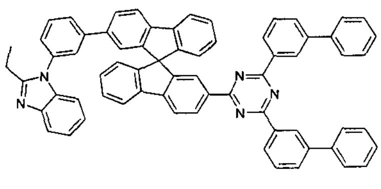 | A | 38% |
| **6d** | 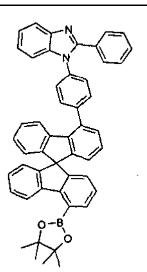 | 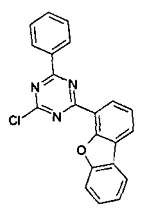 [1472729-25-1] | 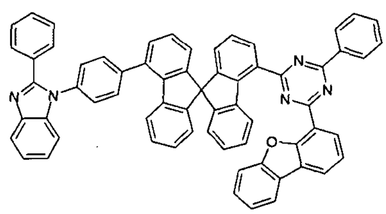 | A | 42% |
| 6e | 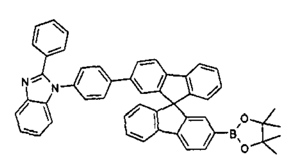 | 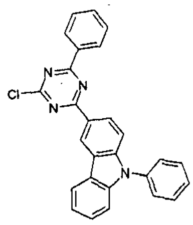 [1476785-42-8] | 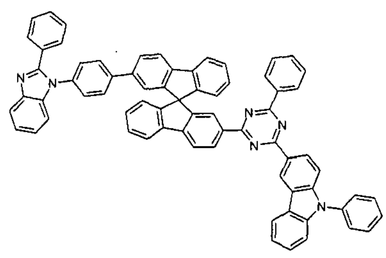 | C | 61% |

(fortgesetzt)

| Nr. | Edukt 4 | Edukt 5 | Produkt 6 | Var. | Ausb |
|---|---|---|---|---|---|
| **6f** | 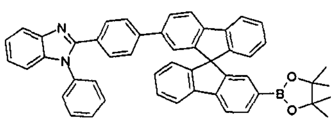 | 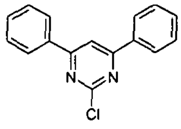 [2915-16-4] | 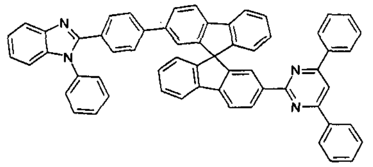 | B | 37% |
| **6g** | 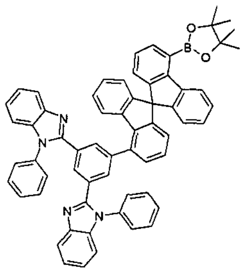 | 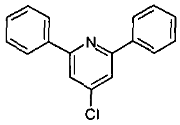 [133785-60-1] | 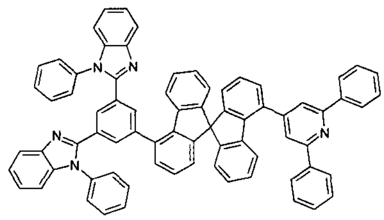 | C | 39% |
| **6h** | 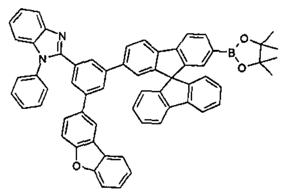 | 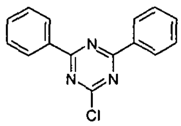 [3842-55-5] | 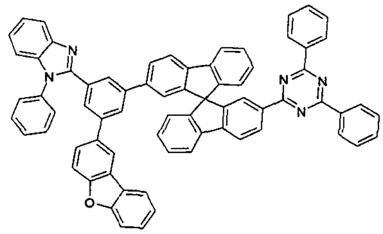 | C | 31% |
| **6i** | 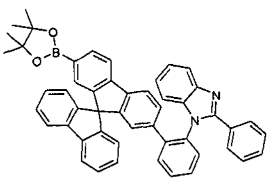 | 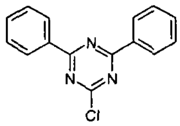 [3842-55-5] | 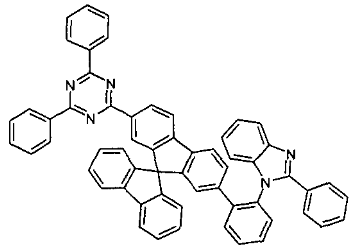 | A | 48% |
| **6j** | 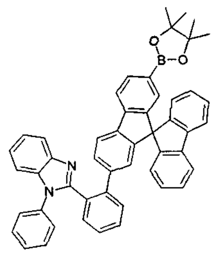 | 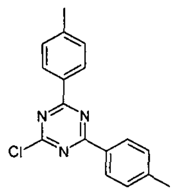 [21902-34-1] | 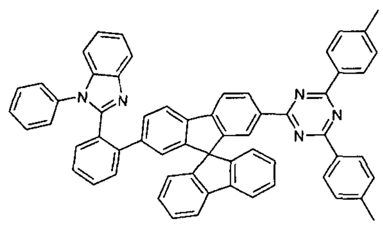 | A | 24% |
| **6k** | 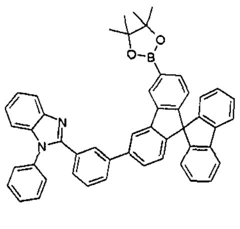 | 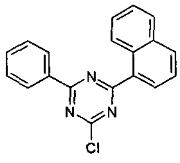 [1472062-95-5] | 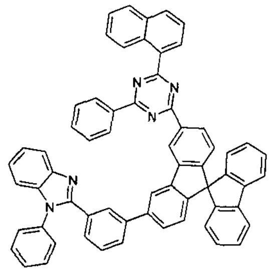 | B | 51% |

(fortgesetzt)

| Nr. | Edukt 4 | Edukt 5 | Produkt 6 | Var. | Ausb |
|---|---|---|---|---|---|
| 6l | 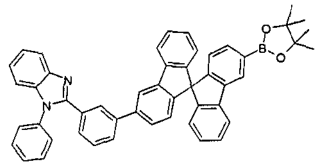 | 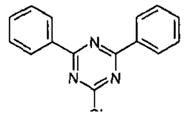 [3842-55-5] | 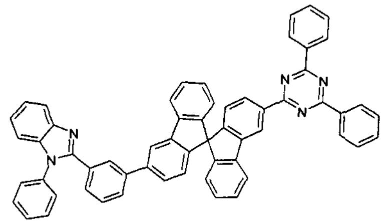 | B | 38% |
| 6m | 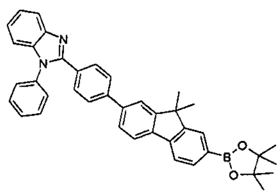 | 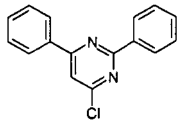 [29509-91-9] | 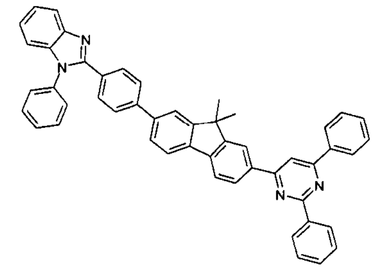 | C | 34% |
| 6n | 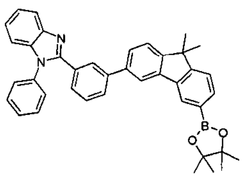 | 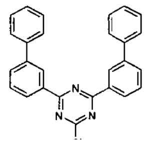 [1205748-61-3] | 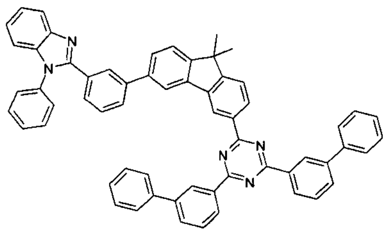 | C | 44% |
| 6o | 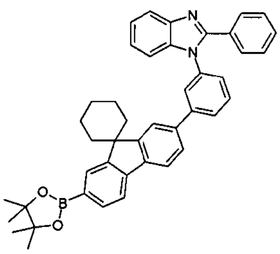 | 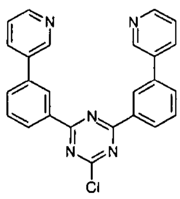 [1396311-93-5] | 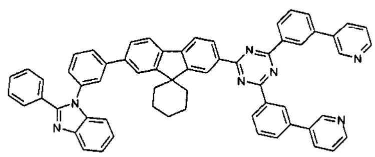 | A | 53% |
| 6p | 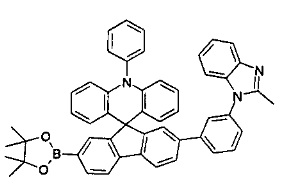 | 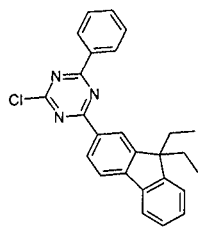 [1015814-06-8] | 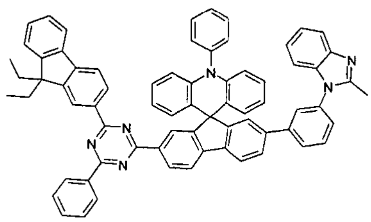 | C | 42% |
| 6q | 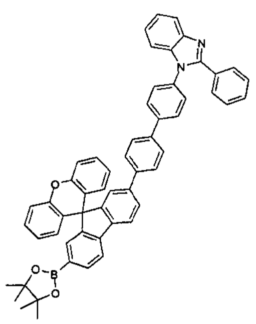 | 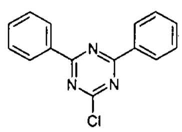 [3842-55-5] | 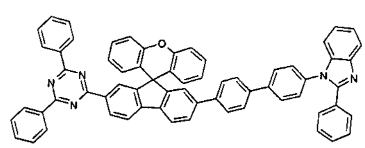 | B | 31% |

(fortgesetzt)

| Nr. | Edukt 4 | Edukt 5 | Produkt 6 | Var. | Ausb |
|---|---|---|---|---|---|
| 6r | 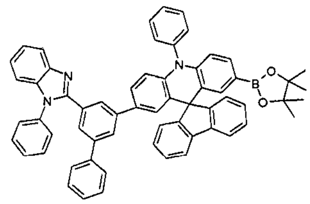 | 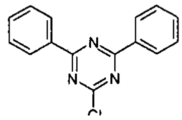 [3842-55-5] | 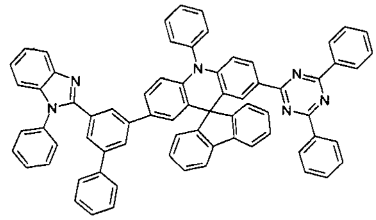 | B | 35% |
| | 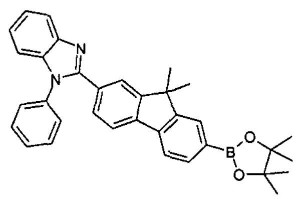 | 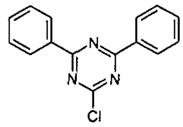 [3842-55-5] | 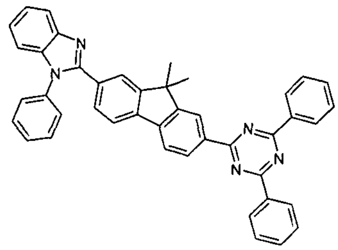 | B | 39% |
| *Var. - Variante; Ausb. - Ausbeute* | | | | | |

**Beispiel 2**

**2-(3-[1-phenyl-1H-benzimidazol-2-yl)phenyl]-4-Phenyl-6-(9,9'-spirobi[9H-fluoren]-2-yl-1,3,5-triazin 11a**

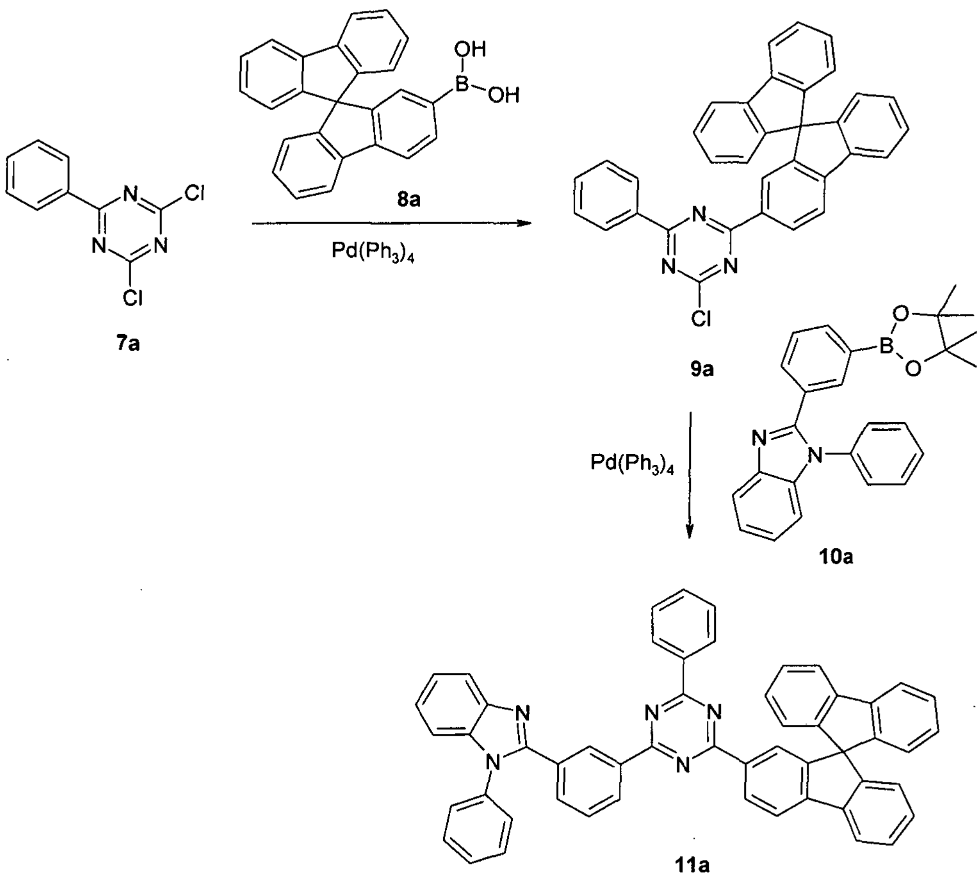


11a

**Synthese von 2-Chlor-4-Phenyl-6-(9,9'-spirobi[9H-fluoren]-2-yl)-1,3,5-triazin 9a**

**Variante A**

In einem 2 L-Vierhalskolben werden unter Schutzgas 24.5 g (67.9 mmol, 1.00 eq) 9,9'-Spirobifluoren-2'-yl-brononsäure **7a,** 15.4 g (68.3 mmol, 1.01 eq) 2,4-Dichloro-6-phenyl-[1,3,5]triazin **8a** und 9.04 g (85.3 mmol, 1.26 eq) Natriumcarbonat in 300 ml Toluol, 300 ml 1,4-Dioxan und 300 ml VE-Wasser vorgelegt und entgast. Anschließend wird 0.850 g (0.736 mmol, 0.01 eq) Tetrakis(triphenylphosphin)-palladium(0) zugegeben 24 Stunden am Rückfluss erhitzt. Nach beendeter Reaktion wird der Ansatz abgekühlt und mit 100 ml Ethylacetat verdünnt. Die Phasen werden im Scheidetrichter getrennt, die wässrige Phase dreimal mit Ethylacetat extrahiert und die vereinten organischen Phasen noch einmal mit Wasser gewaschen. Anschließend wird über Natriumsulfat getrocknet und die Lösung eingeengt bis ein bräunlicher Feststoff ausfällt. Der Feststoff wird abfiltriert, mit heißem Ethanol ausgerührt und nach erneuter Filtration im Vakuum getrocknet. Die Aufreinigung des Produkts erfolgt mittels Säulenchromatographie unter Verwendung von Heptan/Dichlormethan 5:1 als Laufmittel. Es werden 9.90 g (19.6 mmol, 29%) eines farblosen Feststoffs erhalten.

**Variante B**

Die Durchführung erfolgt analog zu der von Variante A, wobei anstelle von Tetrakis(triphenylphosphin)palladium(0) 0.01 eq Palladium(II)-acetat und 0.04eq Tri(o-Tolyl)phosphin verwendet werden.

**Variante C**

Die Durchführung erfolgt analog zu der von Variante A, wobei anstelle von Tetrakis(triphenylphosphin)palladium(0) 0.01 eq Palladium(II)-acetat und 0.01eq Dicyclohexyl-(2',6'-dimethoxy-biphenyl-2-yl-phophan (SPhos) verwendet werden.

**Variante D**

Die Durchführung erfolgt analog zu der von Variante A, wobei anstelle von Tetrakis(triphenylphosphin)palladium(0) 0.02 eq 1,1-Bis(diphenylphos-phino)ferrocen-dichloropalladium(II)-Komplex mit DCM und getrocknetes Toluol als Lösungsmittel verwendet werden.

Analog werden hergestellt:

| Nr. | Edukt 7 | Edukt 8 | Produkt 9 | Var. | Ausb. |
|---|---|---|---|---|---|
| 9b | 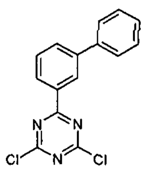 [1402225-89-1] | 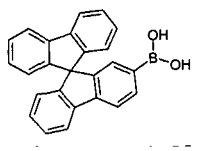 [236389-21-2] | 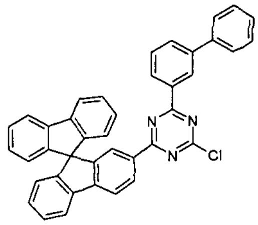 | A | 34% |
| 9c | 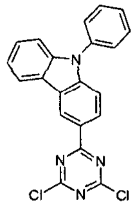 [20354-40-9] | 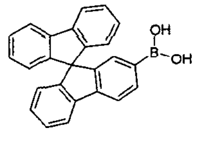 [236389-21-2] | 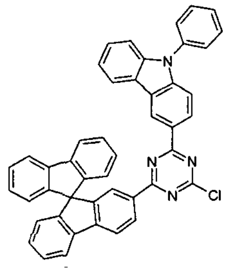 | B | 29% |

(fortgesetzt)

| Nr. | Edukt 7 | Edukt 8 | Produkt 9 | Var. | Ausb. |
|---|---|---|---|---|---|
| **9d** | 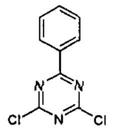 [1700-02-3] | 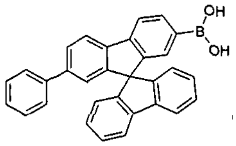 [1361305-38-5] | 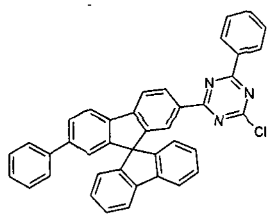 | B | 41% |
| **9e** | 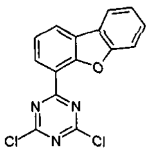 [51800-19-2] | 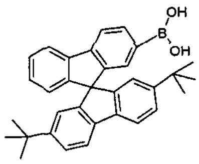 [1241891-39-3] | 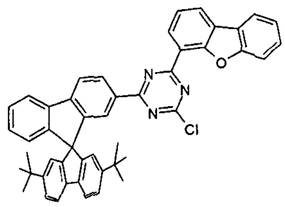 | C | 17% |
| **9f** | 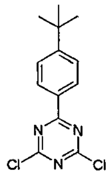 [1491751-928] | 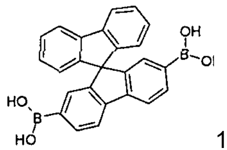 1 [1222537-26-9] | 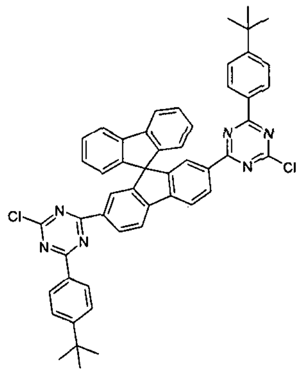 | A | 26% |
| **9g** | 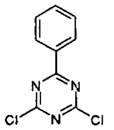 [1700-02-3] | 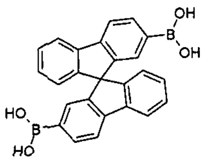 [1222007-94-4] | 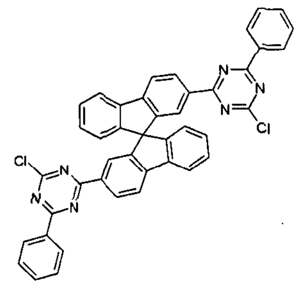 | A | 11% |
| **9h** | 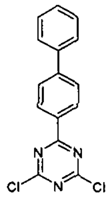 [10202-45-6] | 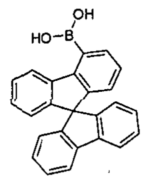 [1421789-05-0] | 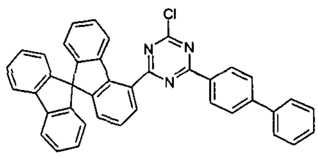 | B | 25% |

(fortgesetzt)

| Nr. | Edukt 7 | Edukt 8 | Produkt 9 | Var. | Ausb. |
|---|---|---|---|---|---|
| 9i | 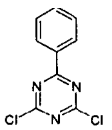 [1700-02-3] | 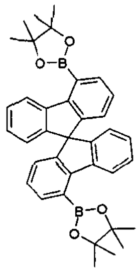 [1257321-47-3] | 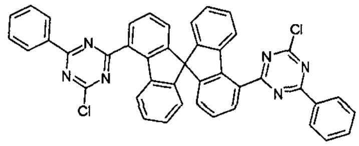 | D | 18% |
| 9j | 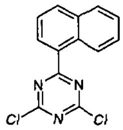 [59336-36-6] | 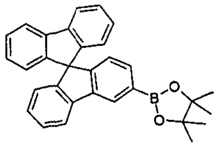 [1346007-05-3] | 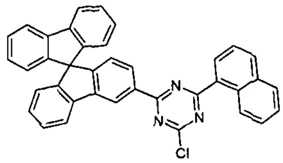 | B | 54% |
| 9k | 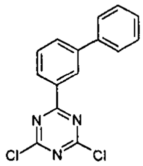 [1402225-89-1] | 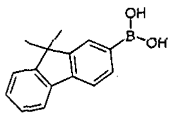 [333432-28-3] | 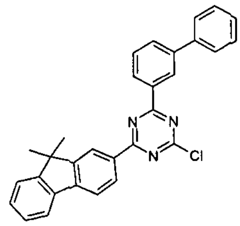 | C | 41% |
| 9l | 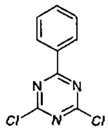 [1700-02-3] | 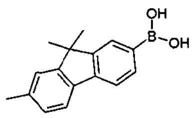 [1454287-24-1] | 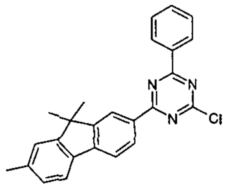 | B | 31% |
| 9m | 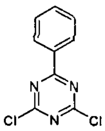 [1700-02-3] | 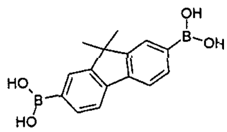 [866100-14-3] | 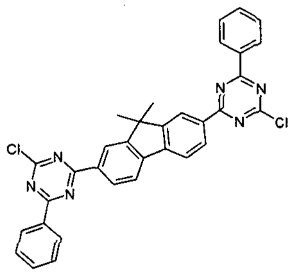 | A | 19% |
| 9n | 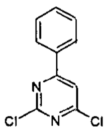 26032-72-4] | 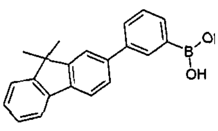 [1092840-71-5] | 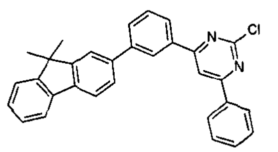 | B | 38% |

(fortgesetzt)

| Nr. | Edukt 7 | Edukt 8 | Produkt 9 | Var. | Ausb. |
|---|---|---|---|---|---|
| **9o** | 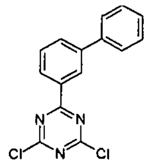 [1402225-89-1] | 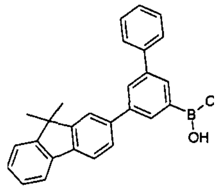 [881-11-75-7] | 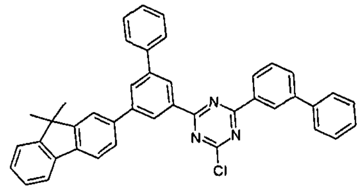 | C | 42% |
| **9p** | 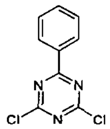 [1700-02-3] | 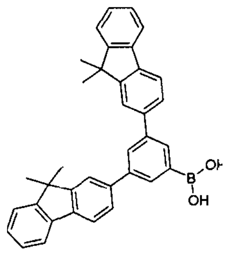 [881913-13-9] | 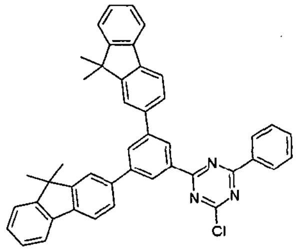 | A | 51% |
| **9q** | 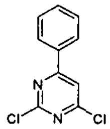 [26032-72-4] | 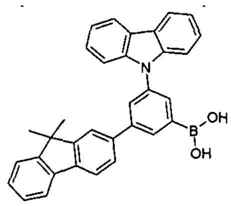 [1292291-83-8] | 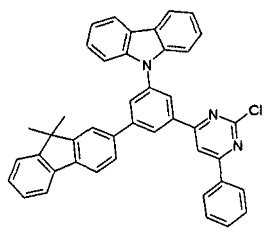 | A | 24% |
| **9r** | 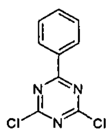 [1700-02-3] | 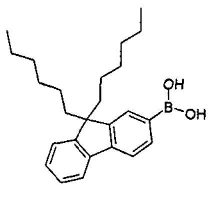 [400607-32-1] | 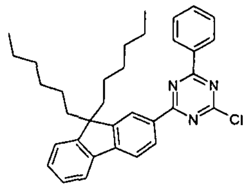 | D | 18% |
| **9s** | 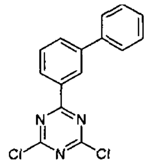 [1402225-89-1] | 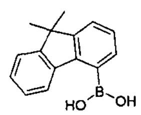 [1246022-50-3] | 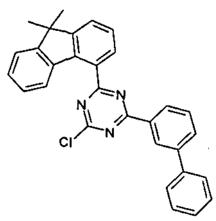 | A | 36% |
| **9t** | 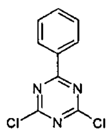 [1700-02-3] | 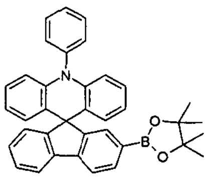 [1241891-65-5] | 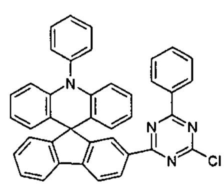 | B | 47% |
| **9u** | 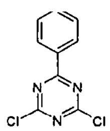 [1700-02-3] | 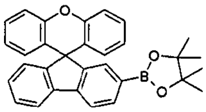 [1160862-12-3] | 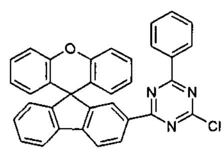 | B | 27% |

(fortgesetzt)

| Nr. | Edukt 7 | Edukt 8 | Produkt 9 | Var. | Ausb. |
|---|---|---|---|---|---|
| 9v | 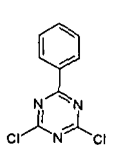 [1700-02-3] | 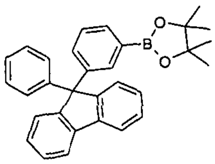 [1260032-45-8] | 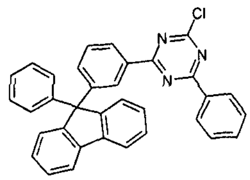 | D | 42% |
| *Var. - Variante; Ausb. - Ausbeute* | | | | | |

**Synthese von 2-(3-[1-phenyl-1H-benzimidazol-2-yl)phenyl]-4-Phenyl-6-(9,9’-spirobi[9*H*-fluoren]-2-yl-1,3,5-triazin 11a**

**Variante A**

In einem 250 ml-Dreihalskolben werden unter Schutzgas 9.90 g (19.6 mmol, 1.00 eq) 2-Chlor-4-Phenyl-6-(9,9’-spirobi[9*H*-fluoren]-2-yl-1,3,5-triazin **9a,** 8.53 g (21.5 mmol, 1.10 eq) 22-Phenyl-2-[3-(4,4,5,5-tetramethyl-[1,3,2]dioxaborolan-2-yl)-phenyl]1*H*-benzimidazol **10a** und 4.57 g (43.1 mmol, 2.20 eq) Natriumcarbonat in 35 ml Toluol, 35 ml 1,4-Dioxan und 35 ml VE-Wasser vorgelegt und entgast. Anschließend wird 1.13 g (0.980 mmol, 0.05 eq) Tetrakis(triphenylphosphin)-palladium(0) zugegeben 48 Stunden am Rückfluss erhitzt. Nach beendeter Reaktion wird der Ansatz abgekühlt und der ausgefallene Feststoff abgesaugt. Das erhaltene Rohprodukt wird mittel *Extraktion,* dreifacher Umkristallisation aus Heptan/Toluol und Sublimation aufgereingt. Es werden 4.83 g (6.54 mmol, 33%) eines Feststoffs mit einer HPLC Reinheit >99.9% erhalten.

**Variante B**

Die Durchführung erfolgt analog zu der von Variante A, wobei anstelle von Tetrakis(triphenylphosphin)palladium(0) 0.02 eq 1,1-Bis(diphenylphos-phino)ferrocen-dichloropalladium(II)-Komplex mit DCM und getrocknetes Toluol als Lösungsmittel verwendet werden.

**Variante C**

Zu einer Lösung aus 10.2 g (22.2 mmol, 1.00 eq) 2-Biphenyl-3-yl-4-chloro-6-(9,9-dimethyl-9H-fluoren-2-yl)-[1,3,5]triazin **9k** und 4.31 g (22.2 mmol, 1.00 eq) 2-Phenyl-1H-benzimidazol **10k** werden in 150 ml THF bei Raumtemperatur 4.15 ml (24.4 mmol, 1.10 eq) Diisopropylethylamin (Hünig-Base) zugegeben. Das Reaktionsgemisch wird über Nacht gerührt und nach beendeter Reaktion das Lösungsmittel im Vakuum entfernt. Nach Zugabe von 200 ml Dichlormethan wird die Lösung dreimal mit Wasser extrahiert, über Natriumsulfat getrocknet und das Lösungsmittel am Rotationsverdampfer entfernt. Die Aufreinigung erfolgt analog Variante A. Es werden 5.57 g (9.02 mmol, 41%) des gewünschten Produkts **11k** erhalten.

Analog werden hergestellt:

| Nr. | Edukt 9 | Edukt 10 | Produkt 11 | Var | Ausb |
|---|---|---|---|---|---|
| 11b | 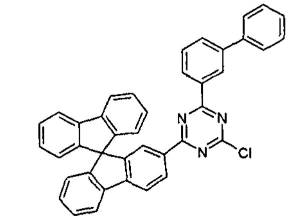 | 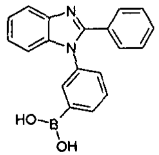 [1256723-93-9] | 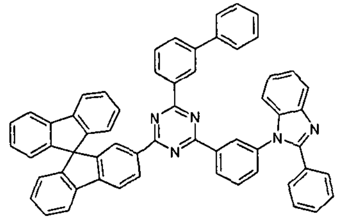 | B | 71% |
| 11c | 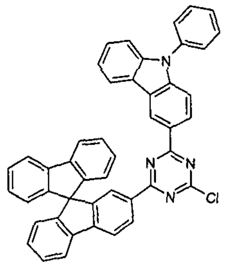 | 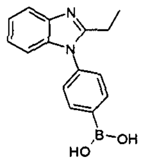 [1507395-74-5] | 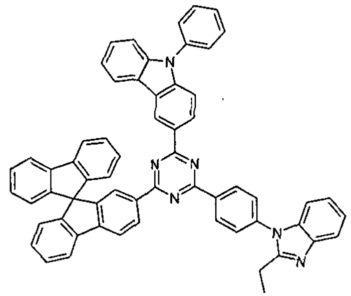 | B | 43% |
| 11d | 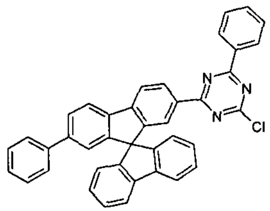 | 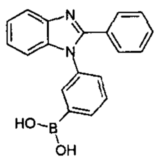 [1256723-93-9] | 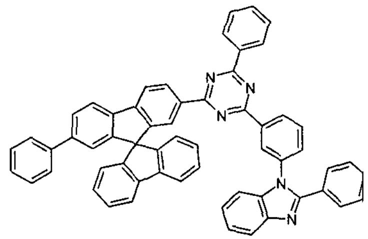 | B | 55% |
| 11e | 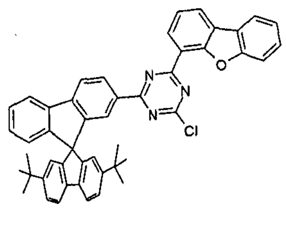 | 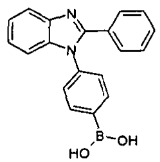 [867044-33-5] | 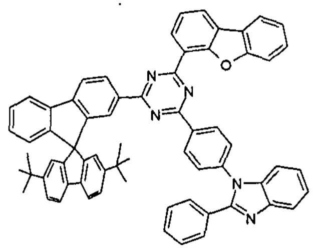 | A | 29% |

(fortgesetzt)

| Nr. | Edukt 9 | Edukt 10 | Produkt 11 | Var | Ausb |
|---|---|---|---|---|---|
| **11f** | 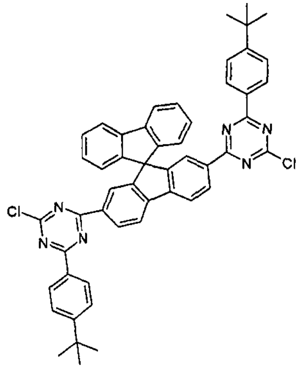 | 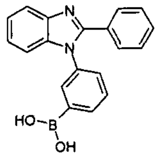 [1256723-93-9] | 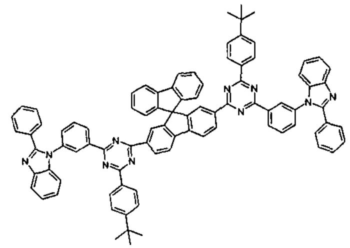 | B | 47% |
| **11g** | 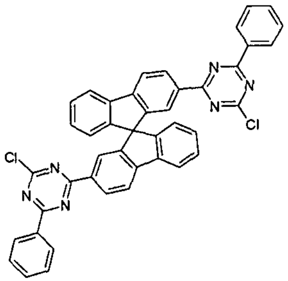 | 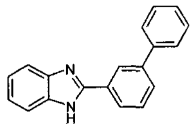 [897445-67-9] | 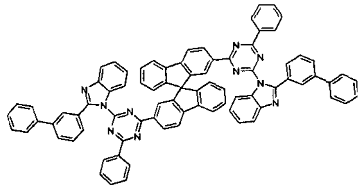 | C | 63% |
| **11h** | 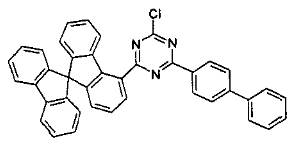 | 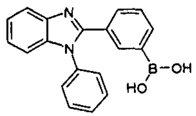 [1214723-26-8] | 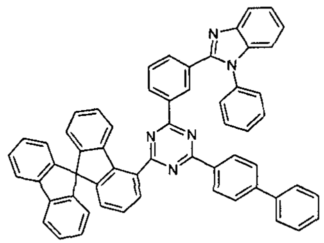 | A | 47% |

(fortgesetzt)

| Nr. | Edukt 9 | Edukt 10 | Produkt 11 | Var | Ausb |
|---|---|---|---|---|---|
| **11i** | 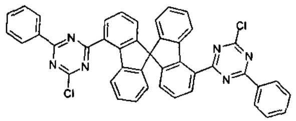 | 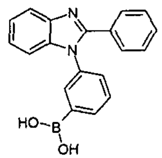 [1256723-93-9] | 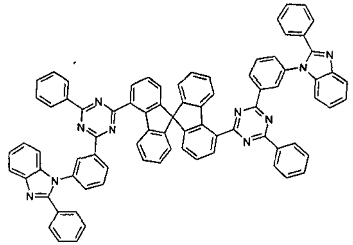 | A | 31% |
| **11j** | 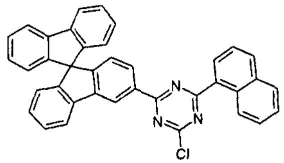 | 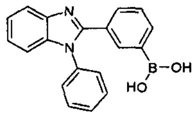 [1214723-26-8] | 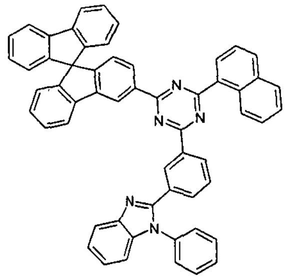 | B | 36% |
| **11k** | 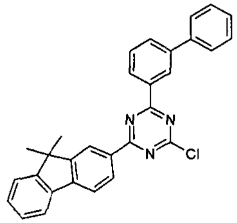 | 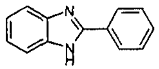 [716-79-0] | 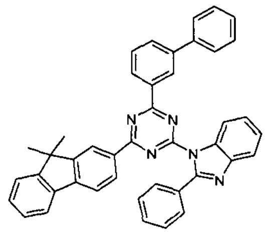 | B | 41% |
| **11l** | 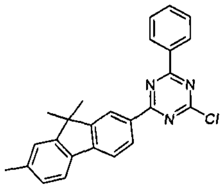 | 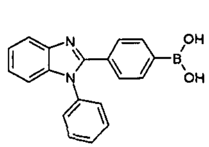 [952514-79-3] | 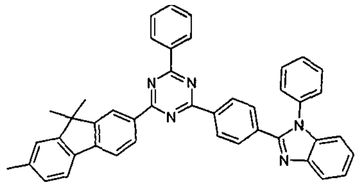 | A | 58% |

(fortgesetzt)

| Nr. | Edukt 9 | Edukt 10 | Produkt 11 | Var | Ausb |
|---|---|---|---|---|---|
| **11m** | 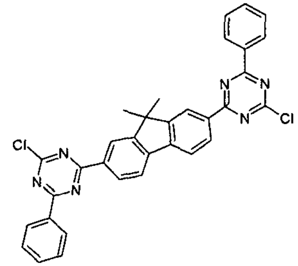 | 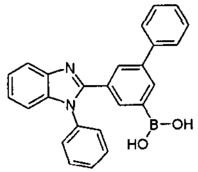 [1172134-16-5] | 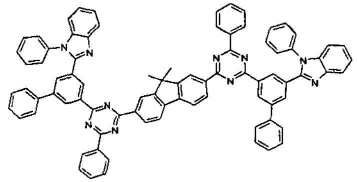 | A | 26% |
| **11n** | 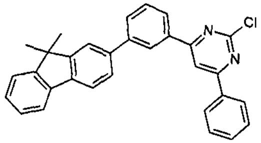 | 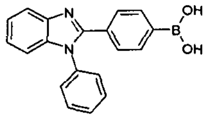 [952514-79-3] | 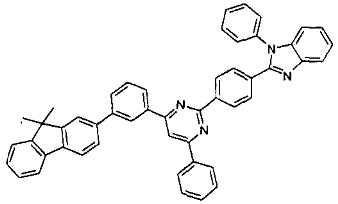 | B | 74% |
| **11o** | 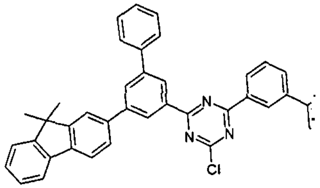 | 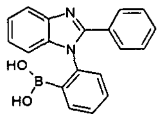 [1553408-82-4] | 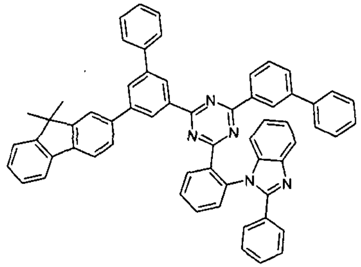 | A | 42% |

(fortgesetzt)

| Nr. | Edukt 9 | Edukt 10 | Produkt 11 | Var | Ausb |
|---|---|---|---|---|---|
| **11p** | 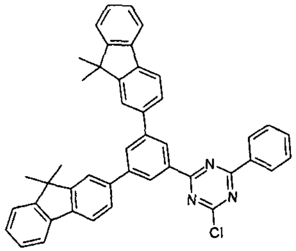 | 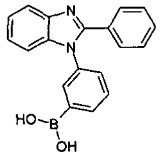 [1256723-93-9] | 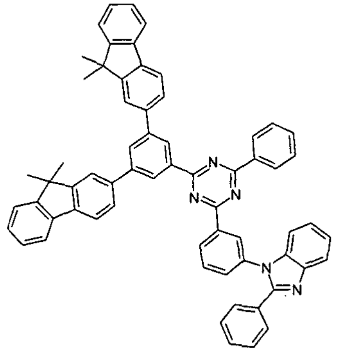 | A | 37% |
| **11q** | 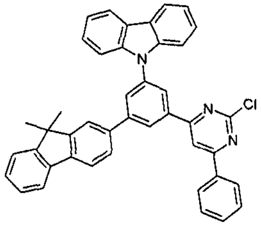 | 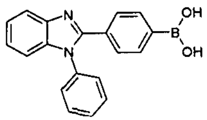 [952514-79-3] | 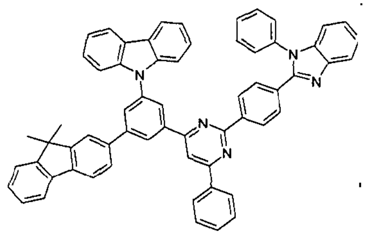 | B | 32% |
| **11r** | 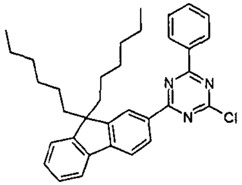 | 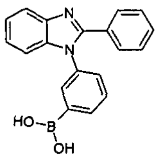 [1256723-93-9] | 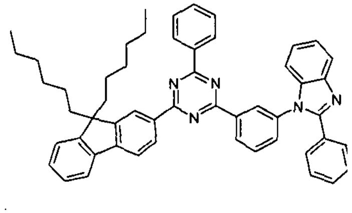 | A | 21% |
| **11s** | 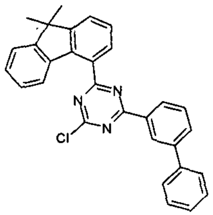 | 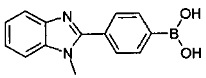 [1191061-90-1] | 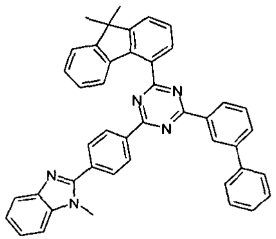 | A | 62% |

(fortgesetzt)

| Nr. | Edukt 9 | Edukt 10 | Produkt 11 | Var | Ausb |
|---|---|---|---|---|---|
| **11t** | 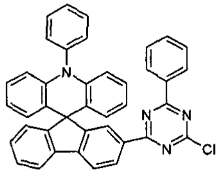 | 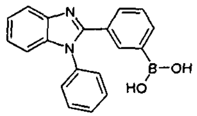 [1214723-26-8] | 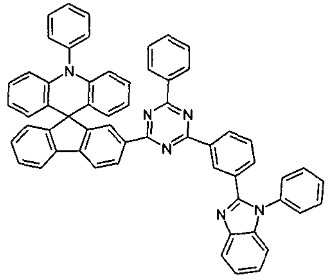 | B | 33% |
| **11u** | 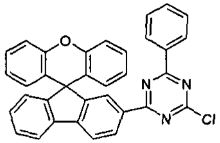 | 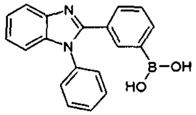 [1214723-26-8] | 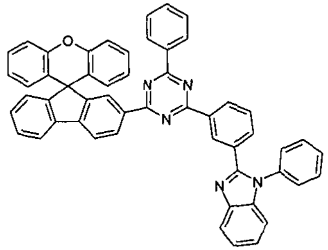 | A | 29% |
| **11v** | 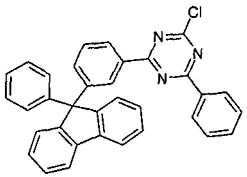 | 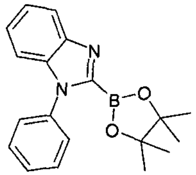 [1316275-47-4] | 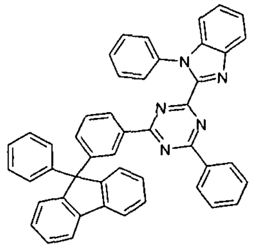 | A | 58% |
| *Var- Variante; Ausb - Ausbeute* | | | | | |

**Beispiel 3**

**Herstellung und Charakterisierung der OLEDs**

In den folgenden Beispielen V1 bis E16 (siehe Tabellen 1 und 2) werden die Daten verschiedener OLEDs vorgestellt.

Vorbehandlung für die Beispiele V1-E16: Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly-(3,4-ethylendioxythiophen) poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC3:TEG1 (55%:35%:10%) bedeutet hierbei, dass das Material IC1 in einem Volumenanteil von 55%, IC3 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 und LE1000 bezeichnen die St rom- bzw. Leistungseffizienz, die bei 1000 cd/$m^2$ erreicht werden. EQE1000 schließlich bezeichnet die externe Quanteneffizienz bei einer Betriebsleuchtdichte von 1000 cd/$m^2$. Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/$m^2$ und L1 = 70% in Tabelle 2 bedeutet, dass die in Spalte LD angegebene Lebensdauer der Zeit entspricht, nach der die Anfangsleuchtdichte von 4000 cd/$m^2$ auf 2800 cd/$m^2$ absinkt. Analog bedeutet L0;j0 = 20 mA/$cm^2$, L1 = 80%, dass die Leuchtdichte bei Betrieb mit 20 mA/$cm^2$ nach der Zeit LD auf 80% ihres Anfangswertes absinkt.

Die Daten der verschiedenen OLEDs sind in Tabelle 2 zusammengefasst. Die Beispiele V1-V3 sind Vergleichsbeispiele gemäß dem Stand der Technik, die Beispiele E1-E16 zeigen Daten von erfindungsgemäßen OLEDs.

Im Folgenden werden einige der Beispiele näher erläutert, um die Vorteile der erfindungsgemäßen OLEDs zu verdeutlichen.

**Verwendung von erfindungsgemäßen Mischungen in der Elektronentransportschicht (ETL bzw. EIL) von OLEDs**

Die erfindungsgemäßen Materialien ergeben bei Einsatz in der Elektroneninjektions- und Elektronentransportschicht in OLEDs wesentliche Verbesserungen gegenüber dem Stand der Technik bezüglich der Lebensdauer der Bauteile und/oder der Effizienz. Durch Einsatz der erfindungsgemäßen Verbindung EG1 lässt sich eine Steigung der Lebensdauer zwischen 20% und 40% gegenüber dem Stand der Technik beobachten (Vergleich der Beispiele V1 und V2 mit E1, sowie Vergleich von V3 mit E2). Ein weiterer technischer Vorteil der erfindungsgemäßen Verbindungen ist eine erhöhte Effizienz um ca. 10% gegenüber dem Stand der Technik (Vergleich von Versuch V1 mit E2).

**Tabelle 1:** Aufbau der OLEDs (die Beispiele E1, E9 und E15 sind nicht Teil der Erfindung):

| HTL/IL (HATCN; 5 nm)/EBL/EML/HBL/ETL/EIL | | | | | | |
|---|---|---|---|---|---|---|
| Bsp | HTL Dicke | EBL Dicke | EML Dicke | HBL Dicke | ETL Dicke | EIL Dicke |
| V1 | SpA1 70nm | SpMA1 90nm | IC1 :IC3:TEG1 (40%:40%: 20%) 30nm | - | SdT1:LiQ (50%:50%) 40nm | --- |

(fortgesetzt)

| HTL/IL (HATCN; 5 nm)/EBL/EML/HBL/ETL/EIL | | | | | | |
|---|---|---|---|---|---|---|
| Bsp | HTL Dicke | EBL Dicke | EML Dicke | HBL Dicke | ETL Dicke | EIL Dicke |
| V2 | SpA1 70nm | SpMA1 90nm | IC1:IC3:TEG1 (40%:40%: 20%) 30nm | --- | SdT2:LiQ (50%:50%) 40nm | - |
| V3 | SpA1 70nm | SpMA1 90nm | IC1:TEG1 (90%: 10%) 30nm | --- | SdT3 40nm | LiF 1nm |
| E1 | SpA1 70nm | SpMA1 90nm | IC1:IC3:TEG1 (40%:40%: 20%) 30nm | --- | EG1.LiQ (50%:50%) 40nm | --- |
| E2 | SpA1 70nm | SpMA1 90nm | IC1:TEG1 (90%:10% 30nm | - | EG2 40nm | LiF 1nm |
| E3 | SpA1 70nm | SpMA1 90nm | IC1:TEG1 (90%:10%) 30nm | IC1 10nm | EG3:LiQ (50%:50%) 30nm | - |
| E4 | SpA1 70nm | SpMA1 90nm | iC1:TEG1 (90%:10%) 30nm | IC1 10nm | EG4 30nm | LiF 1nm |
| E5 | SpA1 70nm | SpMA1 90nm | IC1:TEG1 (90%:10%) 30nm | EG5 10nm | EG5:LiQ (50%:50%) 30nm | --- |
| E6 | SpA1 70nm | SpMA1 90nm | IC1:TEG1 (90%:10%) 30nm | IC1 10nm | EG6 30nm | LiQ 3nm |
| E7 | SpA1 70nm | SpMA1 90nm | IC1:IC3:TEG1 (60%:30%: 10%) 30nm | IC1 10nm | EG7:LiQ (50%:50%) 30nm | LiQ 1nm |
| E8 | SpA1 140nm | SpMA1 20nm | H1:SEB (95%:5%) 20nm | --- | EG8:LiQ (50%:50%) 30nm | - |
| E9 | SpA1 140nm | SpMA1 20nm | H1:SEB 95%:5% 20nm | --- | EG9 (50%:50% 30nm | LiQ 3nm |
| E10 | SpA1 140nm | SpMA1 20nm | H1:SEB (95%:5%) 20nm | --- | EG10:LiQ (50%: 50%) 30nm | - |
| E14 1 | SpA1 140nm | SpMA1 20nm | H1:SEB (95%:5%) 20nm | - | EG11:LiQ (50%: 50%) 30nm | - |
| E12 | SpA1 140nm | SpMA1 20nm | H1:SEB 95%:5% 20nm | - | EG12:LiQ (50%:50% 30nm | - |
| E13 | SpA1 70nm | SpMA1 90nm | EG13:IC3:TEG1 45%: 45%:10% 30nm | --- | ST2:LiQ (50%:50% 40nm | - |
| E14 | SpA1 90nm | SpMA1 130nm | IC5:TER3 (92%:8%) 40nm | --- | EG14:LiQ (50%: 50%) 40nm | --- |
| E15 | SpA1 90nm | SpMA1 130nm | IC5:TER3 (92%:8%) 40nm | --- | EG15:LiQ (50%: 50%) 40nm | - |
| E16 | SpA1 90nm | SpMA1 130nm | IC5:TER3 (92%:8%) 40nm | - | EG16:LiQ (50%: 50%) 40nm | --- |

**Tabelle 2:** Daten der OLEDs

| Bsp. | U1000 (V) | SE1000 (cd/A) | LE1000 (lm/W) | EQE 1000 | CIE x/y bei 1000 cd/$m^2$ | $L_0;j_0$ | L1 % | LD (h) |
|---|---|---|---|---|---|---|---|---|
| V1 | 3.4 | 55 | 51 | 15.1% | 0.33/0.63 | 20 mA/$cm^2$ | 80 | 180 |
| V2 | 3.3 | 60 | 57 | 16.5% | 0.33/0.62 | 20 mA/$cm^2$ | 80 | 160 |

(fortgesetzt)

| Bsp. | U1000 (V) | SE1000 (cd/A) | LE1000 (lm/W) | EQE 1000 | CIE x/y bei 1000 $cd/m^2$ | $L_0;j_0$ | L1 % | LD (h) |
|---|---|---|---|---|---|---|---|---|
| V3 | 3.2 | 61 | 60 | 16.8% | 0.34/0.62 | 20 $mA/cm^2$ | 80 | 125 |
| E1 | 3.3 | 59 | 56 | 16.4% | 0.33/0.62 | 20 $mA/cm^2$ | 80 | 220 |
| E2 | 3.2 | 62 | 61 | 16.7% | 0.33/0.63 | 20 $mA/cm^2$ | 80 | 155 |
| E3 | 3.4 | 57 | 53 | 16.2% | 0.34/0.62 | 20 $mA/cm^2$ | 80 | 135 |
| E4 | 3.5 | 60 | 54 | 16.5% | 032/0.64 | 20 $mA/cm^2$ | 80 | 130 |
| E5 | 3.6 | 59 | 51 | 16.0% | 0.33/0.63 | 20 $mA/cm^2$ | 80 | 120 |
| E6 | 3.2 | 60 | 59 | 16.1% | 0.31/0.64 | 20 $mA/cm^2$ | 80 | 105 |
| E7 | 3.6 | 61 | 53 | 16.6% | 0.33/0.63 | 20 $mA/cm^2$ | 80 | 235 |
| E8 | 4.6 | 8.1 | 5.5 | 7.2% | 0.13/014 | 6000 $cd/m^2$ | 80 | 50 |
| E9 | 4.5 | 7.8 | 5.2 | 6.7% | 0.14/015 | 6000 $cd/m^2$ | 80 | 25 |
| E13 | 4.9 | 7.4 | 4.7 | 6.9% | 0.14/0.13 | 6000 $cd/m^2$ | 80 | 45 |
| E11 | 4.7 | 8.3 | 5.5 | 7.5% | 0.14/013 | 6000 $cd/m^2$ | 80 | 40 |
| E12 | 5.2 | 8.5 | 5.1 | 7.7% | 0.14/013 | 6000 $cd/m^2$ | 80 | 35 |
| E13 | 3.4 | 58 | 54 | 15.4% | 0.32/0.63 | 20 $mA/cm^2$ | 80 | 105 |
| E14 | 4.9 | 11.3 | 7.2 | 12.1% | 0.67/0.33 | 4000 $cd/m^2$ | 80 | 390 |
| E15 | 4.3 | 11.3 | 8.3 | 12.3% | 0.66/0.34 | 4000 $cd/m^2$ | 80 | 360 |
| E16 | 4.5 | 12.2 | 8.5 | 12.8% | 0.67/0.33 | 4000 $cd/m^2$ | 80 | 410 |

**Tabelle 3:** Strukturformeln der Materialien für die OLEDs

| | |
|---|---|
| 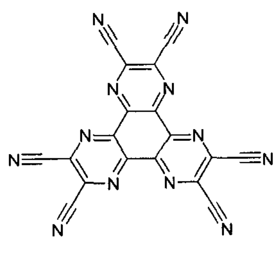 | 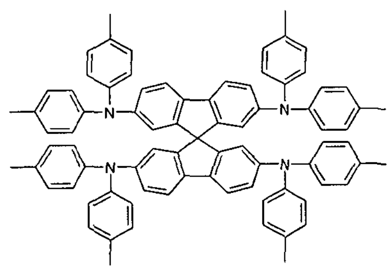 |
| HATCN | SpA1 |
| 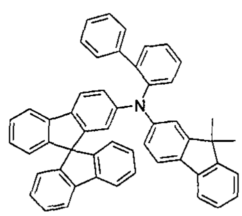 | 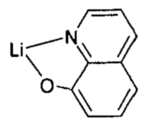 |
| SpMA1 | LiQ |

(fortgesetzt)

| | |
|---|---|
| 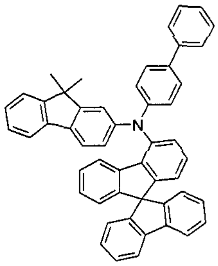 | 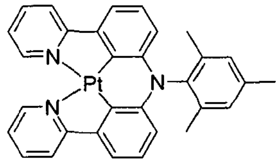 |
| SpMA2 | TER1 |
| 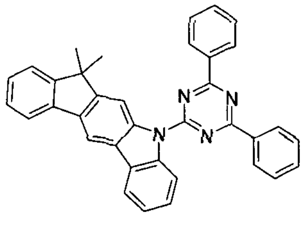 | 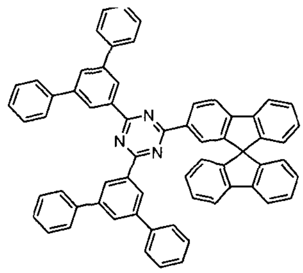 |
| IC1 | ST2 |
| 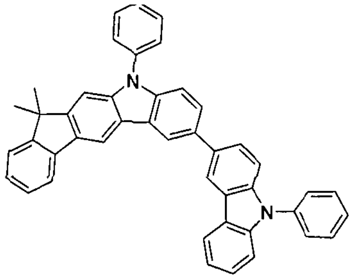 | 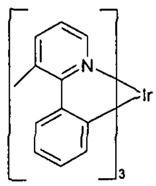 |
| IC3 | TEG1 |
| 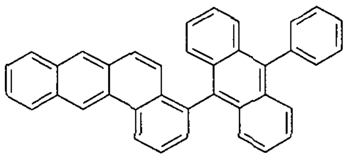 | 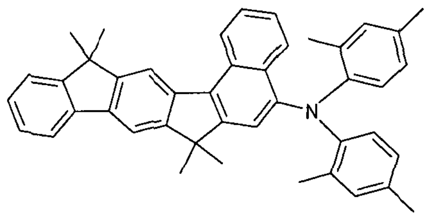 |
| H1 | SEB |
| 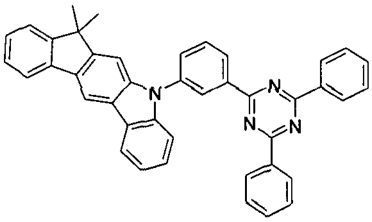 | 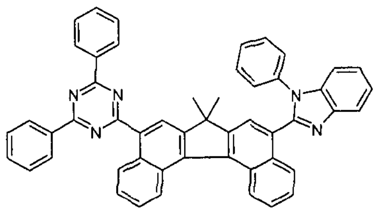 |
| IC5 | SdT1 |

(fortgesetzt)

| | |
|---|---|
| 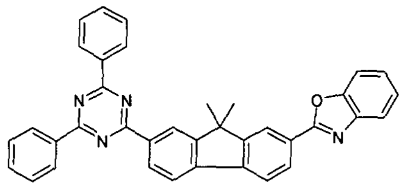 | 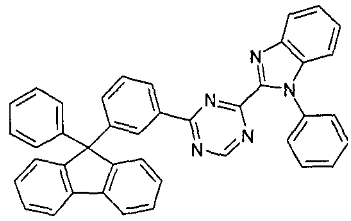 |
| SdT2 | SdT3 |
| 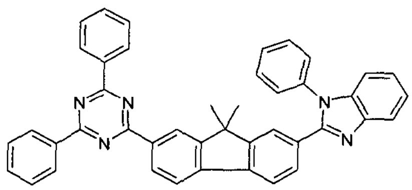 | 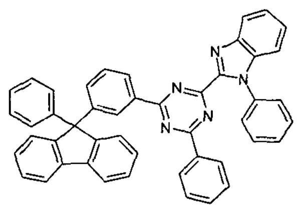 |
| EG1 | EG2 |
| 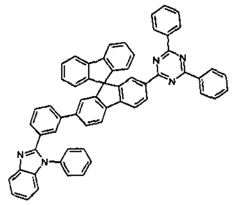 | 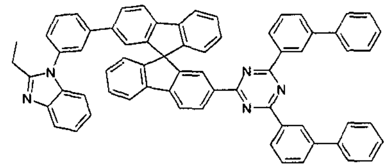 |
| EG3 | EG4 |
| 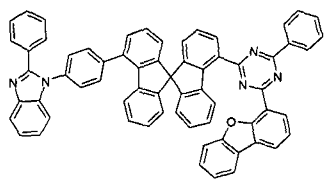 | 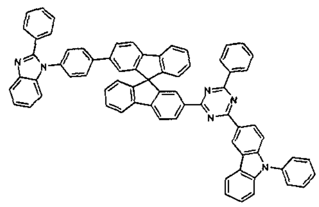 |
| EG5 | EG6 |
| 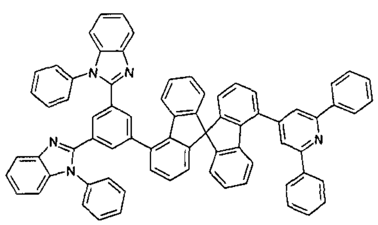 | 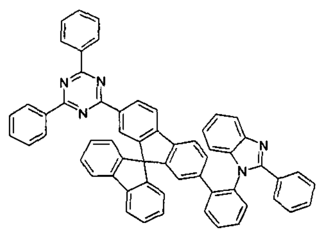 |
| EG7 | EG8 |
| 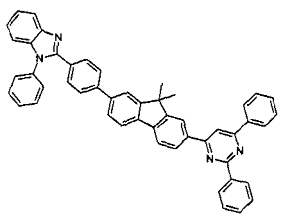 | 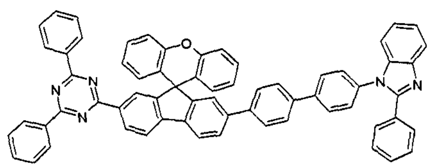 |
| EG9 | EG10 |

(fortgesetzt)

| | |
|---|---|
| 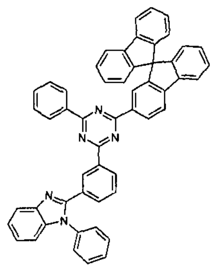 | 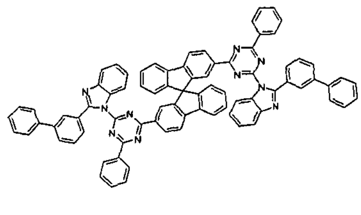 |
| EG11 | EG12 |
| 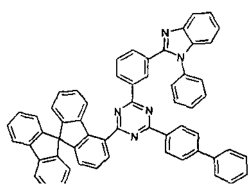 | 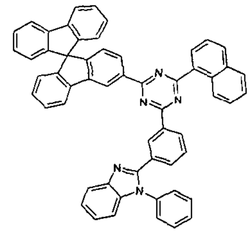 |
| EG13 | EG14 |
| 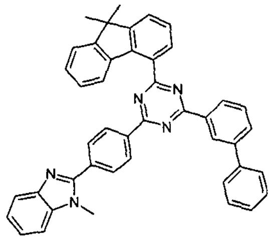 | 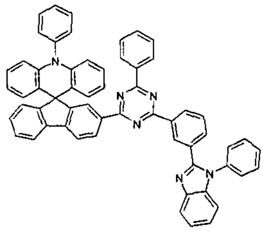 |
| EG15 | EG16 |

## Patentansprüche

**1.** Verbindung der Formel (1)

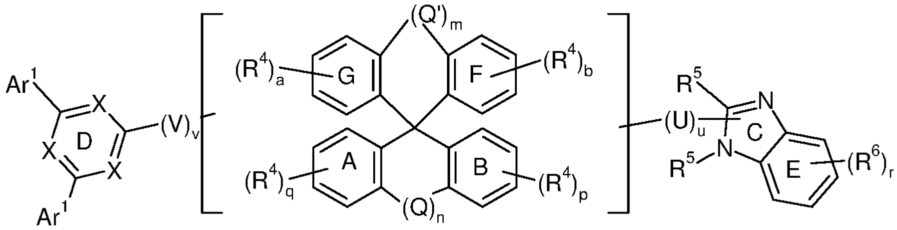

Formel (1)

wobei für die verwendeten Symbole und Indizes folgende Definitionen gelten:

$R^1$ ist bei jedem Auftreten gleich oder verschieden H, D, F, Cl, Br, I, CHO, $N(Ar^1)_2$, $C(=O)Ar^1$, $P(=O)(Ar^1)_2$, $S(=O)Ar^1$, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere bevorzugt nicht benachbarte $CH_2$-Gruppen durch $R^2C=CR^2$, C=C , $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein

oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Kombination dieser Systeme; dabei können zwei oder mehrere benachbarte Substituenten $R^1$ miteinander ein mono- oder polycyclisches, aliphatisches oder aromatisches Ringsystem bilden;

$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^3$ substituiert sein kann, wobei eine oder mehrere nicht benachbarte CH2-Gruppen durch $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy- , Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches Ringsystem bilden; wobei bevorzugt ist, wenn zwei oder mehrere benachbarte Substituenten $R^2$ miteinander kein mono- oder polycyclisches, aliphatisches oder aromatisches Ringsystem bilden;

R3 ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches Ringsystem bilden; wobei bevorzugt ist, wenn zwei oder mehrere benachbarte Substituenten $R^3$ miteinander kein mono- oder polycyclisches, aliphatisches oder aromatisches Ringsystem bilden;

R5 ist bei jedem Auftreten gleich oder verschieden F, Cl, Br, I, CHO, $N(Ar^1)2$, $C(=O)Ar^1$, $P(=O)(Ar^1)_2$, S(=O)Ar1, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere bevorzugt nicht benachbarte $CH_2$-Gruppen durch $R^2C=CR^2$, C=C , $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Kombination dieser Systeme; wobei zwei oder mehrere benachbarte Substituenten $R^5$ miteinander kein mono- oder polycyclisches, aliphatisches oder aromatisches Ringsystem bilden können;

$R^4$ und $R^6$ sind bei jedem Auftreten gleich oder verschieden H, D, F, Cl, Br, I, CHO, $N(Ar^1)2$, $C(=O)Ar^1$, $P(=O)(Ar^1)_2$, S(=O)Ar1, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere bevorzugt nicht benachbarte $CH_2$-Gruppen durch $R^2C=CR^2$, C=C , $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Kombination dieser Systeme; wobei zwei oder mehrere benachbarte Substituenten $R^4$ miteinander kein mono- oder polycyclisches, aliphatisches oder aromatisches Ringsystem bilden können; wobei zwei oder mehrere benachbarte Substituenten $R^6$ miteinander ein mono- oder polycyclisches, aliphatisches oder aromatisches Ringsystem bilden können, wobei bevorzugt ist wenn zwei oder mehrere benachbarte Substituenten $R^6$ miteinander kein mono- oder polycyclisches, aliphatisches oder aromatisches Ringsystem bilden können;

Q ist C=O, C=S, S, $C(R^2)_2$, $NR^2$ oder O, bevorzugt $C(R^2)_2$, $NR^2$ oder O, ganz bevorzugt $C(R^2)_2$ oder $NR^2$ und

besonders bevorzugt $C(R^2)2$;
n ist 0 oder 1, wobei n = 0 bevorzugt ist und n = 0 bedeutet, dass die beiden aromatischen Ringe A und B nicht über die Gruppe Q, sondern durch eine Einfachbindung miteinander verknüpft sind;
p ist 0, 1, 2, 3 oder 4, bevorzugt 0, 1, oder 2, ganz bevorzugt 0 oder 1 und besonders bevorzugt gleich 0;
q ist 0, 1, 2, 3 oder 4, bevorzugt 0, 1, oder 2, ganz bevorzugt 0 oder 1 und besonders bevorzugt gleich 0;
r ist 0, 1, 2, 3 oder 4, bevorzugt 0, 1 oder 2, ganz bevorzugt 0 oder 1 und besonders bevorzugt ist r gleich 0;
X ist gleich oder verschieden bei jedem Auftreten N oder $CR^1$, wobei wenigstens ein X gleich N ist, bevorzugt sind wenigstens zwei der X gleich N und ganz bevorzugt sind alle drei X gleich N;
$Ar^1$ ist bei jedem Auftreten gleich oder verschieden ein aromatisches oder heteroaromatisches Ringsystem mit 5 bis 30 aromatischen Ringatomen, das mit einem oder mehreren Resten $R^2$ substituiert sein kann; dabei können auch zwei der Reste $Ar^1$ durch eine Einfachbindung oder eine Brücke, ausgewählt aus $B(R^2)$, $C(R^2)_2$, $Si(R^2)_2$, C=O, $C=NR^2$, $C=C(R^2)2$, O, S, S=O, $SO_2$, $N(R^2)$, $P(R^2)$ und $P(=O)R^2$, miteinander verknüpft sein; bevorzugt ist, wenn zwei Reste $Ar^1$ nicht miteinander verknüpft sind;
Q' sind, gleich oder verschieden voneinander, C=O, C=S, S, $C(R^2)_2$, $NR^2$ oder O, bevorzugt $C(R^2)_2$, $NR^2$ oder O, ganz bevorzugt $C(R^2)_2$ oder $NR^2$ und besonders bevorzugt $C(R^2)_2$;
a ist 0, 1, 2, 3 oder 4, bevorzugt 0, 1, oder 2, ganz bevorzugt 0 oder 1 und besonders bevorzugt gleich 0;
b ist 0, 1, 2, 3 oder 4, bevorzugt 0, 1, oder 2, ganz bevorzugt 0 oder 1 und besonders bevorzugt gleich 0;

dabei gilt, dass a+b immer kleiner oder gleich 7 ist, bevorzugt ist a+b gleich 0, 1, 2 oder 3, ganz bevorzugt ist a+b gleich 0, 1 oder 2, besonders bevorzugt ist a+b gleich 0 oder 1 und ganz besonders bevorzugt ist a+b gleich 0;

m ist 0 oder 1, wobei m = 0 bevorzugt ist und m = 0 bedeutet, dass die beiden aromatischen Ringe nicht über die Gruppe Q', sondern durch eine Einfachbindung miteinander verknüpft sind;
V ist eine bivalente Gruppe;
U ist eine bivalente Gruppe;
v ist 0 oder 1, wobei v = 0 bedeutet, dass der Ring D direkt über eine kovalente Einfachbindung mit dem Rest der Verbindung verbunden ist;
u ist 0 oder 1; wobei u = 0 bedeutet, dass der Ring C direkt über eine kovalente Einfachbindung mit dem Rest der Verbindung verbunden ist.

2. Verbindung gemäß Anspruch 1, **dadurch gekennzeichnet, dass** es sich bei der Verbindung um ein kleines Molekül mit einem Molekulargewicht von maximal 3000 g/mol handelt.

3. Verbindung gemäß Anspruch 1 oder 2, **dadurch gekennzeichnet, dass** die Verbindung die allgemeine Formel (4) aufweist.

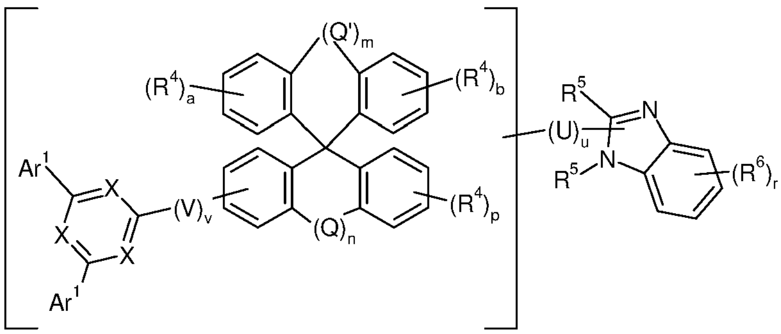

Formel (4)

4. Verbindung gemäß einem oder mehreren der Ansprüche 1 bis 3, **dadurch gekennzeichnet, dass** die Verbindung die allgemeine Formel (5) aufweist.

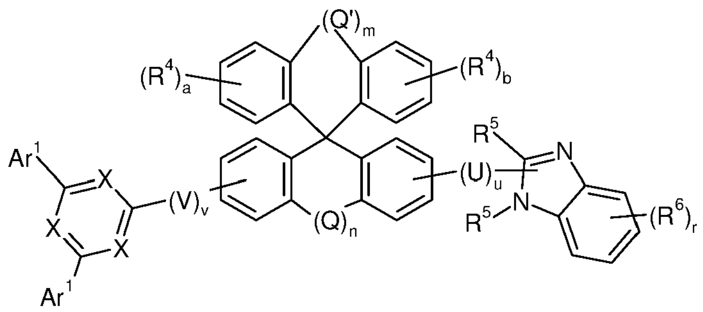

Formel (5)

5. Verbindung gemäß einem oder mehreren der Ansprüche 1 bis 3, **dadurch gekennzeichnet, dass** die Verbindung die allgemeine Formel (14) aufweist.

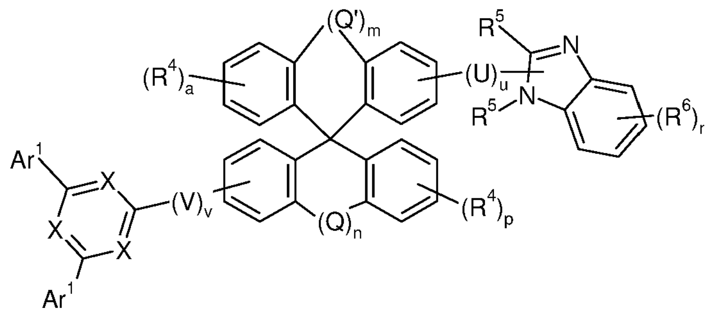

Formel (14)

6. Zusammensetzung enthaltend wenigstens eine Verbindung gemäß einem oder mehreren der Ansprüche 1 bis 5 sowie wenigstens eine weitere Verbindung ausgewählt aus der Gruppe bestehend aus fluoreszierenden Emittern, phosphoreszierenden Emittern, MatrixMaterialien, Elektronentransportmaterialien, Elektroneninjektionsmaterialien, Lochleitermaterialien, Lochinjektionsmaterialien, Elektronenblockiermaterialien und Lochblockiermaterialien, wide band gap Materialien oder n-Dotanden.

7. Zusammensetzung gemäß Anspruch 6, **dadurch gekennzeichnet, dass** die zusätzliche Verbindung Elektronentransportmaterial oder Matrixmaterial ist.

8. Formulierung enthaltend wenigstens eine Verbindung gemäß einem oder mehreren der Ansprüche 1 bis 5 oder wenigstens eine Zusammensetzung gemäß Anspruch 6 oder 7 sowie wenigstens ein Lösungsmittel.

9. Verwendung wenigstens einer Verbindung gemäß einem oder mehreren der Ansprüche 1 bis 5 oder wenigstens einer Zusammensetzung gemäß Anspruch 6 oder 7 in einer elektronischen Vorrichtung, bevorzugt in einer organischen Elektrolumineszenzvorrichtung, ganz bevorzugt in einer organischen lichtemittierenden Diode (OLED) oder organischen lichtemittierenden elektrochemischen Zelle (OLEC, LEEC, LEC), ganz besonders bevorzugt in einer OLED, bevorzugt in einer Emissionsschicht (EML), Elektronentransportschicht (ETL) und in einer Lochblockierschicht (HBL), und ganz bevorzugt in einer EML und ETL.

10. Elektronische Vorrichtung enthaltend wenigstens eine Verbindung gemäß einem oder mehreren der Ansprüche 1 bis 5 oder wenigstens eine Zusammensetzung gemäß Anspruch 6 oder 7, bevorzugt in einer Emissionsschicht (EML), Elektronentransportschicht (ETL) und in einer Lochblockierschicht (HBL), und ganz bevorzugt in einer EML oder ETL und ganz besonders bevorzugt in der ETL.

11. Elektronische Vorrichtung gemäß Anspruch 10, **dadurch gekennzeichnet, dass** sie ausgewählt ist aus organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren, organischen Photorezeptoren, bevorzugt eine organische Elektrolumineszenzvorrichtung.

**12.** Elektronische Vorrichtung gemäß Anspruch 10 oder 11, **dadurch gekennzeichnet, dass** sie eine organische Elektrolumineszenzvorrichtung ist, die ausgewählt ist auch der Gruppe bestehend aus organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und organischen lichtemittierenden Dioden (OLEDs), bevorzugt OLECs und OLEDs, ganz bevorzugt OLEDs.

**13.** Verfahren zur Herstellung einer elektronischen Vorrichtung gemäß einem oder mehreren der Ansprüche 10 bis 12, **dadurch gekennzeichnet, dass** mindestens eine organische Schicht durch Gasphasenabscheidung oder aus Lösung aufgebracht wird.

**Claims**

**1.** Compound of the formula (1)

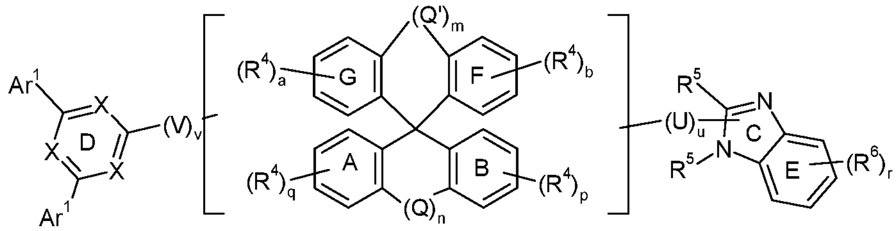

formula (1)

where the following definitions apply to the symbols and indices used:

$R^1$ is on each occurrence, identically or differently, H, D, F, Cl, Br, I, CHO, $N(Ar^1)2$, $C(=O)Ar^1$, $P(=O)(Ar^1)_2$, $S(=O)Ar^1$, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, which may in each case be substituted by one or more radicals $R^2$, where one or more, preferably non-adjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy or heteroaryloxy group having 5 to 60 aromatic ring atoms, which may be substituted by one or more radicals $R^2$, or a combination of these systems; two or more adjacent substituents $R^1$ may form a mono- or polycyclic, aliphatic or aromatic ring system with one another;

$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^3$, where one or more non-adjacent $CH_2$ groups may be replaced by $R^3C=CR^3$, C=C, $Si(R^3)2$, $Ge(R^3)2$, $Sn(R^3)_2$, C=O, C=S, C=Se, $C=NR^3$, $P(=O)(R^3)$, SO, $SO_2$, $NR^3$, O, S or $CONR^3$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroarylamino group having 10 to 40 aromatic ring atoms, which may be substituted by one or more radicals $R^3$, or a combination of two or more of these groups; two or more adjacent radicals $R^2$ may form a mono- or polycyclic, aliphatic or aromatic ring system with one another; where it is preferred if two or more adjacent substituents $R^2$ do not form a mono- or polycyclic, aliphatic or aromatic ring system with one another;

$R^3$ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents $R^3$ may also form a mono- or polycyclic, aliphatic or aromatic ring system with one another; where it is preferred if two or more adjacent substituents $R^3$ do not form a mono- or polycyclic, aliphatic

or aromatic ring system with one another;

$R^5$ is on each occurrence, identically or differently, F, Cl, Br, I, CHO, $N(Ar^1)2$, $C(=O)Ar^1$, $P(=O)(Ar^1)_2$, $S(=O)Ar^1$, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, which may in each case be substituted by one or more radicals $R^2$, where one or more, preferably non-adjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy or heteroaryloxy group having 5 to 60 aromatic ring atoms, which may be substituted by one or more radicals $R^2$, or a combination of these systems; where two or more adjacent substituents $R^5$ cannot form a mono- or polycyclic, aliphatic or aromatic ring system with one another;

$R^4$ and $R^6$ are on each occurrence, identically or differently, H, D, F, Cl, Br, I, CHO, $N(Ar^1)2$, $C(=O)Ar^1$, $P(=O)(Ar^1)_2$, $S(=O)Ar^1$, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, which may in each case be substituted by one or more radicals $R^2$, where one or more, preferably non-adjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy or heteroaryloxy group having 5 to 60 aromatic ring atoms, which may be substituted by one or more radicals $R^2$, or a combination of these systems; where two or more adjacent substituents $R^4$ cannot form a mono- or polycyclic, aliphatic or aromatic ring system with one another; where two or more adjacent substituents $R^6$ may form a mono- or polycyclic, aliphatic or aromatic ring system with one another, where it is preferred if two or more adjacent substituents $R^6$ cannot form a mono- or polycyclic, aliphatic or aromatic ring system with one another;

Q is C=O, C=S, S, $C(R^2)_2$, $NR^2$ or O, preferably $C(R^2)_2$, $NR^2$ or O, very preferably $C(R^2)_2$ or $NR^2$ and particularly preferably $C(R^2)2$;

n is 0 or 1, where n = 0 is preferred and n = 0 means that the two aromatic rings A and B are not linked to one another via the group Q, but instead by a single bond;

p is 0, 1, 2, 3 or 4, preferably 0, 1 or 2, very preferably 0 or 1 and particularly preferably equal to 0;

q is 0, 1, 2, 3 or 4, preferably 0, 1 or 2, very preferably 0 or 1 and particularly preferably equal to 0;

r is 0, 1, 2, 3 or 4, preferably 0, 1 or 2, very preferably 0 or 1 and r is particularly preferably equal to 0;

X is, identically or differently on each occurrence, N or $CR^1$, where at least one X is equal to N, preferably at least two of the X are equal to N and very preferably all three X are equal to N;

$Ar^1$ is on each occurrence, identically or differently, an aromatic or heteroaromatic ring system having 5 to 30 aromatic ring atoms, which may be substituted by one or more radicals $R^2$; two of the radicals $Ar^1$ may also be linked to one another by a single bond or a bridge selected from $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ and $P(=O)R^2$; it is preferred if two radicals $Ar^1$ are not linked to one another;

Q' is, identically to or differently from one another, C=O, C=S, S, $C(R^2)_2$, $NR^2$ or O, preferably $C(R^2)_2$, $NR^2$ or O, very preferably $C(R^2)_2$ or $NR^2$ and particularly preferably $C(R^2)2$;

a is 0, 1, 2, 3 or 4, preferably 0, 1 or 2, very preferably 0 or 1 and particularly preferably equal to 0;

b is 0, 1, 2, 3 or 4, preferably 0, 1 or 2, very preferably 0 or 1 and particularly preferably equal to 0;

a+b is always less than or equal to 7, a+b is preferably equal to 0, 1, 2 or 3, a+b is very preferably equal to 0, 1 or 2, a+b is particularly preferably equal to 0 or 1 and a+b is very particularly preferably equal to 0;

m is 0 or 1, where m = 0 is preferred and m = 0 means that the two aromatic rings are not linked to one another via the group Q', but instead by a single bond;

V is a divalent group;

U is a divalent group;

v is 0 or 1, where v = 0 means that the ring D is connected directly to the remainder of the compound via a single covalent bond;

u is 0 or 1, where u = 0 means that the ring C is connected directly to the remainder of the compound via a single covalent bond.

2. Compound according to Claim 1, **characterised in that** the compound is a small molecule having a molecular weight of at most 3000 g/mol.

3. Compound according to Claim 1 or 2, **characterised in that** the compound has the general formula (4).

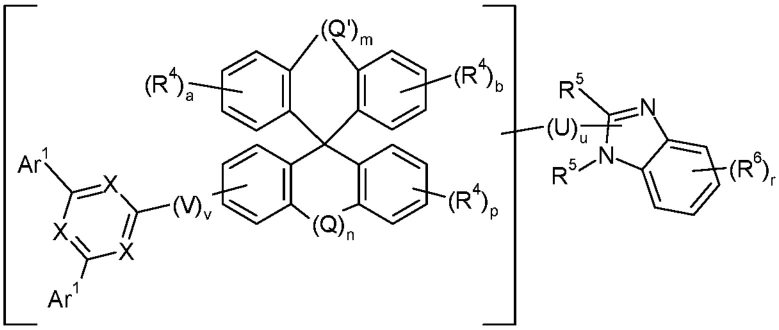

formula (4)

4. Compound according to one or more of Claims 1 to 3, **characterised in that** the compound has the general formula (5).

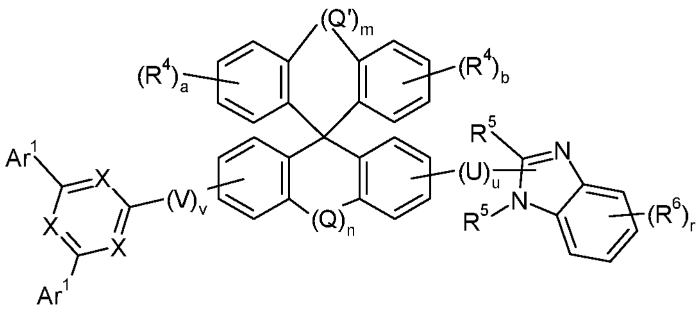

formula (5)

5. Compound according to one or more of Claims 1 to 3, **characterised in that** the compound has the general formula (14).

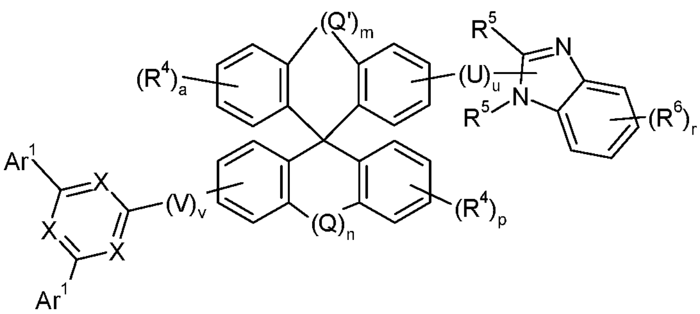

formula (14)

6. Composition comprising at least one compound according to one or more of Claims 1 to 5 and at least one further compound selected from the group consisting of fluorescent emitters, phosphorescent emitters, matrix materials, electron-transport materials, electron-injection materials, hole-conductor materials, hole-injection materials, electron-blocking materials and hole-blocking materials, wide band gap materials and n-dopants.

7. Composition according to Claim 6, **characterised in that** the additional compound is an electron-transport material or matrix material.

8. Formulation comprising at least one compound according to one or more of Claims 1 to 5 or at least one composition according to Claim 6 or 7 and at least one solvent.

**9.** Use of at least one compound according to one or more of Claims 1 to 5 or at least one composition according to Claim 6 or 7 in an electronic device, preferably in an organic electroluminescent device, very preferably in an organic light-emitting diode (OLED) or organic light-emitting electrochemical cell (OLEC, LEEC, LEC), very particularly preferably in an OLED, preferably in an emission layer (EML), electron-transport layer (ETL) and in a hole-blocking layer (HBL), and very preferably in an EML and ETL.

**10.** Electronic device comprising at least one compound according to one or more of Claims 1 to 5 or at least one composition according to Claim 6 or 7, preferably in an emission layer (EML), electron-transport layer (ETL) and in a hole-blocking layer (HBL), and very preferably in an EML or ETL and very particularly preferably in an ETL.

**11.** Electronic device according to Claim 10, **characterised in that** it is selected from organic integrated circuits (OICs), organic field-effect transistors (OFETs), organic thin-film transistors (OTFTs), organic electroluminescent devices, organic solar cells (OSCs), organic optical detectors, organic photoreceptors, preferably an organic electroluminescent device.

**12.** Electronic device according to Claim 10 or 11, **characterised in that** it is an organic electroluminescent device selected from the group consisting of organic light-emitting transistors (OLETs), organic field-quench devices (OFQDs), organic light-emitting electrochemical cells (OLECs, LECs, LEECs), organic laser diodes (O-lasers) and organic light-emitting diodes (OLEDs), preferably OLECs and OLEDs, very preferably OLEDs.

**13.** Process for the production of an electronic device according to one or more of Claims 10 to 12, **characterised in that** at least one organic layer is applied by gas-phase deposition or from solution.

## Revendications

**1.** Composé de formule (1)

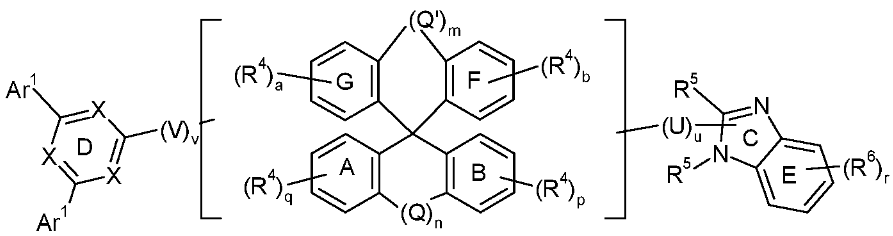

formule (1)

dans lequel les définitions suivantes s'appliquent aux symboles et indices utilisés :

$R^1$ est à chaque occurrence, de manière identique ou différente, H, D, F, Cl, Br, I, CHO, N($Ar^1$)2, C(=O)$Ar^1$, P(=O)$(Ar^1)_2$, S(=O)Ar1, S$(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, un groupement alkyle, alcoxy ou thioalcoxy à chaîne linéaire ayant de 1 à 40 atomes de C ou un groupement alcényle ou alcynyle à chaîne linéaire ayant de 2 à 40 atomes de C ou un groupement alkyle, alcényle, alcynyle, alcoxy ou thioalcoxy ramifié ou cyclique ayant de 3 à 40 atomes de C, pouvant être dans chaque cas substitué par un ou plusieurs radicaux $R^2$, où un ou plusieurs groupements $CH_2$ préférablement non adjacents peuvent être remplacés par $R^2C=CR^2$, C=C, $Si(R^2)_2$, $Ge(R^2)_2$, $Sn(R^2)_2$, C=O, C=S, C=Se, C=$NR^2$, P(=O)($R^2$), SO, $SO_2$, $NR^2$, O, S ou CON$R^2$ et où un ou plusieurs atomes de H peuvent être remplacés par D, F, Cl, Br, I, CN ou $NO_2$, ou un noyau aromatique ou hétéroaromatique ayant de 5 à 60 atomes de cycle aromatique, pouvant être dans chaque cas substitué par un ou plusieurs radicaux $R^2$, ou un groupement aryloxy ou hétéroaryloxy ayant de 5 à 60 atomes de cycle aromatique, pouvant être substitué par un ou plusieurs radicaux $R^2$, ou une combinaison de ces noyaux ; deux, ou plus, substituants $R^1$ adjacents peuvent former un noyau mono- ou polycyclique, aliphatique ou aromatique les uns avec les autres ;

$R^2$ est, de manière identique ou différente à chaque occurrence, H, D, F, Cl, Br, I, $N(R^3)_2$, CN, $NO_2$, $Si(R^3)_3$, $B(OR^3)_2$, C(=O)$R^3$, P(=O)$(R^3)_2$, S(=O)$R^3$, S$(=O)_2R^3$, $OSO_2R^3$, un groupement alkyle, alcoxy ou thioalcoxy à chaîne linéaire ayant de 1 à 40 atomes de C ou un groupement alcényle ou alcynyle à chaîne linéaire ayant de 2 à 40 atomes de C ou un groupement alkyle, alcényle, alcynyle, alcoxy, alkylalcoxy ou thioalcoxy ramifié

ou cyclique ayant de 3 à 40 atomes de C, pouvant être dans chaque cas substitué par un ou plusieurs radicaux $R^3$, où un ou plusieurs groupements $CH_2$ non adjacents peuvent être remplacés par $R^3C=CR^3$, C=C, $Si(R^3)2$, $Ge(R^3)2$, $Sn(R^3)_2$, C=O, C=S, C=Se, $C=NR^3$, $P(=O)(R^3)$, SO, $SO_2$, $NR^3$, O, S ou $CONR^3$ et où un ou plusieurs atomes de H peuvent être remplacés par D, F, Cl, Br, I, CN ou $NO_2$, ou un noyau aromatique ou hétéroaromatique ayant de 5 à 60 atomes de cycle aromatique, pouvant être dans chaque cas substitué par un ou plusieurs radicaux $R^3$, ou un groupement aryloxy, arylalcoxy ou hétéroaryloxy ayant de 5 à 60 atomes de cycle aromatique, pouvant être substitué par un ou plusieurs radicaux $R^3$, ou un groupement diarylamino, un groupement dihétéroarylamino ou un groupement arylhétéroarylamino ayant de 10 à 40 atomes de cycle aromatique, pouvant être substitué par un ou plusieurs radicaux $R^3$, ou une combinaison de deux, ou plus, parmi ces groupements ; deux, ou plus, radicaux $R^2$ adjacents peuvent former un noyau mono- ou polycyclique, aliphatique ou aromatique les uns avec les autres ; où on préfère que deux, ou plus, substituants $R^2$ adjacents ne forment pas de noyau mono- ou polycyclique, aliphatique ou aromatique les uns avec les autres ;

$R^3$ est, de manière identique ou différente à chaque occurrence, H, D, F ou un radical hydrocarboné aliphatique, aromatique et/ou hétéroaromatique ayant de 1 à 20 atomes de C, dans lequel, de plus, un ou plusieurs atomes de H peuvent être remplacés par F ; deux, ou plus, substituants $R^3$ peuvent également former un noyau mono- ou polycyclique, aliphatique ou aromatique les uns avec les autres ; où on préfère que deux, ou plus, substituants $R^3$ adjacents ne forment pas de noyau mono- ou polycyclique, aliphatique ou aromatique les uns avec les autres ;

$R^5$ est à chaque occurrence, de manière identique ou différente, F, Cl, Br, I, CHO, $N(Ar^1)2$, $C(=O)Ar^1$, $P(=O)(Ar^1)_2$, S(=O)Ar1, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, un groupement alkyle, alcoxy ou thioalcoxy à chaîne linéaire ayant de 1 à 40 atomes de C ou un groupement alcényle ou alcynyle à chaîne linéaire ayant de 2 à 40 atomes de C ou un groupement alkyle, alcényle, alcynyle, alcoxy ou thioalcoxy ramifié ou cyclique ayant de 3 à 40 atomes de C, pouvant être dans chaque cas substitué par un ou plusieurs radicaux $R^2$, où un ou plusieurs groupements $CH_2$ préférablement non adjacents peuvent être remplacés par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atomes de H peuvent être remplacés par D, F, Cl, Br, I, CN ou $NO_2$, ou un noyau aromatique ou hétéroaromatique ayant de 5 à 60 atomes de cycle aromatique, pouvant être dans chaque cas substitué par un ou plusieurs radicaux $R^2$, ou un groupement aryloxy ou hétéroaryloxy ayant de 5 à 60 atomes de cycle aromatique, pouvant être substitué par un ou plusieurs radicaux $R^2$, ou une combinaison de ces noyaux ; où deux, ou plus, substituants $R^5$ adjacents ne peuvent pas former de noyau mono- ou polycyclique, aliphatique ou aromatique les uns avec les autres ;

$R^4$ et $R^6$ sont à chaque occurrence, de manière identique ou différente, H, D, F, Cl, Br, I, CHO, $N(Ar^1)2$, $C(=O)Ar^1$, $P(=O)(Ar^1)_2$, $S(=O)Ar^1$, $S(=O)_2Ar^1$, $CR^2=CR^2Ar^1$, CN, $NO_2$, $Si(R^2)_3$, $B(OR^2)_2$, $B(R^2)_2$, $B(N(R^2)_2)_2$, $OSO_2R^2$, un groupement alkyle, alcoxy ou thioalcoxy à chaîne linéaire ayant de 1 à 40 atomes de C ou un groupement alcényle ou alcynyle à chaîne linéaire ayant de 2 à 40 atomes de C ou un groupement alkyle, alcényle, alcynyle, alcoxy ou thioalcoxy ramifié ou cyclique ayant de 3 à 40 atomes de C, pouvant être dans chaque cas substitué par un ou plusieurs radicaux $R^2$, où un ou plusieurs groupements $CH_2$ préférablement non adjacents peuvent être remplacés par $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atomes de H peuvent être remplacés par D, F, Cl, Br, I, CN ou $NO_2$, ou un noyau aromatique ou hétéroaromatique ayant de 5 à 60 atomes de cycle aromatique, pouvant être dans chaque cas substitué par un ou plusieurs radicaux $R^2$, ou un groupement aryloxy ou hétéroaryloxy ayant de 5 à 60 atomes de cycle aromatique, pouvant être substitué par un ou plusieurs radicaux $R^2$, ou une combinaison de ces noyaux ; où deux, ou plus, substituants $R^4$ adjacents ne peuvent pas former de noyau mono- ou polycyclique, aliphatique ou aromatique les uns avec les autres ; où deux, ou plus, substituants $R^6$ adjacents peuvent former un noyau mono- ou polycyclique, aliphatique ou aromatique les uns avec les autres, où on préfère que deux, ou plus, substituants $R^6$ adjacents ne peuvent pas former de noyau mono- ou polycyclique, aliphatique ou aromatique les uns avec les autres ;

Q est C=O, C=S, S, $C(R^2)_2$, $NR^2$ ou O, préférablement $C(R^2)_2$, $NR^2$ ou O, très préférablement $C(R^2)_2$ ou $NR^2$ et particulièrement préférablement $C(R^2)_2$ ;

n vaut 0 ou 1, où n = 0 est préféré et n = 0 signifie que les deux cycles aromatiques A et B ne sont pas liés l'un à l'autre via le groupement Q, mais plutôt par une liaison simple ;

p vaut 0, 1, 2, 3 ou 4, préférablement 0, 1 ou 2, très préférablement 0 ou 1 et particulièrement préférablement est égal à 0 ;

q vaut 0, 1, 2, 3 ou 4, préférablement 0, 1 ou 2, très préférablement 0 ou 1 et particulièrement préférablement est égal à 0 ;

r vaut 0, 1, 2, 3 ou 4, préférablement 0, 1 ou 2, très préférablement 0 ou 1 et r est particulièrement préférablement égal à 0 ;

X est, de manière identique ou différente à chaque occurrence, N ou $CR^1$, où au moins un X est égal à N, préférablement au moins deux parmi les X sont égaux à N et très préférablement les trois X sont égaux à N ;

$Ar^1$ est à chaque occurrence, de manière identique ou différente, un noyau aromatique ou hétéroaromatique ayant de 5 à 30 atomes de cycle aromatique, pouvant être substitué par un ou plusieurs radicaux $R^2$ ; deux parmi les radicaux $Ar^1$ peuvent également être liés l'un à l'autre par une liaison simple ou un pont choisi parmi $B(R^2)$, $C(R^2)_2$, $Si(R^2)_2$, C=O, $C=NR^2$, $C=C(R^2)2$, O, S, S=O, $SO_2$, $N(R^2)$, $P(R^2)$ et $P(=O)R^2$ ; on préfère que deux radicaux $Ar^1$ ne soient pas liés l'un à l'autre ;

Q' est, de manière identique ou différente l'un de l'autre, C=O, C=S, S, $C(R^2)_2$, $NR^2$ ou O, préférablement $C(R^2)_2$, $NR^2$ ou O, très préférablement $C(R^2)_2$ ou $NR^2$ et particulièrement préférablement $C(R^2)_2$ ;

a vaut 0, 1, 2, 3 ou 4, préférablement 0, 1 ou 2, très préférablement 0 ou 1 et particulièrement préférablement est égal à 0 ;

b vaut 0, 1, 2, 3 ou 4, préférablement 0, 1 ou 2, très préférablement 0 ou 1 et particulièrement préférablement est égal à 0 ;

a+b est toujours inférieur ou égal à 7, a+b est préférablement égal à 0, 1, 2 ou 3, a+b est très préférablement égal à 0, 1 ou 2, a+b est particulièrement préférablement égal à 0 ou 1 et a+b est très particulièrement préférablement égal à 0 ;

m vaut 0 ou 1, où m = 0 est préféré et m = 0 signifie que les deux cycles aromatiques ne sont pas liés l'un à l'autre via le groupement Q', mais plutôt par une liaison simple ;

V est un groupement divalent ;

U est un groupement divalent ;

v vaut 0 ou 1, où v = 0 signifie que le cycle D est relié directement au reste du composé via une liaison covalente simple ;

u vaut 0 ou 1, où u = 0 signifie que le cycle C est relié directement au reste du composé via une liaison covalente simple.

2. Composé selon la revendication 1, **caractérisé en ce que** le composé est une petite molécule ayant un poids moléculaire d'au plus 3000 g/mol.

3. Composé selon la revendication 1 ou 2, **caractérisé en ce que** le composé répond à la formule générale (4).

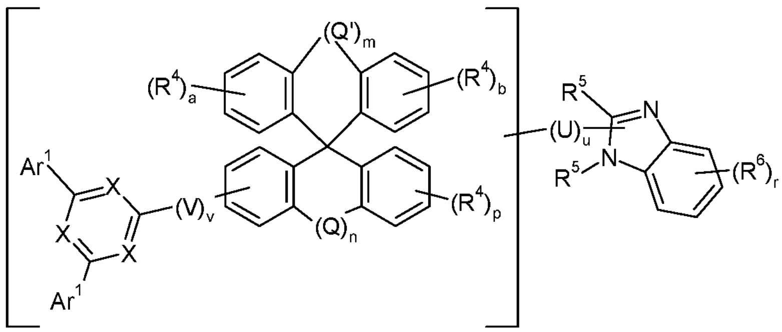

formule (4)

4. Composé selon l'une ou plusieurs parmi les revendications 1 à 3, **caractérisé en ce que** le composé répond à la formule générale (5).

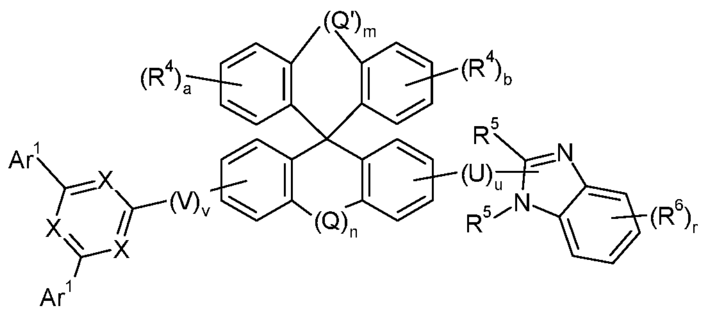

formule (5)

5. Composé selon l'une ou plusieurs parmi les revendications 1 à 3, **caractérisé en ce que** le composé répond à la formule générale (14).

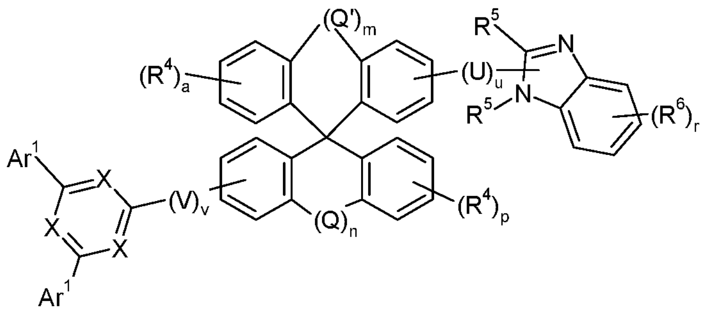

formule (14)

6. Composition comprenant au moins un composé selon l'une ou plusieurs parmi les revendications 1 à 5 et au moins un autre composé choisi dans le groupe constitué par les émetteurs fluorescents, les émetteurs phosphorescents, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous, les matériaux à large bande interdite et les dopants de type n.

7. Composition selon la revendication 6, **caractérisée en ce que** le composé supplémentaire est un matériau de transport d'électrons ou un matériau de matrice.

8. Formulation comprenant au moins un composé selon l'une ou plusieurs parmi les revendications 1 à 5 ou au moins une composition selon la revendication 6 ou 7 et au moins un solvant.

9. Utilisation d'au moins un composé selon l'une ou plusieurs parmi les revendications 1 à 5 ou d'au moins une composition selon la revendication 6 ou 7 dans un dispositif électronique, de préférence dans un dispositif électroluminescent organique, très préférablement dans une diode électroluminescente organique (OLED) ou une cellule électrochimique électroluminescente organique (OLEC, LEEC, LEC), très particulièrement préférablement dans une OLED, de préférence dans une couche d'émission (EML), une couche de transport d'électrons (ETL) et dans une couche de blocage de trous (HBL), et très préférablement dans une EML et une ETL.

10. Dispositif électronique comprenant au moins un composé selon l'une ou plusieurs parmi les revendications 1 à 5 ou au moins une composition selon la revendication 6 ou 7, de préférence dans une couche d'émission (EML), une couche de transport d'électrons (ETL) et dans une couche de blocage de trous (HBL), et très préférablement dans une EML ou une ETL et très particulièrement préférablement dans une ETL.

11. Dispositif électronique selon la revendication 10, **caractérisé en ce qu'**il est choisi parmi les circuits intégrés organiques (OIC), les transistors organiques à effet de champ (OFET), les transistors organiques à couche mince (OTFT), les dispositifs électroluminescents organiques, les cellules solaires organiques (OSC), les détecteurs organiques optiques, les photorécepteurs organiques, préférablement un dispositif électroluminescent organique.

**12.** Dispositif électronique selon la revendication 10 ou 11, **caractérisé en ce qu'**il s'agit d'un dispositif électroluminescent organique choisi dans le groupe constitué par les transistors organiques émetteurs de lumière (OLET), les dispositifs organiques à extinction de champ (OFQD), les cellules électrochimiques organiques émettrices de lumière (OLEC, LEC, LEEC), les diodes laser organiques (O-lasers) et les diodes électroluminescentes organiques (OLED), de préférence les OLEC et les OLED, très préférablement les OLED.

**13.** Procédé de production d'un dispositif électronique selon l'une ou plusieurs parmi les revendications 10 à 12, **caractérisé en ce qu'**au moins une couche organique est appliquée par dépôt en phase gazeuse ou à partir d'une solution.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- WO 2010126270 A
- KR 101257695
- WO 2013100464 A
- US 2013200356 A1
- DE 102010033080 A1
- DE 102008036982 A1
- DE 102013013876
- WO 200586251 A2
- WO 2012175535 A1
- WO 200900237 A1
- WO 2012168358 A1
- US 20070145355 A1
- EP 2452946 A1
- EP 2463927 A1
- WO 2012031735 A1
- EP 1837926 A1
- WO 2007107306 A1
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2010108579 A
- WO 2011116865 A
- WO 2011137951 A
- WO 2013064206 A
- WO 2014056567 A
- EP 13003343
- EP 14000729
- EP 676461 A
- WO 2004081017 A
- WO 2004058911 A
- WO 2005084081 A
- WO 2005084082 A
- WO 2006048268 A
- WO 2006117052 A
- WO 2008145239 A
- US 20050069729 A
- WO 2005039246 A
- JP 2004288381 A
- EP 1205527 A
- WO 2008086851 A
- WO 2011088877 A
- WO 2011128017 A
- WO 2010136109 A
- WO 2011000455 A
- EP 1617710 A
- EP 1617711 A
- EP 1731584 A
- JP 2005347160 A
- WO 2007063754 A
- WO 2008056746 A
- WO 2004093207 A
- WO 2010006680 A
- WO 2005003253 A
- WO 2007137725 A
- WO 2005111172 A
- WO 2010015306 A
- EP 652273 A
- WO 2009062578 A
- WO 2010054729 A
- WO 2010054730 A
- US 7294849 B
- WO 2005011013 A


### In der Beschreibung aufgeführte Nicht-Patentliteratur


- **M. A. BALDO et al.** *Appl. Phys. Lett.,* 1999, vol. 75, 4-6